(19) Europäisches Patentamt / European Patent Office / Office européen des brevets

(11) EP 1 539 976 B1

(12) **EUROPÄISCHE PATENTSCHRIFT**

(45) Veröffentlichungstag und Bekanntmachung des Hinweises auf die Patenterteilung:
**02.02.2011 Patentblatt 2011/05**

(21) Anmeldenummer: **03747816.1**

(22) Anmeldetag: **22.08.2003**

(51) Int Cl.:
***C12P 7/62*** *(2006.01)* ***A61K 47/48*** *(2006.01)*

(86) Internationale Anmeldenummer:
**PCT/DE2003/002799**

(87) Internationale Veröffentlichungsnummer:
**WO 2004/020623 (11.03.2004 Gazette 2004/11)**

(54) **VERFAHREN ZUR HERSTELLUNG VON BIOLOGISCH ABBAUBAREN, FUNKTIONALISIERTEN POLYMERPARTIKELN UND DEREN VERWENDUNG ALS ARZNEIMITTELTRÄGER**

METHOD FOR PRODUCING BIODEGRADABLE, FUNCTIONALISED POLYMER PARTICLES, AND USE OF THE SAME AS MEDICAMENT CARRIERS

PROCEDE DE PRODUCTION DE PARTICULES POLYMERES FONCTIONNALISEES BIODEGRADABLES ET UTILISATION DESDITES PARTICULES EN TANT QUE SUPPORTS DE MEDICAMENTS

(84) Benannte Vertragsstaaten:
**AT BE BG CH CY CZ DE DK EE ES FI FR GB GR HU IE IT LI LU MC NL PT RO SE SI SK TR**

(30) Priorität: **30.08.2002 DE 10240035**

(43) Veröffentlichungstag der Anmeldung:
**15.06.2005 Patentblatt 2005/24**

(60) Teilanmeldung:
**10181200.6**

(73) Patentinhaber: **MASSEY UNIVERSITY Palmerston North (NZ)**

(72) Erfinder: **REHM, Bernd Helmut Adam Palmerston North (NZ)**

(74) Vertreter: **Donald, Jenny Susan et al Forrester & Boehmert Pettenkoferstrasse 20-22 80336 München (DE)**

(56) Entgegenhaltungen:
**WO-A-99/20256 US-A- 6 022 729 US-A- 6 146 665**

- **MADISON L L ET AL: "METABOLIC ENGINEERING OF POLY(3-HYDROXYALKANOATES): FROM DNA TO PLASTIC" MICROBIOLOGY AND MOLECULAR BIOLOGY REVIEWS, AMERICAN SOCIETY FOR MICROBIOLOGY, US, Bd. 63, Nr. 1, März 1999 (1999-03), Seiten 21-53, XP000869647 ISSN: 1092-2172**
- **STEINBUCHEL A ET AL: "Bacterial and other biological systems for polyester production" TRENDS IN BIOTECHNOLOGY, ELSEVIER PUBLICATIONS, CAMBRIDGE, GB, Bd. 16, Nr. 10, 1. Oktober 1998 (1998-10-01), Seiten 419-427, XP004145649 ISSN: 0167-7799**
- **SIM SANG JUN ET AL: "PHA synthase activity controls the molecular weight and polydispersity of polyhydroxybutyrate in vivo" NATURE BIOTECHNOLOGY, Bd. 15, Nr. 1, 1997, Seiten 63-67, XP002268360 ISSN: 1087-0156**
- **STEINBUECHEL A ET AL: "DIVERSITY OF BACTERIAL POLYHYDROXYALKANOIC ACIDS" FEMS MICROBIOLOGY LETTERS, AMSTERDAM, NL, Bd. 128, Nr. 3, 15. Mai 1995 (1995-05-15), Seiten 219-228, XP000828495 ISSN: 0378-1097**
- **KREUTER J: "NANOPARTICULATE SYSTEMS FOR BRAIN DELIVERY OF DRUGS" ADVANCED DRUG DELIVERY REVIEWS, AMSTERDAM, NL, Bd. 47, Nr. 1, 23. März 2001 (2001-03-23), Seiten 65-81, XP001119510 ISSN: 0169-409X**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 539 976 B1

## Beschreibung

Die vorliegende Erfindung betrifft ein Verfahren zur Herstellung von biologisch abbaubaren, funktionalisierten Polymerpartikeln und deren Verwendung als Arzneimittelträger.

Stand der Technik

Die Effektivität vieler Wirkstoffe hängt davon ab, ob sie genau an den gewünschten Zielort im tierischen Körper transportiert werden, da die verwendeten Wirkstoffe häufig nur an einer bestimmten Stelle ihre volle Wirkung entfalten können und an anderen Stellen nicht wirken oder dort sogar negative Auswirkungen haben. Letzteres gilt besonders bei der Bekämpfung von krebsartigen Erkrankungen und insbesondere von krebsartigen Erkrankungen, die im Zentralen Nervensystem (ZNS) auftreten.

Ein gutes Beispiel für die Schwierigkeiten, die beim Transport von Wirkstoffen im tierischen Körper auftreten, bietet die Blut-Hirn-Schranke (BHS). Dort müssen Wirkstoffe, die ihren Zielort im ZNS haben, zuvor diese Barriere überwinden. Dieses Netzwerk von Blutgefäßen und Zellen schützt das ZNS und verhindert, dass nicht wasserlösliche Substanzen in das ZNS eindringen können. Fettlösliche Substanzen können diese Schranke ohne Schwierigkeiten durch einfache Diffusion durchschreiten. Für den Transport von polaren Substanzen und Ionen sind allerdings aktive und passive Transportsysteme notwendig. Da aber z.B. mehr als 98% aller neu entdeckten Medikamente, deren Zielort im ZNS liegt, nicht wasserlöslich sind, können sie die BHS nicht überqueren und somit ihren Zielort nicht erreichen.

Anhand dieses Beispiels sollen die Schwierigkeiten aufgezeigt werden, die beim Transport von Wirkstoffen im tierischen Organismus auftreten können. Bei der BHS wird zum Beispiel versucht durch Veränderung der Permeabilität der Membran, diese für Wirkstoffe durchlässiger zu machen. Die Durchlässigkeit der BHS für Wirkstoffe kann zum Beispiel durch künstliche Erhöhung des osmotischen Drucks oder der Verabreichung von Bradykinin Analoga erreicht werden. Sanovich et al. (Sanovich, E. et al., Brain Res. 1995, Vol.705(1-2), S.125-135) und andere demonstrierten z.B. eine erhöhte Permeabilität der BHS für Lanthan durch gleichzeitige Verabreichung mit dem Bradykinin Analogon RMP-7. Ein grundsätzlicher Nachteil beim Öffnen der BHS durch einen der vorgenannten Mechanismen ist, dass sich die Permeabilität dadurch für alle Substanzen und damit auch für toxische Substanzen, die den Zielzellen im ZNS schaden können, erhöht.

Eine andere Möglichkeit besteht darin, den Wirkstoff chemisch so zu modifizieren, dass er lipophiler wird und damit auch leichter die BHS durchschreiten kann, wie es zum Beispiel an Chlorambucil-Derivaten gezeigt worden ist (Greig, N.H. et al., Cancer Chemother. Pharmacol. 1990, Vol.25(5), S.320-325).

Eine Alternative zu beiden vorgenannten Systemen bietet die Verwendung von Nano- bzw. Mikropartikeln. Durch ihre Verwendung müssen weder die Durchlässigkeit der Membran noch der Wirkstoff modifiziert werden. Kreuter (Kreuter, J. J. Anat. 1996, Vol. 189(3), S.503-505) zeigt auf, dass die dazu verwendeten Polymerpartikel im wesentlichen durch chemische Verfahren, wie Emulsionspolymerisation, Grenzflächenpolymerisation, Desolvatisieren, Abdampfen und Lösungsmittelabscheidung hergestellt werden.

Gemäß der DE 197 45 950 A1 werden Polymerpartikel aus den verschiedensten Substanzen (Polymere (hier: Polybutylcyanoacrylat), feste oder flüssige Lipide, o/w-Emulsionen, w/o/w-Emulsionen oder Phospholipidvesikel) verwendet, an die Arzneistoffe gebunden werden, um sie ins ZNS zu transportieren.

Ein anderer kritischer Punkt beim Transport von Nano- und Mikropartikeln über eine Membran und insbesondere die Membranen der BHS, ist die Größe der Polymerpartikel. Bisherige Forschungsergebnisse zeigen, dass Polymerpartikel bis zu einer Größe von 270 nm die BHS überwinden können (Lockman, P.R et al., Drug Develop. Indust. Pharmacy 2002, Vol.28(1), S.1-12).

Daher ist es wichtig, dass die verwendeten Polymerpartikel ein bestimmte Größe aufweisen. Nach Lockmann (Lockman, P.R. et al., Drug Develop. Indust. Pharmacy 2002, Vo1.28(1), S.1-12) wird die Größe der durch die nach den obigen Verfahren hergestellten Polymerpartikel meistens durch Photonenkorrelationsspektroskopie bestimmt. Diese auf der Brownschen Molekularbewegung beruhende Technik vermisst das Polymerpartikel mit einem Laserstrahl, wobei die Zeitabhängigkeit der Lichtveränderung zur Bestimmung der Partikelgröße herangezogen wird. Jedoch ist dieses Analyseverfahren zur nachträglichen Bestimmung der Größe der hergestellten Polymerpartikel sehr zeitund kostenaufwendig.

Daher ist es die Aufgabe der vorliegenden Erfindung, ein schnell einsetzbares und kostengünstiges Transportsystem für biologisch aktive Substanzen zur Verfugung zu stellen, dass einen effektiven und sicheren Transport von Wirkstoffen im tierischen Organismus ermöglicht.

Als "biologisch aktive Substanz" im Sinne dieser Erfindung wird jede Substanz bezeichnet, die eine biologische Reaktion des Organismus auslösen kann. Diese Substanzen umfassen sowohl Enzyme und Abzyme, die eine bestimmte Reaktion im Organismus katalysieren, Proteine, wie zum Beispiel Antikörper, die eine indirekte Reaktion des Organismus auf die Anwesenheit dieser Substanz im Organismus hervorrufen als auch anorganische und organische Moleküle, die nicht biologischen Ursprungs sind, das heißt, nicht von einem natürlich vorkommenden Organismus gebildet werden, sondern künstlich hergestellt werden. Zur letzten Gruppe gehören auch die meisten pharmazeutischen Wirkstoffe. Die biologisch aktiven Substanzen können je nach ihrer Art auch dazu geeignet sein, weitere biologisch aktive Substanzen zu binden.

Um die oben genannte Aufgabe zu lösen, wird ein Verfahren zur Herstellung von biologisch abbaubaren Polymerpartikeln bereitgestellt. Dieses Verfahren umfasst das Einbringen zumindest eines induzierbaren Gens in einen Mikroorganismus, wobei das Gen für ein Protein codiert, das die Größe der Polymerpartikel kontrolliert , und wenigstens eines weiteren Gens, das für eine Polymer-Synthase kodiert, wobei es sich bei der wenigstens einen Polymer-Synthase um ein ein biologisch aktives Protein umfassendes Fusionsprotein handelt, oder wobei es sich bei der wenigstens einen Polymer-Synthase um ein Fusionsprotein handelt, das dazu in der Lage ist, sich direkt oder über ein Kopplungsreagenz an eine biologisch aktive Substanz zu binden, und das Kultivieren des Mikroorganismus unter Induktion des oben genannten zumindest einen induzierbaren Gens in einem Kulturmedium unter Bedingungen, die zur Herstellung der biologisch abbaubaren Polymerpartikel durch den Mikroorganismus geeignet sind. Durch dieses Verfahren ist es möglich biokompatible, biologisch abbaubare Polymerpartikel herzustellen, die sich für den Transport von biologisch aktiven Substanzen eignen und wobei durch die Steuerung der Größe der sich bildenden Polymerpartikel, diese je nach Bedarf in gewünschter Größe produziert werden können. Durch die kontrollierte Herstellung von Polymerpartikel bestimmter Größe wird die Ausbeute an Polymerpartikeln der gewünschten Größe erhöht, was zur Steigerung der Effektivität des Verfahrens führt und gleichzeitig hilft Kosten zu sparen. Außerdem können durch das erfindungsgemäße Verfahren Polymerpartikel hergestellt werden, die der bereits zuvor genannten Anforderung an die Partikelgröße für den Transport über die BHS entsprechen. Durch dieses Verfahren können vor allem Polymerpartikel hergestellt werden, die kleiner sind als die durch Mikroorganismen natürlich hergestellten Polymerpartikel dieser Art. Die durchschnittliche Größe der durch Mikroorganismen natürlich hergestellten Polymerpartikel beträgt 300 bis 500 nm (Wieczorek, R. et al., J. Bacteriol. 1997, Vol.177(9), 5.2425-2435), während das erfindungsgeurtälle Verfahren es ermöglicht, die Herstellung der Polymerpartikel so zu steuern, dass sie deutlich unter diesem Durchschnittswert liegen.

Die Induktion des die Partikelgröße bestimmenden Gens erfolgt dabei über einen vorgeschalteten induzierbaren Promotor, wie z.B. einen bad-Promotor, den man durch Arabinose induziert. Die Mikroorganismen, die man dafür einsetzt, besitzen kein Gen für die Kontrolle der Größe der Polymerpartikel oder man inaktiviert dieses Gen und ersetzt es durch das im erfindungsgemäßen Verfahren beschriebene zumindest eine induzierbare Gen, das für ein Protein codiert, das die Größe der Polymerpartikel kontrolliert. Dieses Gen wird dabei über einen Vektor in die Zelle eingebracht, der im experimentellen Teil dieser Beschreibung näher beschrieben wird. Dadurch ist es erstmalig möglich, biokompatible, biologisch abbaubare Polymerpartikel definierter Größe in einem mikrobiellen Verfahren herzustellen.

Diese Polymerpartikel werden als cytoplasmatische Einschlüsse in der Zelle abgelagert. Der Kern dieser Polymerpartikel besteht aus Polyhydroxyalkylcarboxylaten insbesondere Polyhydroxyalkanoaten und wird von einer aus Proteinen und Phospholipiden bestehenden Hüllmembran umgeben. Die Hüllmembran besteht aus Lipiden und in sie eingebettete Proteine. Die Polyhydroxyalkylcarboxylate, die den Kern dieser Polymerpartikel bilden, haben Schmelzpunkte von 50°C bis 176°C, eine Kristallinität von 30% bis 70% und Zerreisdehnungswerte von 5% bis 300%.

Um dieses vorteilhafte Verfahren auch in Mikroorganismen zur Anwendung zu bringen, die sich für die biotechnologische Züchtung besser eignen (z.B. bestimmte Formen von *E. coli,* die als GRAS Organismen klassifiziert sind) aber die oben genannten Polymerpartikel aufgrund ihrer genetischen Ausstattung nicht bilden können, bringt man neben dem zumindest einem induzierbaren Gen, das für ein Protein codiert, das die Größe der Polymerpartikel kontrolliert, mindestens ein weiteres Gen ein, das für ein bei der Bildung der Polymerpartikel beteiligtes Protein codiert. Denkbar ist jedes Protein, dass den Stoffwechsel, der zur Bildung der Polymerpartikel führt beeinflussen kann und damit die Zusammensetzung der sich bildenden Polymerpartikel. Dabei wählt man das mindestens eine weitere Gen, das für ein bei der Bildung der Polymerpartikel beteiligtes Protein codiert, so aus, das es für eine Thiolase, eine Reduktase oder eine Polymer-Synthase codiert. Unter einer Polymer-Synthase versteht man jedes Protein, das den letzten Schritt zur Bildung eines Polymers katalysieren kann. Neben den in dieser Erfindung beschriebenen Polymer-Synthasen kann die Bildung eines Polymers z.B. auch von einer Lipase übernommen werden. Bevorzugt wählt man das mindestens eine weitere Gen, das für ein bei der Bildung der Polymerpartikel beteiligtes Protein codiert, so aus, dass es für die phaA-Thiolase, die phaB-Ketoacyl-Reduktase oder die phaC-Synthase aus *Ralstonia eutropha* codiert. Durch das Einbringen dieser zusätzlichen Gene wird die Zelle dazu in die Lage versetzt, Proteine zu produzieren, die ihr die Bildung der Polymerpartikel ermöglichen. Durch gezieltes auswählen des mindestens einen weiteren Gens, das für ein bei der Bildung der Polymerpartikel beteiligtes Protein codiert, kann auch die spätere Zusammensetzung der Polymerpartikel beeinflusst werden. Gene, die für Proteine codieren, die am Stoffwechselweg zur Bildung der Polymerpartikel beteiligt sind, können unterschiedliche Substratspezifität besitzen, unterschiedliche Reaktionsprodukte bilden oder Abzweigungen im Stoffwechselweg blockieren, um so die Substrate und Moleküle, die an der Bildung der Polymerpartikel beteiligt sind, gezielt zu beeinflussen.

Bei Mikroorganismen, wie den in Beispiel 4 beschriebenen Mutanten der Gattung *E. coli,* die einen veränderten Fettsäuremetabolismus aufweisen, benötigt man neben dem zumindest einem induzierbaren Gen, das für ein Protein codiert, das die Größe der Polymerpartikel kontrolliert, nur die Polymer-Synthase, um die Herstellung der Polymerpartikel zu ermöglichen. Je nach dem welchen Organismus man verwendet, können weiter Gene in die Zelle eingebracht werden, um die Herstellung der Polymerpartikel unter den gegebenen Bedingungen zu ermöglichen. Enthält die Zelle nicht alle für die Bildung der Polymerpartikel erforderlichen Gene, so kann die Herstellung der Polymerpartikel trotzdem erfolgen, wenn man die durch die fehlenden Proteine hergestellten Zwischenprodukte der Zelle über das Nährmedium zuführt. Für die Bildung der Polymerpartikel ist jedoch immer mindestens eine Polymer-Synthase erforderlich.

Durch die Steuerung der Zusammensetzung der Polymerpartikel können ihre Eigenschaften beeinflusst werden. Durch die Beeinflussung ihrer Eigenschaften kann man zum Beispiel die Geschwindigkeit der biologischen Abbaubarkeit der Polymerpartikel beeinflussen. Neben der bereits zuvor geschilderten Möglichkeit, durch die gezielte Auswahl der in die Zelle eingebrachten weiteren Gene, die für ein bei der Bildung der Polymerpartikel beteiligtes Protein codieren, ist zur Beeinflussung der Zusammensetzung der sich bildenden Polymerpartikel *in vivo* besonders bevorzugt, das Einbringen von zumindest einem zusätzlichen Gen in die Zelle, das für eine Thiolase und/oder eine Polymer-Synthase codiert. Die unterschiedliche Substratspezifität der Thiolasen und der Polymer-Synthasen führt zu unterschiedlichen Zwischen- und Endprodukten und damit zu einer anderen Zusammensetzung des gebildeten Polymerkerns des Partikels.

Das Herstellungsprinzip dieser Polymerpartikel wird anhand der Abbildung 2 beispielhaft verdeutlicht. Aktivierte Vorstufen für die Biosynthese der Polymere können grundsätzlich von dem zentralen Metaboliten Acetyl-CoA oder von Intermediaten der primären Stoffwechselwege Citrat-Zyklus, Fettsäure-β-Oxidation und Fettsäure-*de novo*-Biosynthese, sowie dem Stoffwechsel von Aminosäuren abgeleitet werden. Dienen Fettsäuren als Kohlenstoffquelle so werden über die Fettsäure-β-Oxidation Intermediate (Acyl-CoA, insbesondere 3-Hydroxyacyl-CoA) bereitgestellt, die als aktivierte Vorstufen für die PHA-Biosynthese dienen.

Die Kontrolle der Partikelgröße erfolgt, indem man das zumindest eine induzierbare Gen, das für ein Protein codiert, das die Größe der Polymerpartikel kontrolliert, aus der Familie der Phasin-ähnlichen Proteine ableitet und bevorzugt aus der Gruppe auswählt, die das Phasin-Gen phaP aus *Ralstonia eutropha* und das Phasin-Gen phaF aus *Pseudomonas oleovorans* umfasst. Phasine sind amphiphile Proteine mit einem Molekulargewicht von 14 bis 28 kDa, die fest an die hydrophobe Oberfläche der Polymerpartikel binden.

Polymerpartikel mit unterschiedlicher Zusammensetzung des sie bildenden Polymers weisen unterschiedliche mechanische Eigenschaften auf und setzen biologisch aktive Substanzen, insbesondere pharmazeutische Wirkstoffe, unterschiedlich schnell frei. Polymerpartikel zum Beispiel, die aus C6-C14 3-Hydroxyfeltsäuren zusammengesetzt sind, zeigen infolge der niedrigen Kirstallinität des Polymers eine höhere Abbaurate des Polymers auf. Für gewöhnlich verringert eine Erhöhung des molaren Verhältnisses der Polymerbestandteile mit größeren Seitenketten am Polymerrückgrat die Kristallinität und führt zu erhöhten elastomeren Eigenschaften. Durch die Kontrolle der Polymerzusammensetzung gemäß dem in der Offenbarung beschriebenen Verfahren kann somit die biologische Abbaubarkeit der Polymerpartikel beeinflusst werden und damit auch die Freisetzungsrate für biologisch aktive Substanzen, insbesondere pharmazeutischen Wirkstoffe.

Vorzugsweise bringt man in das Kulturmedium als Substrat für die Bildung der Polymerpartikel, mindestens eine Fettsäure mit funktionellen Seitengruppen und besonders bevorzugt mindestens eine Hydroxyfettsäure und/oder mindestens eine Mercaptofettsäure und/oder mindestens eine β-Aminofettsäure ein. Unter "Fettsäuren mit funktionellen Seitengruppen" sind gesättigte oder ungesättigte Fettsäuren zu verstehen. Darunter fallen auch Fettsäuren, die funktionelle Seitengruppen enthalten, die ausgewählt werden aus der Gruppe, die Methylgruppen, Alkylgruppen, Hydroxylgruppen, Phenylgruppen, Sulfhydrylgruppen, primäre, sekundäre und tertiäre Aminogruppen, Aldehydgruppen, Ketogruppen, Ethergruppen, Carboxylgruppen, O-Estergruppen, Thioestergruppen, Carbonsäureamidgruppen, Halbacetalgruppen, Acetalgruppen, Phosphatmonoestergruppen und Phosphatdiestergruppen umfasst.

Der Einsatz der Substrate richtet sich nach der gewünschten Zusammensetzung und den gewünschten Eigenschaften der Polymerpartikel, die sowohl genetisch durch den Einsatz verschiedener Gene, die für Proteine mit unterschiedlicher Substratspezifität codieren als auch durch die verwendeten Zusatzstoffe, Substrate und Reaktionsbedingungen, die im Kulturmedium vorliegen, beeinflusst werden.

Um eine noch genauere Kontrolle der Größe der sich bildenden Polymerpartikel zu erlangen, gibt man das Substrat dem Kulturmedium in einer solchen Menge zu, dass es ausreichend bemessen ist, um die Kontrolle der Größe der Polymerpartikel zu gewährleisten. Dadurch ist eine zusätzliche Möglichkeit gegeben, die Steuerung der Partikelgröße noch wirksamer zu kontrollieren.

Für die Bildung der Polymerpartikel nach dem erfindungsgemäßen Verfahren wählt man den verwendeten Mikroorganismus aus der Gattung aus, die *Ralstonia, Alcaligenes, Pseudomonas* und *Halobiforma* umfasst. Bevorzugt wählt man den verwendeten Mikroorganismus aus der Gruppe aus, die *Ralstonia eutropha, Alcaligenes latus, Escherichia coli, Pseudomonas fragi, Pseudomonas putida, Pseudomonas oleovorans, Pseudomonas aeruginosa, Pseudomonas fluorescens,* und *Halobiforma haloterrestris* umfasst. Diese Gruppe umfasst sowohl Mikroorganismen, die von Natur aus in der Lage sind, biokompatible, biologisch abbaubare Polymerpartikel herzustellen, als auch Mikroorganismen, wie zum Beispiel *E. coli,* die dazu aufgrund ihrer genetischen Ausstattung nicht in der Lage sind. In die zuletzt genannten Mikroorganismen bringt man die dafür erforderlichen Gene nach dem erfindungsgemäßen Verfahren ein, um sie zur Herstellung der Polymerpartikel zu befähigen. Prinzipiell kann man mit dem oben beschriebenen Verfahren jeden kultivierbaren Mikroorganismus für die Herstellung von Polymerpartikel verwenden, auch wenn er die für die Polymerpartikelbildung erforderlichen Substrate aufgrund eines anderen Stoffwechsels nicht herstellen kann. In solchen Fällen gibt man die erforderlichen Substrate dem Kulturmedium zu, die dann von den Proteinen, die von den Genen exprimiert worden sind, die in die Zelle eingebracht worden sind, zu Polymerpartikeln umgesetzt werden.

Um die Polymerpartikel aus den Zellen zu erhalten, schließt man die kultivierten Mikroorganismen auf an sich bekannte Weise auf und trennt die Polymerpartikel anschließend von den Zellresten ab. Um den Größenbereich der so gewonnenen Polymerpartikel noch weiter einzuengen, können Standardverfahren, wie z.B. eine Ausschlusschromatographie oder eine Dichtegradientenzentrifugation, zur Selektion der Polymerpartikel der gewünschten Größe eingesetzt werden.

Die aus Proteinen und Lipiden bestehende Hüllmembran der erfindungsgemäß hergestellten Polymerpartikel kann man ebenfalls modifizieren, um den Partikeln günstigere Eigenschaften für den Transport der Wirkstoffe im tierischen Körper zu verleihen. Dafür trennt man eine sich auf der Oberfläche der Polymerpartikel befindliche Lipidschicht von den gemäß dem erfindungsgemäßen Verfahren erhaltenen Polymerpartikeln ab, und ersetzt sie durch eine Lipidschicht anderer Zusammensetzung.

Beim Ersetzen der Lipidschicht durch eine Lipidschicht anderer Zusammensetzung sind die Eigenschaften der neuen Lipidschicht von Bedeutung, die den Transport der Polymerpartikel über eine biologische Membran beeinflussen. Wenn die Lipidschicht der Hüllmembran mit der Lipidschicht der Zielmembran übereinstimmt, kann eine bessere Aufnahme von Partikeln beobachtet werden (Fernart, L. et al., J. Pharmacol. Exp. Ther. 1999, Vol.291(3), S.1017-1022). Deshalb kann die Lipidschicht der Polymerpartikel entfernt werden und durch eine Lipidschicht anderer Zusammensetzung ersetzt werden. Dies geschieht vorzugsweise durch eine Aceton-Extraktion oder es werden Phospholipasen oder nichtdenaturierende Detergentien verwendet. Anschließend wird eine Mischung der entsprechenden amphiphilen Moleküle zu den nun nahezu Lipid-freien Polymerpartikeln gegeben, um eine Lipidschicht gewünschter Zusammensetzung zu erhalten. Die neue Lipidschicht besteht dabei bevorzugt aus einer Mischung von amphiphilen Molekülen, die man aus der Gruppe auswählt, die Phospholipide und Etherlipide umfasst.

Neben den Lipiden kann man durch den Einsatz der oben genannten Detergenzien auch die sich auf der Oberfläche der Polymerpartikel befindlichen Proteine, bis auf die Polymer-Synthase, entfernen und durch funktionalisierte Proteine ersetzen. Dadurch kann man nackte Polymerpartikel für eine Vielzahl von Modifikationsmöglichkeiten bereitstellen, die im Weiteren noch beschrieben werden. Für diese Art der Modifikation eignen sich besonders Polymerpartikel, die aus dem Mikroorganismus *H. haloterrestris* gewonnen werden.

Je nach späterer Anwendung der Polymerpartikel steuert man die Partikelgröße durch das zumindest eine induzierbare Gen so, dass die gebildeten Polymerpartikel einen Durchmesser von 10 nm bis 3 μm, bevorzugt 1 nm bis 900 nm und besonders bevorzugt 10 nm bis 100 nm haben. In einer besonderen Ausführungsform der vorliegenden Erfindung kann man diese Größenkontrolle auch durch die Steuerung der Verfügbarkeit eines Substrates im Kulturmedium oder durch eine Kombination beider Mechanismen erzielen.

Für die Funktionalisierung der nach dem erfindungsgemäßen Verfahren hergestellten Polymerpartikel ist es erforderlich, dass das im Verfahren eingebrachte zumindest eine induzierbare Gen, das für ein Protein codiert, das die Größe der Polymerpartikel kontrolliert, eine Polymerpartikelbindedomäne und zumindest eine Bindedomäne umfasst, wobei die zumindest eine Bindedomäne eine biologisch aktive Substanz und/oder ein Kopplungsreagenz binden kann. Besonders bevorzugt ist ebenfalls, dass das mindestens ein weitere Gen, das für ein bei der Bildung der Polymerpartikel beteiligtes Protein codiert, eine Polymerpartikelbindedomäne und zumindest eine Bindedomäne umfasst, wobei die zumindest eine Bindedomäne eine biologisch aktive Substanz und/oder ein Kopplungsreagenz binden kann.

Eine "Bindedomäne" bezeichnet den Teil des Proteins, den man durch genetische Modifikation des zuvor in die Zelle eingebrachten zumindest einen induzierbaren Gens und/oder des mindestens einen weiteren Gens, das für ein bei der Bildung der Polymerpartikel beteiligtes Protein codiert, erhält. Die Bindedomäne, die biologisch aktive Substanzen und/oder ein Kopplungsreagenz binden kann, wählt man aus der Gruppe aus, die Oligopeptide, Enzyme, Abzyme und nicht-katalytische Proteine umfasst. Bevorzugt umfasst diese Gruppe FLAG-Epitope oder mindestens ein Cystein. Diese Gruppen werden im experimentellen Teil auch als Funktionsproteine bezeichnet. Die codierende Sequenz für die in dieser Gruppe genannten Mitglieder wird in die codierende Sequenz des zuvor in die Zelle eingebrachten zumindest einen induzierbaren Gens und/oder des mindestens einen weiteren Gens, das für ein bei der Bildung der Polymerpartikel beteiligtes Protein codiert, eingefügt. Dadurch werden Proteine produziert, die nicht nur an der Bildung oder der Kontrolle der Bildung der Polymerpartikel beteiligt sind, sondern auch noch über ihre durch genetische Modifikation erhaltene Bindedomäne biologisch aktive Substanzen und/oder Kopplungsreagenzien binden können. Die Bindedomäne kann auch noch nach Herstellung der Polymerpartikel durch chemische Modifikation der auf der Oberfläche lokalisierten Proteine mit Kopplungsreagenzien erhalten werden (siehe Beispiel 8).

Ein "Kopplungsreagenz" in diesem Sinne ist eine anorganische oder organische Verbindung, die dazu geeignet ist, auf der einen Seite eine biologisch aktive Substanz oder weitere Kopplungsreagenzien und auf der anderen Seite die Bindedomäne an sich zu binden.

Dieser Aufbau ermöglicht es, multifunktionelle Polymerpartikel herzustellen, die für den Transport von biologisch aktiven Substanzen geeignet sind. Die "Polymerpartikelbindedomäne" besteht bevorzugt aus einem Teil eines Proteins, der es dazu befähigt, auf der hydrophoben Oberfläche der Polymerpartikel anzubinden. Die Polymerpartikeldomäne, die einen Teil eines auf der Oberfläche des Polymerpartikels gebundenen Proteins umfasst, wählt man dabei aus der Gruppe von Proteinen aus, die eine Polymer-Depolymerase, einen Polymer-Regulator, eine Polymer-Synthase und ein die Partikelgröße kontrollierendes Protein umfasst. Diese Proteine stammen bevorzugt aus Mikroorganismen, die in der Lage sind Polymerpartikel zu bilden, insbesondere solche aus der Gattung *Ralstonia, Alcaligenes* und *Pseudomonas.* Das die Partikelgröße kontrollierende Protein wird dabei bevorzugt aus der Familie der Phasin-ähnlichen Proteine abgeleitet und noch bevorzugter verwendet man dafür das Phasin aus *R. eutropha* und *P. oleovorans.*

Ein "Polymer-Regulator" im Sinne der Erfindung ist ein Protein, welches die Transkription der an der Bildung der Polymerpartikel beteiligten Gene phaA, phaB und phaC reguliert. Es wird durch Bindung an die Partikeloberfläche der Transkriptionsregulation entzogen. Ein Beispiel eines solchen Regulators ist der Phasin-Repressor (phaR) aus *R. eutropha,* der an den Promotor eines Phasin-ähnlichen Gens bindet, dessen Expressionsprodukt die Größe der sich bildenden Polymerpartikel reguliert und verhindert, dass das Gen abgelesen wird. Durch das Binden des Phasin-Repressors an der Oberfläche der sich bildenden Polymerpartikel wird diese Stelle auf dem Promotor freigegeben und die Transkription des dahinterliegenden Gens kann beginnen.

Die Nutzung der Bindedomäne einer Polymer-Synthase zur Bindung von Kopplungsreagenzien und/oder biologisch aktiven Substanzen, ergibt sich aus der hohen Stabilität der Bindung zwischen der Polymerpartikelbindedomäne dieses Proteins und dem Kern des Polymerpartikels. Der Erfinder hat überraschend herausgefunden, dass diese Bindung weder durch denaturierenden Reagenzien, wie z.B. Sodiumdodecylsulfat (SDS), Harnstoff, Guanidiumhydrochlorid oder Dithiothreitol, noch durch Verwendung azider Bedingungen vom Kern des biologisch abbaubaren Polymerpartikels gelöst werden kann. Bevorzugt wird dafür die Polymer-Synthase verwendet, die aus *R. eutropha, P. oleovorans, P. putida* oder *P. aeruginosa* abgeleitet wird.

Dabei ist es ein besonderer Vorteil des erfindungsgemäßen Verfahrens, dass die gentechnische Modifikation der auf der Oberfläche der Polymerpartikel bindenden Proteine nicht die Funktionalität des an der Bildung der Polymerpartikel beteiligten Proteine beeinflusst. So bleibt zum Beispiel die Funktionalität der Polymer-Synthase erhalten, wenn mit ihrem N-terminalen Ende ein Protein fusioniert wird und somit eine Bindedomäne zur Bindung von biologisch aktiven Substanzen und/oder Kopplungsreagenzien erzeugt wird. Sollte die Funktionalität eines Proteins durch die Fusion doch beeinträchtigt sein, kann durch die Anwesenheit eines weiteren Gens, das für ein Protein codiert, dass die gleiche Funktion ausführt und in einem aktiven Zustand vorliegt, dieser Mangel kompensiert werden.

Dadurch kann eine stabile Verbindung der über die Bindedomäne der Proteine, insbesondere der Polymer-Synthase, gebundenen biologisch aktiven Substanzen und/oder Kopplungsreagenzien an das Polymerpartikel gewährleistet werden.

Bei der gentechnischen Modifikation der Gene, die für Proteine codieren, die nach ihrer Expression an die Partikeloberfläche binden, können auch Gene mit unterschiedlichen Modifikationen in die Zelle eingebracht werden. Nach der Expression dieser Proteine mit ihren unterschiedlichen Bindedomänen und der Bildung der Polymerpartikel kann so eine Multifunktionalisierung der Partikeloberfläche über die unterschiedlichen Bindedomänen erfolgen. Mit Hilfe dieses Verfahrens wird eine einfache und effiziente Massenproduktion von funktionalisierten Polymerpartikeln ermöglicht.

Für die nachträgliche Funktionalisierung der auf der Oberfläche der Polymerpartikel gebundenen Proteine verwendet man Kopplungsreagenzien, die man bevorzugt aus der Gruppe auswählt, die Bis-(2-oxo-3-oxazolydinyl)-Phosphonchlorid (BOP-C1), Bromo-tris-pyrrolidino-phosphoniumhexa-fluorophosphat (PyBroP), Benzotriazole-1-yl-oxy-tris-pyrrolidino phosphoniumhexafluorophosphat (PyBOP), N-Hydroxysuccinimid-Biotin, 2-(1H-benzotriazole-1-yl)-1,1,3,3-tetramethyluroniumhexafluorophosphat (HBTU), Dicyclohexyl-carbodiimide, Disuccinimidylcarbonat, 1-(3-dimethylaminopropyl)-3-ethylcarbodiimid (EDC), Bis-(2-oxo-3-oxazolydinyl)-phosphin, Diisopropylcarbodiimide (DIPC), 2-(1H-benzotrioxazolyl)-1,1,3,3-tetramethyluroniumtetrafluoroborat (TBTU), 2-(5-norboren-2,3-dicarboxyimido)-1,1,3,3-tetramethyluroniumtetrafluoroborat (TNTU), para-Nirophenylchloroformate, und O-(N-succinimidy)-1,1,3,3- tetramethyluronium tetrafluoroborate (TSTU) umfasst.

Als biologisch aktiven Substanzen verwendet man bevorzugt, Pestizide, Herbizide, pharmazeutisch aktive Substanzen und Proteine.

Die pharmazeutisch aktiven Substanzen wählt man aus der Gruppe aus, die Dideoxyinosin, Floxuridin, 6-Mercaptopurin, Doxorubicin, Daunorubicin, 1-Darubicin, Cisplatin, Methotrexat, Taxol, Antibiotika, Anticoaguranzien, Germizide, Antiarrythmische Agenzien und Wirkstoffvorstufen und -derivate der angeführten Wirkstoffgruppen umfasst.

Die Proteine wählt man bevorzugt aus der Gruppe aus, die Insulin, Calcitonin, ACTH, Glucagon, Somatostatin, Somatotropin, Somatomedin, Parathyroidhormon, Erythropoietin, hypothalamische Freisetzungfaktoren, Prolactin, Thyroid-stimulierendes Hormon, Endorphine, Enkephaline, Vasopressine, nicht natürlich vorkommende Opiate, Superoxiddismutase, Antikörper, Interferone, Asparaginase, Arginase, Arginindeaminase, Adenosindeaminase, Ribonuclease, Trypsin, Chymotrypsin und Pepsin umfasst.

Ein weiterer Aspekt der Erfindung ist ein Verfahren zur *in vitro* Herstellung von biologisch abbaubaren Polymerpartikeln, wobei das Verfahren das Bereitstellen einer für die Polymerpartikelbildung geeigneten Lösung mit zumindest einem Substrat, das Einbringen eines Proteins in die Lösung, das dazu geeignet ist, die Größe der Polymerpartikel zu kontrollieren und das Einbringen mindestens eines weiteren Proteins, das an der Bildung der Polymerpartikel beteiligt ist, umfasst. Auch das *in vitro* Verfahren bietet den Vorteil, das die Größe der Polymerpartikel schon während der Herstellung der Polymerpartikel gesteuert werden kann und eine spätere kostenaufwendige und zeitraubende Größenbestimmung und Auftrennung der gebildeten Polymerpartikel in einzelne Größenklassen vermieden wird.

Dabei setzt man für das mindestens eine weitere Protein, das an der Bildung der Polymerpartikel beteiligt ist, eine Polymer-Synthase ein, wobei die Polymer-Synthase ein biologisch aktives Protein umfassendes Fusionsprotein ist, oder wobei die Polymer-Synthase ein Fusionsprotein ist, das dazu in der Lage ist, sich direkt oder über ein Kopplungsreagenz an eine biologisch aktive Substanz zu binden, wobei man diese olymer-Synthase bevorzugt aus der Gruppe auswählt, welche die Polymer-Synthase aus *R. eutropha, P. oleovorans, P. putida* und *P. aeruginosa* umfasst.

Im Gegensatz zur *in vivo* Synthese ist eine *in vitro* Synthese, bei der im Labor aus Mikroorganismen isolierten Proteine und Enzyme verwendet werden, normalerweise sehr kostenintensiv, da sowohl die Enzyme und teilweise auch die Enyzmsubstrate zuvor isoliert und gereinigt werden müssen. In einer besonderen Ausführungsform der vorliegenden Erfindung zur *in vitro* Synthese der biokompatiblen, biologisch abbaubaren Polymerpartikel gibt man der für die Polymerpartikelbildung geeigneten Lösung mindestens eine Fettsäure, besonders bevorzugt eine β-Mercaptosäure und/ oder eine β-Aminofettsäure und eine Acyl-CoA Oxidase oder andere oxidierende und aktivierende Enzyme für die Bildung der Polymerpartikel zu. Durch die Verwendung dieser Substrate anstelle der R/S-3-Hydroxyfettsäuren und der Acyl-CoA Synthetase entsteht ein CoA-Wiederverwertungssystem, in dem die Acyl-CoA Oxidase die Fettsäure aktivieren und oxidieren wird, während sie CoA verbraucht und ATP hydrolysiert. Während der Polymerisierung spaltet die Polymer-Synthase CoA ab, das dann wiederum von der Acyl-CoA Oxidase verwendet werden kann. Die Kosten dieses *in vitro* Verfahrens lassen sich dadurch merklich verringern.

Ein weiterer Vorteil des *in vitro* Verfahrens zur Herstellung der Polymerpartikel ist, dass man das zumindest eine Substrat der für die Polymerpartikelbildung geeigneten Lösung in einer solchen Menge zugibt, dass es ausreichend bemessen ist, um die Kontrolle der Größe der Polymerpartikel zu gewährleisten.

Außerdem kann die Größe der sich bildenden Polymerpartikel auch noch dadurch kontrolliert werden, indem man die Polymer-Synthase der Lösung in einer solchen Menge zugibt, die ausreichend ist, um die Kontrolle der Größe des sich bildenden Polymerpartikel zu gewährleisten. Wie beim *in vivo* Verfahren gibt es also auch im *in vitro* Verfahren weitere Möglichkeiten, die Steuerung der Polymerpartikelgröße noch genauer zu kontrollieren und damit die Ausbeute an Polymerpartikeln der gewünschten Größe zu erhöhen und damit das Verfahren effizienter und kostengünstiger zu gestalten.

Die eigentliche Kontrolle der Partikelgröße erfolgt beim *in vitro* Verfahren allerdings durch Einbringen eines die Größe der Polymerpartikel kontrollierenden Proteins in die Lösung, das man aus der Familie der Phasin-ähnlichen Proteine ableitet und bevorzugt aus der Gruppe auswählt, die das Phasin aus *Ralstonia eutropha* und das Phasin aus *Pseudomonas oleovorans* umfasst.

Durch die Wahl des zumindest einen Substrates als auch der verwendeten Enzyme ist es, wie beim *in vivo* Verfahren auch, möglich, die Zusammensetzung des Polymers zu regulieren und dadurch Polymerkerne mit unterschiedlichen Eigenschaften zu gewinnen. Bei Verwendung von mehr als einem Substrat, können zum Beispiel je nach Art der aus den Enzymen gebildeten Polymeren, Polymerpartikel mit unterschiedlicher Zusammensetzung des Polymerkerns gewonnen werden. Wie bereits weiter oben beschrieben verleihen die dadurch hergestellten Polymere dem Polymerpartikel unterschiedlichste Eigenschaften.

Im *in vitro* Verfahren zur Herstellung der Polymerpartikel gibt man zur Kontrolle der Zusammensetzung der Lipidschicht auf der Oberfläche des Polymerpartikels mindestens ein amphiphiles Molekül aus der Gruppe der Phospholipide und Etherlipide zur Lösung hinzu (Ohne zusätzliche amphiphile Moleküle enstehen in vitro Partikel, die nur von Proteinen umgeben sind, was einen weiteren Partikeltyp darstellt.). So lassen sich zum Beispiel Polymerpartikel mit einer spezifischer Oberflächenladung herstellen, die speziell auf die später zu überwindende biologische Membran im tierischen Körper abgestimmt ist. Der Vorteil bei der Modifikation der Lipidschicht der Hüllmembran im *in vitro* Verfahren ist, dass die nachträgliche Modifikation der Lipidschicht, wie beim *in vivo* Verfahren, entfällt. Da die amphiphilen Moleküle, wie zum Beispiel Phospholipide oder Etherlipide, bereits zur Ausgangslösung zugegeben werden, wird von Anfang an eine Lipidschicht mit gewünschter Zusammensetzung erzeugt.

Ein weiterer Aspekt des *in vitro* Verfahrens, der auch im *in vivo* Verfahren zur Anwendung kommt, ist, dass man der für die Polymerpartikelbildung geeigneten Lösung mindestens eine pharmazeutisch aktive Substanz zugibt. Diese wird während der Polymerpartikelsynthese in das Polymerpartikel eingebaut und kann später im tierischen Körper über Diffusion durch die Partikelmatrix oder durch Abbau des Polymerpartikels freigesetzt werden. Letztgenannte Variante hat bei den durch das erfindungsgemäße Verfahren hergestellten Polymerpartikeln den weiteren Vorteil, dass die Geschwindigkeit des biologischen Abbaus der Polymerpartikel durch die oben beschriebene Steuerung der Zusammensetzung des Polymerkerns reguliert werden kann. Dadurch ist eine kontinuierliche Freisetzung des Wirkstoffes über einen bestimmten Zeitraum möglich.

Neben dem Einbau von Wirkstoffen in das wachsende Polymerpartikel, erfolgt die Funktionalisierung, wie beim *in vivo* Verfahren, dadurch, dass man mindestens eines der in die für die Polymerpartikelbildung geeigneten Lösung eingebrachten Proteine so auswählt, dass das mindestens eine eingebrachte Protein eine Polymerpartikelbindedomäne und zumindest eine Bindedomäne umfasst, wobei die zumindest eine Bindedomäne eine biologisch aktive Substanz und/oder ein Kopplungsreagenz binden kann. Die hier verwendeten Proteine für die Bildung der Polymerpartikel im *in vitro* Verfahren kann man aus dem weiter oben beschriebenen *in vivo* Verfahren gewinnen und sie weisen dann, je nach Art ihrer oben beschriebenen Herstellungsart, die entsprechenden Eigenschaften auf. Sie werden dann in die für die Polymerpartikelbildung geeigneten Lösung gegeben und sind dort an der Bildung der Polymerpartikel beteiligt. Wie im *in vivo* Verfahren kann man die so hergestellten Polymerpartikel auch nach ihrer Herstellung noch durch nachträgliche Modifikation mit den bereits weiter oben beschriebenen Kopplungsreagenzien modifizieren oder durch Zugabe von biologisch aktiven Substanzen, die an die Bindedomäne der Proteine binden, die auf der Oberfläche der Polymerpartikel gebunden worden sind.

Die Erfindung umfasst des weiteren ein Polymerpartikel mit definierter Größe, mit einer Oberflächenschicht aus amphiphilen Molekülen und wenigstens einer Polymer-Synthase, bei der es sich um ein Fusionsprotein handelt, das ein biologisch aktives Protein umfasst, oder wobei es sich bei der wenigstens einen Polymer-Synthase um ein Fusionprotein handelt, das dazu in der Lage ist, sich direkt oder über ein Kopplungsreagenz an eine biologisch aktive Substanz zu binden, , in die mindestens ein Protein, das aus der Gruppe ausgewählt wird, die eine Polymer-Depolymerase, einen Polymer-Regulator, eine Polymer-Synthase und ein die Partikelgröße beeinflussendes Protein umfasst, wobei das mindestens eine Protein eine Polymerpartikelbindedomäne und eine Bindedomäne, die eine biologisch aktive Substanz und/oder ein Kopplungsreagenz binden kann, umfasst und das in einer bevorzugten Ausführungsform nach dem oben beschriebenen Verfahren hergestellt wird.

Aufgrund ihrer vorteilhaften Eigenschaften eignen sich die Polymerpartikel der vorliegenden Erfindung besonders für die Herstellung eines Arzneimittels, eines Pestizids oder eines Herbizids, wobei das Arzneimittel bevorzugt für die Behandlung von Erkrankungen des zentralen Nervensystems geeignet ist. Durch die durch das vorliegende Verfahren beschriebenen Modifikationsmöglichkeiten der Polymerpartikel werden die Voraussetzungen für die Überwindung der BHS gegeben.

Die Kontrolle der Partikelgröße, die Steuerung der Zusammensetzung der Hüllmembran und insbesondere auch die Funktionalisierung der Partikeloberfläche, machen diese biologisch abbaubaren Polymerpartikel zu einem geeigneten Transportvehikel für biologisch aktive Substanzen aller Art und ermöglichen außerdem auch den zielgerichteten Transport der Polymerpartikel an ihren Wirkort. Die Multifunktionalisierung erlaubt zum Beispiel sowohl das gleichzeitige Anbinden von mindestens einer pharmazcutischen aktiven Substanz an die Partikeloberfläche als auch eines Antikörpers, der aufgrund seiner Bindungsspezifität eine genaue Ansteuerung des Zielortes ermöglicht. Diese und weitere Vorteile werden anhand der folgenden Ausführungsbeispiele näher dargestellt.

## Beschreibung der Figuren

Abbildung 1 stellt eine schematische Übersicht eines *in vivo* hergestellten biologisch abbaubaren Polymerpartikels und der Proteine und Lipide dar, die an die Oberfläche gebunden sind.
Dabei beziehen sich die Abkürzungen auf die folgenden Proteine:

A: Polymer-Depolymerase
B: Phasin (Name des codierenden Gens bei *R. eutropha:* phaP, bei *P. oleovorans*: phaF)
C: Polymer-Synthase
D: Phospholipid
E: Polymer-Regulatoren (z.B. der Phasin-Repressor (PhaR aus R. eutropha)

Abbildung 2 stellt exemplarisch die Synthese eines der möglichen biologisch abbaubaren Polymere in *R. eutropha* dar. Das einfache Polyhydroxyalkanoat, Polyhdroxybuttersäure (PHB), wird ausgehend von dem Stoffwechselintermediat Acetyl-CoA in einem dreistufigen Prozess hergestellt. Die sich wiederholende C4-Einheit in PHB ist die β-Hydroxybuttersäure. Der letzte Schritt der Synthese führt zur Bildung des Polymerpartikels, auf deren Oberfläche die Polymer-Synthase gebunden ist.

Abbildung 3 und 4 stellen die Ergebnisse aus den *in vivo* Experimenten dar. Abbildung 3 beschreibt dabei das Verhalten des Polymerpartikeldurchmessers bei verstärkter Phasinexpression (Erhöhung der Induktormenge in der Lösung), während Abbildung 4 das Verhalten der Polymerpartikelanzahl in der Zelle bei verstärkter Phasinexpression (Erhöhung der Induktormenge in der Lösung) darstellt.

Abbildung 5 und 6 stellen die Ergebnisse der *in vitro* Experiment dar. Abbildung 5 beschreibt den Einfluß des Mengenverhältnisses von Phasin zur Polymer-Synthase auf den Polymerpartikeldurchmesser, während in Abbildung 6 der Einfluss des Verhältnisses von Substrat zur Polymer-Synthase auf den Polymerpartikeldurchmesser dargestellt wird.

Abbildung 7 stellt den Vektor pBBad22K (Sukchawalit, R. et al., FEMS Microbiol Lett. 1999, Vol.181(2), S.217-223) dar, der als Ausgangsplasmid zur Konstruktion der Plasmide pBBad-P (trägt das Gen phaP aus *R. eutropha*) und pBBad-F (trägt das Gen phaF aus *P. oleovorans*) verwendet wird.

Abbildung 8 stellt den Vektor pBBad-P dar, der das Gen phaP für die Expression des die Partikelgrößebestimmenden Proteins Phasin aus *R. eutropha* trägt.

Abbildung 9 stellt das die Polymer(Polyhydroxybuttersäure)-Speicherung vermittelnde Plasmid pBHR68 (Spiekermann, P. et al., Arch. Microbiol. 1999, Vol. 171, S.73-80) dar, dass die Gene $phB_{Re}$, $phbA_{Re}$ und $phbC_{Re}$ aus Ralstonia eutropha trägt, die das Biosyntheseoperon für die Expression der phaA-Thiolase, phaB-Ketoacyl-Reduktase und der phaC-Synthase bilden.

Abbildung 10 stellt das die Polymer(Polyhydroxyalkanoate)-Speicherung vermittelnde Plasmid pBHR71 (Langenbach, S. et al., FEMS Microbiol. Lett. 1997, Vol.150, S.303-309) dar, welches das Gen phaC1 für die Expression der Polymer-Synthase trägt.

Abbildung 11 stellt den Vektor pBBad-F dar, der das Gen phaF für die Expression des die Partikelgrößebestimmenden Phasin-ähnlichen Proteins aus *P. oleovorans* trägt.

Abbildung 12 stellt den Vektor pBHR71-Cys dar, der ausgehend von dem Plasmid pBHR71 aus Abbildung 10 konstruiert worden ist. Das Gen phaC1, dass für die Polymer-Synthase codiert, trägt in dieser Konstruktion zwei Cystein-Reste am N-Terminus, um die direkte Anbindung von biologisch aktiver Substanzen oder die Anbindung von biologisch aktiven Substanzen über Kopplungsreagenzien, zu ermöglichen.

Abbildung 13 stellte den Vektor pBHR71-FLAG dar, der ebenfalls ausgehend von dem Plasmid pBHR71 aus Abbildung 10 konstruiert worden ist. Das Gen phaC1, dass für die Polymer-Synthase codiert, trägt in dieser Konstruktion zwei FLAG-Epitope am N-Terminus. Dies ermöglicht sowohl *in vivo* den Einbau von Gensequenzen für Funktionsproteine über die SpeI-Schnittstelle innerhalb der FLAG Gensequenz als auch die Anbindung von Kopplungsreagenzien und/oder biologisch aktiven Substanzen *in vitro* an die bereits exprimierte Polymer-Synthase. Die verwendeten biologisch aktiven Substanzen, die direkt oder über Kopplungsreagenzien gebunden sind, vermitteln die Bindung am Zielort (z.B. Zelloberfläche) oder eine biologische Aktivität, insbesondere eine enzymatische Aktivität.

Beispiel 1: Kontrolle der Größe der *in vivo* hergestellten biologisch abbaubaren Polymerpartikel

Beispiel 1.1: Herstellung von Polymerpartikeln in *R. eutropha*

Für die Herstellung der biologisch abbaubaren Polymerpartikel wird eine *"knock-out"* Mutante von *Ralstonia eutropha* (früher *Alcaligenes eutrophus*) York et al. (York, G.M. et al. J. Bacteriol. 2001, Vol.183, S.4217-4226) verwendet, die bezüglich des Gens, dass für die Expression des Oberflächenproteins auf den Polymerpartikeln Phasin codiert, einen defekt aufweist (phaP(-)), wodurch der Organismus nicht mehr in der Lage ist, dass durch das phaP-Gen codierte Phasin zu exprimieren. Des Weiteren können zur Herstellung der biologisch abbaubaren Polymerpartikel auch Mikroorganismen eingesetzt werden, welche dieses Gen und die weiteren für die Biosynthese von Polymerpartikeln erforderlichen Gene nicht enthalten. Beispiele für solche Mikroorganismen wären *Escherichia coli,* und *Halobiforma haloterrestris.* In einem Ausführungsbeispiel wird *Escherichia coli* (siehe Beispiel 2), das naturgemäß nicht in der Lage ist, die bereits zuvor beschriebenen biologisch abbaubaren Polymerpartikel herzustellen, verwendet.

Die zuletzt genannten Mikroorganismen werden dann mit einem Vektor transformiert, der im Falle von *R. eutropha* das phaP-Gen, das für Phasin codiert und die Promotorsequenz enthält. Dieses Gen wird von einem induzierbaren Promotor, vorzugsweise einem bad-Promotor kontrolliert, der durch Arabinose induziert wird.

Klonierungsschritte

Für die Klonierung wird die für phaP codierende DNA-Sequenz aus *R. eutropha* verwendet, die in der "GenBank" Datenbank mit der Nummer AF0791155 geführt wird (Hanley, S.Z. et al., FEBS Letters 1999, Vol.447, S.99-105). Diese wird in die NcoI/BamHI Schnittstelle des Vektors pBBad22K (Sukchawalit, R. et al., FEMS Microbiol Lett. 1999, Vol.181 (2), S.217-223, s.a. Abb. 7) eingefügt, der einen induzierbaren Promotor ($P_{BAD}$) enthält. Für die Klonierung werden die entsprechenden Restriktionsschnittstellen NcoI und BamHI durch PCR-Mutagenese mittels der Primer 5'-aaaggccc**ccatgg**tcctcaccccggaaca-3' (SEQ ID Nr.1, NcoI-Schnittstelle fettgedruckt) und 5'-aaaggcc**ggatcc**tcagggcactaccttcatcg-3' (SEQ ID Nr.2, BamHI-Schnittstelle fettgedruckt) in die für Phasin codierende Sequenz eingefügt. Das dadurch entstehende DNA-Fragment der SEQ ID Nr.3 wird mit den Restriktionsenzymen NcoI und BamHI hydrolysiert und in den ebenso hydrolysierten Vektor pBBad22K (Abb. 7) ligiert. Das nun die Nukleotidsequenz für das Protein Phasin enthaltende Plasmid wird im Folgenden als pBBad-P bezeichnet (Abb. 8). Dieses Plasmid wird mittels der für den entsprechenden Organismus im Stand der Technik beschriebenen Transformationstechniken in den Mikroorganismus übertragen. Der Induktor des bad-Promotors des pBBad-P ist Arabinose, der es ermöglicht, die Expression dieses Gens in den verwendeten Mikroorganismen quantitativ zu kontrollieren. Über diese Steuerungsmöglichkeit wird die Expression des Phasin-Gens kontrolliert und über die Menge des vorhandenen Phasins in der Zelle letztendlich die Größe der Polymerpartikel.

Beispiel 1.2: Erzeugung von Mikroorganismen für die Herstellung von Polymerpartikeln, die ursprünglich nicht zur Bildung von Polymerpartikeln befähigt waren

Bei Mikroorganismen, die ursprünglich nicht in der Lage waren Polymerpartikel dieses Typs zu erzeugen, enthält ein zusätzlicher oder der gleiche Vektor weitere Gene, die an der Bildung der Polymerpartikel beteiligt sind. Die Anzahl und Art der erforderlichen Gene, die für die Herstellung der Polymerpartikel in einem mikrobiellen Organismus eingebracht werden müssen, bestimmen sich nach der Grundausstattung des verwendeten Organismus. Im einfachsten Fall, z.B. bei *E. coli,* ist mindestens eine Thiolase, eine Reduktase und eine Polymer-Synthase notwendig, um Polymerpartikel auf dem in Abbildung 2 dargestellten Weg herzustellen. Werden Organismen verwendet, die spezifische Mutationen aufweisen, wie zum Beispiel der *E. coli* Stamm aus Beispiel 4, reichen auch weniger als die oben genannten Gene aus, um die Herstellung der Polymerpartikel zu ermöglichen. Das gleiche gilt, wenn dem Organismus bereits bestimmte Vorstufensubstrate aus dem Polymersynthesestoffwechsel zur Verfügung gestellt werden.

Falls ein weiteres Plasmid verwendet wird um die für die Bildung der Polymerpartikel notwendigen Gene in die Zelle einzubringen, eignet sich besonders das Plasmid pBHR68 (Abb. 9), welches das 5.2 kb SmaI/EcoRI Fragment aus der chromosomalen DNA aus *R. eutropha* enthält. Dieses Plasmid enthält das Biosyntheseoperon zur Herstellung der biologisch abbaubaren Polymerpartikel aus *R. eutropha* (Spiekermann, P. et al., Arch. Microbiol. 1999, Vol.171, S. 73-80).

Beispiel 1.3: Kontrolle der Partikelgröße über den pBBad-P Vektor

Die so modifizierten Mikroorganismen werden bei 30°C in Luria Broth-Medium inkubiert. Die Induktion des Promotors mit Arabinose erfolgt in der spätlogarithmischen Wachstumsphase. 24 h nach der Inkubation wird die Partikelgröße in den Zellen bestimmt. Dazu werden die Zellen durch Zentrifugation vom Nährmedium abgetrennt und aufgeschlossen. Danach erfolgt die mikroskopische Bestimmung der Polymerpartikelgröße mit Hilfe der Tunnelelektronenmikroskopie in Kombination mit einer analytischen Gelfiltrationschromatographie. Zur Bestimmung der Polymerpartikelanzahl werden die intakten Zellen mit einem Lichtmikroskop untersucht und die Anzahl der Polymerpartikel/Zelle ausgezählt.

Wie die Ergebnisse zeigen, ist es durch die Expressionskontrolle möglich, sowohl die Durchschnittsgröße der gebildeten Polymerpartikel (siehe Abb. 3) als auch ihre durchschnittliche Anzahl (siehe Abb. 4) in den einzelnen Zellen zu steuern. Durch kontrollierte Erhöhung der Kopienzahl von Phasin wird eine Abnahme des durchschnittlichen Polymerpartikeldurchmessers bei gleichzeitiger Zunahme der durchschnittlichen Anzahl der Polymerpartikel erreicht. So kann die Ausbeute der Polymerpartikel mit dem gewünschten Durchmesser deutlich erhöht werden. Dies ermöglicht eine schnellere und kostengünstigere Herstellung der Polymerpartikel.

Beispiel 1.3: Kontrolle der Partikelgröße über die Verfügbarkeit der Substrate

Ein weiterer Mechanismus zur Steuerung der Größe der Polymerpartikel ist die Regulation der Verfügbarkeit der Substrate oder der Polymer-Synthase, die für die Synthese der Polymerpartikel benötigt werden. Die Verfügbarkeit der Polymer-Synthase kann mittels Antisense-Technologie, durch genetische Regulation oder durch die Verfügbarkeit der Substrate, die für die Bildung der Polymer-Synthase notwendig sind, im Nährmedium gesteuert werden.

Als Beispiel für eine Regulation der Partikelgröße durch die Verfügbarkeit eines Substrates im Nährmedium dient ein Experiment, bei dem die Konzentration der vorhandenen Kohlenstoffquelle verringert wird, um so den Durchmesser der gebildeten Polymerpartikel zu steuern. Dazu wird ein *E. coli* Stamm, der das bereits zuvor genannte Plasmid pBHR68 (Abb. 9) mit dem Biosyntheseoperon zur Herstellung der biologisch abbaubaren Polymerpartikel aus *R. eutropha* enthält (Abb. 8), in M9-Medium mit 1.5 (w/v) Glucose bei 30°C herangezogen. Zu Beginn der stationären Wachstumsphase wird durch Zugabe von M9-Medium ohne Glucose die Konzentration der Kohlenstoffquelle auf 1/50 seines ursprünglichen Wertes verringert und die Mikroorganismen werden bei ansonsten gleichbleibenden Wachstumsbedingungen für weitere 20 h inkubiert. Nach Beendigung des Versuches enthalten die Mikroorganismen Polymerpartikel mit einem Durchmesser von durchschnittlich 130 nm.

Beispiel 2: Beeinflussung der Zusammensetzung der *in vivo* hergestellten Polymerpartikel

Um die Zusammensetzung des biologisch abbaubaren Polymerpartikels zu verändern, können neben dem für phaP-codierendem Gen weitere Gene, die für Enzyme codieren, die Substrate für die Synthase bereitstellen, insbesondere Thiolasen oder weitere Polymer-Synthasen, in die Zelle eingebracht werden. Dadurch ist die Zelle in der Lage, bei der Bildung der Polymerpartikel eine Reihe von unterschiedlichen Monomeren in die wachsende Polymerkette einzubauen und so Polymerpartikel herzustellen, dessen Kern aus unterschiedlich zusammengesetzten Polymeren besteht.

Als Beispiele seien hier verschiedene Polymer-Synthasen aufgezeigt, die aufgrund ihrer Substratspezifität sowohl im *in vivo* als auch im *in vitro* Verfahren unterschiedlich 3-Hydroxyfettsäuren (C4-C16) in die wachsende Polymerkette einbauen. Dabei können zum Beispiel die Polymer-Synthase aus *R. eutropha,* die biologisch abbaubare Polymerketten aus C4 Fettsäuren herstellt (C4), Polymer-Synthasen aus *Aeromonas punctata* (C4 und C6), *Thiocapsa pfennigii* (C4 und C8) und *P. aeruginosa* (C6 bis C14) verwendet werden. Auch das gleichzeitige Einbringen mehrerer Polymer-Synthasen in die Zelle, erlaubt es Polymerpartikel mit unterschiedlichsten Eigenschaften herzustellen. In einem weiterführende Ansatz können durch Zufallsmutagenese (*in vitro* evolution) auch neue Polymer-Synthasen mit veränderter Substratspezifität gewonnen werden.

Die Zusammensetzung der sich bildenden Polymerpartikel lässt sich auch noch auf anderem Wege beeinflussen. Die Bereitstellung von unterschiedlichen Kohlenstoffquellen, Vorstufen verschiedener Kohlenstoffquellen und Intermediaten des Stoffwechselweges, die zur Bildung der Polymerpartikel führt, haben ebenfalls Einfluss auf die Art des sich bildenden Polymers. Des Weiteren können die an der Bildung der Polymerpartikel beteiligten Stoffwechselwege durch Inhibitoren, Verwendung von *Knock-out* Mutanten der betreffenden Stoffwechselwege und der Expression von Enzymen, welche im Stoffwechselweg zur Bildung anderer Zwischen- oder Endprodukte fühlen, gesteuert werden.

Für die Beeinflussung des Fettsäurestoffwechsels werden die folgenden Enzyme eingesetzt, welche die Eigenschaft haben, die Zwischenprodukte des Fettsäurestoffwechsels zu modifizieren und so unterschiedliche Produkte, z.B. Fettsäuren mit unterschiedlichen Seitenketten, für die Bildung der Polymerpartikel zur Verfügung zu stellen: (R)-spezifische Enoyl-CoA Hydratasen, Transacylasen und Ketoacyl-CoA/ACP Reduktasen. Diese Enzyme haben eine unterschiedliche Spezifität im Bezug auf die Kettenlänge des für die Polymer-Synthase bereitgestellten Substrates (R)-Hydroxyacyl-CoA. Dadurch entstehen Bausteine im Polymer mit verschiedenen Seitenkettenlängen und somit Polymere unterschiedlicher Zusammensetzungen.

Die Beeinflussung des Stoffwechselweges kann aber nicht nur über das Einbringen von zusätzlichen Enzymen in die Zelle erfolgen sondern auch über den Zusatz von Inhibitoren des Stoffwechselweges zum Medium. Beispiele für solche Inhibitoren sind die Acrylsäure und Triclosan (Synonyme: TCC oder 5-Chlor-2-(2,4-dichlorphenoxy)-phenol), um nur einige zu nennen.

Beispiel 3: *In vivo* Herstellung von biologisch abbaubaren Polymerpartikeln aus (R)-3-Hydroxybuttersäure mit hoch kristallinem Kern

Für dieses Experiment wurde ein *E. coli* Stamm verwendet, der das bereits im Beispiel 1 genannte Plasmid pBBad-P und das Plasmid pBHR68 enthält. Die Kultivierung erfolgte unter den in Beispiel 1 genannten Bedingungen mit Glucose als Kohlenstoffquelle. Die gebildeten Polymerpartikel bestehen aus (R)-3-Hydroxybuttersäure und haben einen Durchmesser von 50 bis 500 nm, je nachdem wie viel Induktor (Arabinose) dem Medium zugegeben wird. Die Durchschnittliche Größe der so gebildeten Polymerpartikel schwankt dabei nur um etwa 20 bis 50 nm. Als Verdeutlichung der Kontrolleigenschaften, die durch das über das pBBad-P Plasmid eingeführte Phasin erzielt werden, wird das Experiment mit dem oben genannten *E. coli* Stamm ohne das pBBad-P Plasmid wiederholt. Hierbei werden Polymerpartikel mit einem Durchmesser von 150 - 250 nm erhalten. Die Steuerung der Partikelgröße über das Phasin ermöglicht im Vergleich zum Kontrollexperiment die Herstellung viel größerer aber vor allem auch viel kleinerer Polymerpartikel.

Eine weitere Fraktionierung der Polymerpartikel nach ihrer Größe kann anschließend durch im Stand der Technik bekannte Verfahren, wie z.B. der Ausschlußchromatographie, der Dichtegradientenzentrifugation, oder der Ultrafiltration in 5 mM Phosphatpuffer (pH 7.5) erfolgen.

Beispiel 4: *In vivo* Herstellung von biologisch abbaubaren Polymerpartikeln aus (R)-3-Hydroxyfettsäuren mit niedrig kristallinem Kern

In diesem Experiment werden biologisch abbaubare elastomere Polymerpartikel in *E. coli* hergestellt, deren Kerne aus (R)-3-Hydroxyfettsäuren bestehen. Die Polymerketten sind im Durchschnitt aus 6 bis 14 Kohlenstoffatomen aufgebaut. Dies wird durch die regulierte Expression des phaF-Gens aus *P. oleovorans* in *E. coli* erreicht, dass dem für Phasin codierendem Gen phaP aus *R. eutropha* sehr ähnlich ist und dem Gen für die Polymer-Synthase aus *P. aeruginosa* (phaC). In diesem Beispiel wird das Expressionsprodukt des Gens phaF zur Kontrolle der Partikelgröße eingesetzt. Die Polymerpartikel können in *E. coli*-Zellen mit verändertem Fettsäuremetabolismus alleine durch die Polymer-Synthase (phaC) der Pseudomonaden hergestellt werden (Langenbach, S. et al., FEMS Microbiol. Lett. 1997, Vol. 150, S.303-309). Der Fettsäuremetabolismus ist so verändert, dass bei Verwendung von Fettsäuren als Kohlenstoffquelle CoA-aktivierte Intermediate (z.B. Enoyl-CoA) der Fettsäure-β-Oxidation akkumuliert werden, die wiederum als Vorstufen der Polymersynthese dienen. Dafür werden fadB-Mutanten von *E. coli* verwendet (Langenbach, S. et al., FEMS Microbiol. Lett. 1997, Vol.150, S.303-309) oder es werden Inhibitoren eingesetzt, welche den Fettsäuremetabolismus entsprechend inhibieren (z.B. Acrylsäure; Qi, Q. et al., FEMS Microbiol. Lett. 1998, Vol.167, S.89-94). Die ansonsten benötigte Ketoacyl-Reduktase und die Thiolase sind bei Verwendung der Mutanten oder inhibierten Mikroorganismen nicht mehr erforderlich. *E. coli* eigene Enoyl-CoA-Hydratasen katalysieren dann die Bildung von R-3-Hydroxyacyl-CoA aus Enoyl-CoA. Dies wird dann von der Polymer-Synthase in Poly(R)-3-Hydroxyfettsäure umgewandelt, welches den Polymerkern des sich bildenden Polymerpartikels bildet.

Klonierung

Das phaF-Gen von *P. oleovorans,* dass bereits von Prieto et al. (Prieto, M.A. et al., J. Bacteriol. 1999, Vol.181 (3), S.858-868) beschrieben worden ist und in der "GenBank" Datenbank mit der Nummer AJ010393 geführt wird, wird über den Vektor pBBad-F (Abb. 11) in *E. coli* transformiert. Dieser Vektor wird ausgehend vom Vektor pBBad22K erhalten (Abb. 7). Die einzelnen Klonierungsschritte entsprechen dabei den im Beispiel 1 beschriebenen. Das phaF-Gen wird in die NcoI/BamHI Schnittstelle des Vektors pBBad22K kloniert. Die Primer, die zur PCR-Mutagenese des oben genannten phaF-Gens verwendet werden, haben die folgenden Sequenzen: 5'-aaaggg**ccatgg**ctggcaagaagaattccgagaa-3' (SEQ ID Nr. 4, NcoI-Schnittstelle fettgedruckt) und 5'-aaaggg**ggatcc**tcagatcagggtaccggtgcctgtctg-3' (SEQ ID Nr. 5, BamHI-Schnittstelle fettgedruckt). Das dadurch entstehende DNA-Fragment der SEQ ID Nr. 6 wird dann in das bereits beschriebene Plasmid pBBad22K kloniert. Das nun die Sequenz für phaF enthaltende Plasmid wird im folgenden als pBBad-F bezeichnet (Abb. 11). Zusätzlich zu diesem Plasmid wird auch das die Nukleotidsequenz für die Polymer-Synthase enthaltende Plasmid pBHR71 (Abb. 10; Langenbach, S. et al., FEMS Microbiol. Lett. 1997, Vol. 150, S.303-309) in *E. coli* transformiert.

Als Kohlenstoffquelle für die anschließende Expression, wird die Fettsäure Decansäure verwendet. Die Expression des Phasin phaF-Gens wird ebenfalls über den Induktor Arabinose kontrolliert. Der Versuch wird dabei, wie bereits in Beispiel 1 beschrieben, durchgeführt. Die so gebildeten Polymerpartikel haben je nach Menge des zuvor eingesetzten Induktors einen Durchmesser von 100-500 nm.

Beispiel 4: Kontrolle der Größe von *in vitro* hergestellten biologisch abbaubaren Polymerpartikel

Die Kontrolle der Größe der gebildeten biologisch abbaubaren Polymerpartikel wird auch bei der *in vitro* Herstellung durch die Verfügbarkeit der Polymer-Synthase, von Phasin oder Phasin-ähnlicher Proteine und die Verfügbarkeit der Substrate und Stoffwechselintermediate bestimmt.

Für den *in vitro* Ansatz zur Herstellung der biologisch abbaubaren Polymerpartikel, müssen zuerst die notwendigen Enzyme und Substrate zur Verfügung gestellt werden. Für diesen Versuch wird die rekombinante Polymer-Synthase aus *R. eutropha* oder *P. aeruginosa* verwendet (Gerngross, T.U. und Martin, D.P., Proc. Natl. Acad. Sci. USA 1995, Vol.92, S.6279-6283; Qi, Q. et al., Appl. Microbiol. Biotechnol. 2000, Vol. 54, S.37-43), die mittels Affinitätschromatographie (mit His-Tag-Fusion oder $Ni^{2+}$-NTA-Agarose) gereinigt werden. Analog dazu werden für die Kontrolle der Partikelgröße jeweils die Polymerpartikelgröße-bestimmenden Proteine aus *R. eutropha* bzw. *P. aeruginosa* aufgereinigt. Zur Expression dieses Proteins in *R. eutropha* wird der Vektor verwendet, der bereits im Beispiel 1 zur Expression des Phasin-Gens verwendet worden ist. Der Reaktionsansatz zur *in vitro* Herstellung der biologisch abbaubaren Polymerpartikel enthält neben der Polymer-Synthase und dem Polymerpartikelgröße-bestimmenden Protein Phasin weiterhin: R-3-Hydroxybutyryl-CoA oder R-3-Hydroxydecanoyl-CoA als Substrat für die Synthese der Polymerpartikel, 50 mM Phosphat-Puffer (pH 7.5), 1 mM $MgCl_2$ und zur Stabilisierung 5% Glycerol (v/v). Aufgrund der Verwendung von R-3-Hydroxybutyryl-CoA oder R-3-Hydroxydecanoyl-CoA als Vorstufenprodukte für die Polymer-Synthase ist der Einsatz der Thiolase und der Reduktase nicht erforderlich (s.a. Abb. 2).

Die Ergebnisse der Abb. 5 stellen den Einfluss des Mengenverhältnisses von Phasin zur Polymer-Synthase auf den Polymerpartikeldurchmesser dar, während Abb. 6 den Einfluss des Verhältnisses von Substrat zur Polymer-Synthase auf den Polymerpartikeldurchmesser darstellt. Diese Ergebnisse zeigen auch, dass sich die Regulation der Polymerpartikelgröße über die Menge der vorhandenen Polymer-Synthase regulieren lässt. Somit kann über das erfindungsgemäße Verfahren eine effektive Regulation der Partikelgröße erzielt werden.

Beispiel 5: Beeinflussung der Zusammensetzung der *in vitro* gebildeten Polymerpartikel

Beispiel 5.1: Beeinflussung der Polymerzusammensetzung

Um die Zusammensetzung des biologisch abbaubaren Polymerpartikels zu verändern, können dem Reaktionsansatz verschiedene Substrate zugegeben werden und/oder jeweils verschiedene Polymer-Synthasen mit unterschiedlichem Substratspektrum verwendet werden (s.a. Beispiel 2). Dadurch werden bei der Bildung der Polymerpartikel eine Reihe von unterschiedlichen Monomeren in die wachsende Polymerkette eingebaut und es werden Polymerpartikel mit unterschiedlicher Polymerzusammensetzung hergestellt. Durch die Verwendung der Polymer-Synthase aus *P. aeruginosa* werden R-3-Hydroxyfettsäure-Bausteine mit 6-14 C-Atomen in die wachsende Polymerkette eingebaut.

Wird z.B. nur ein Substrat angeboten, so werden bei Verwendung der Polymer-Synthase aus *P. aeruginosa* homopolymere Polymerpartikel gewonnen.

Weitere Beispiele, wie die Zusammensetzung der Polymerpartikel verändert werden kann, finden sich in Beispiel 2.

Beispiel 5.2: Beeinflussung der Membranzusammensetzung der Polymerpartikel

Für eine spätere Fusion mit der Membran einer Zelle, in der z.B. ein Wirkstoff eingebracht werden soll, bedarf es der Möglichkeit die Zusammensetzung des Phospholipidschicht auf der Oberfläche der biologisch abbaubaren Polymerpartikel zu kontrollieren. Diese Regulation erfolgt bei der *in vitro* Herstellung (Beispiel 4) der biologisch abbaubaren Polymerpartikel bereits bei der Bereitstellung der für die Polymerpartikelbildung geeigneten Lösung. Durch Zugabe eines Gemisches verschiedener amphipiler Moleküle, kann die Membran den entsprechenden Erfordernissen angepasst werden.

Zum *in vitro* Reaktionsansatz werden Phospholipide hinzugegeben. Eine definierte Anzahl von Molekülen der Polymer-Synthase sind nun an der Entstehung der Polymerpartikel beteiligt. Die erfindungsgemäßen Polymerpartikel werden mit zunehmender Polymerisation größer und die Polymer-Synthasen an der Oberfläche, können die Oberfläche der Polymerpartikel nicht mehr vollständig abschirmen. Folglich werden hydrophobe Bereiche (Polymer) exponiert, an die sich amphiphile Moleküle spontan anlagern.

Bei den *in vivo* hergestellten Partikeln muss zunächst die bereits vorhandene Phospholipidschicht entfernt werden. Dafür wird eine Aceton-Extraktion durchgeführt oder es werden Phospholipasen oder nichtdenaturierenden Detergentien verwendet, um die Phospholipidschicht zu zerstören. Danach wird, wie bereits oben beschrieben, eine Mischung der entsprechenden amphiphilen Moleküle zu den nun nahezu Lipid-freien Polymerpartikeln gegeben. Bevorzugt werden dabei negativ geladene Phospholipide oder Phosphatidylcholin verwendet. In einem Ausführungsbeispiel für die gezielte Steuerung der Membranzusammensetzung werden die Polymerpartikel in 1% Octylglycosid-haltigen PBS (pH 7.5) suspendiert und unter Rühren gegen einen Überschuss Phosphatidylcholin dialysiert. Die so erzeugte Partikeloberfläche eignet sich besonders gut für die Fusion mit Hirnkapillar-Endothelzellen (BCEC).

Beispiel 5.3: Einbau verschiedener Substanzen in die wachsenden Polymerpartikel

Mit Hilfe des lipophilen Fluoreszenzfarbstoffes Nilrot (Sigma, St. Louis, Mo. USA) bzw. Rhodamin 123 wurde bereits die Aufnahme von Substanzen in den Kern des biologisch abbaubaren Polymerpartikels qualitativ untersucht (Spiekermann, P. et al., Arch. Microbiol. 1999, Vo1.171, S.73-80). Wenn bei der *in vivo* oder der *in vitro* Herstellung, wie oben beschrieben, der Fluoreszenzfarbstoff Nilrot ins Medium oder dem Reaktionsansatz gegeben wird, kann eine Färbung der hergestellten Polymerpartikel beobachtet werden, d.h. die Färbung der Polymerpartikel tritt bereits während ihrer Synthese und sogar vor der Isolation aus der Zelle ein.

Durch Zugabe pharmazeutisch aktiver Wirkstoffe können diese in den Polymerkern des Partikels eingebaut werden. Zum Beispiel wird das unpolare anti-Tumormittel Paclitaxel bei einem *in vitro* Experiment zur Herstellung der Polymerpartikel zum Reaktionsansatz zugegeben. Das wasserunlösliche Paclitaxel löst und konzentriert sich im hydrophoben Polymer-Kern der Polymerpartikel. Paclitaxel wird der Lösung für die Bildung der Polymerpartikel dabei lediglich zugesetzt, woraufhin sich der Wirkstoff in den Partikeln konzentriert. Dies wird überprüft, indem nach der Bildung der Polymerpartikel diese aus dem Reaktionsansatz entfernt werden und die Lösung mittels HPLC auf die Anwesenheit von Paclitaxel untersucht wird. Die Abnahme der Paclitaxel-Konzentration in der Lösung zeigt dabei, dass es in die Polymerpartikel eingebaut worden ist. Mit dem biologischen Abbau der Polymerpartikel im Organismus erfolgt später die Freisetzung des Wirkstoffs. Als Kontrollversuch dient ein Reaktionsansatz ohne Polymerpartikel, wobei hier die Paclitaxel-Konzentration in der für die Polymerpartikelbildung geeigneten Lösung nicht abgenommen hat.

Beispiel 6: Funktionalisierung der Partikeloberfläche

Biologisch aktive Substanzen können an Proteine gebunden werden, die sich bereits auf der Oberfläche der Polymerpartikel befinden. Dafür kommen alle in Abbildung 1 genannten Proteine in Betracht. Dadurch ergeben sich eine Vielzahl von "*cross-linking*"-Strategien, die eine kovalente Verknüpfung der biologisch aktiven Substanz über Proteine, die an die Partikeloberfläche gebunden sind, ermöglichen.

Beispiel 6.1 Gezielte Modifikation eines Oberflächenproteins zur Anbindung eines pharmazeutischen Wirkstoffes

Ein Beispiel für die Funktionalisierung der Polymerpartikel ist die Anbindung von Hydrazongebundenem Doxorubicin (Hydrazon-gebundenes Doxorubicin wurde beschrieben von King, H.D. et al., Biconjugate Chem. 1999, Vor. 10, S.279-288) an die Polymer-Synthase PhaCl aus *P. aeruginosa,* die auf der Oberfläche der Polymerpartikel gebunden ist und die zwei N-terminal eingefügte Cystein-Reste enthält. Diese Cystein-Reste bilden die Bindedomäne der Polymer-Synthase, über die biologisch aktive Substanzen gebunden werden können.

Klonierung

Die Cystein-Reste codierenden Tripletts werden mittels PCR-Mutagenese in das für PhaC1 codierende Gen kloniert, dass dann in die XbaI und BamHI Schnittstelle des in Abbildung 10 dargestellten Plasmids pBHR71 kloniert wird. Bei dieser PCR-Mutagenese werden auch die Schnittstellen für XbaI und BamHI in das Gen eingefügt. Die Primer, die zur PCR-Mutagenese des für PhaC1 codierende Gens verwendet werden, haben die folgenden Sequenzen: Primer für den N-Terminus 5'-gggc**tctaga**aataaggagatatacatatgtgttgtaagaacaataacgagctt-3' (SEQ ID Nr.7, XbaI-Schnittstelle fettgedruckt, Cys-Tripletts unterstrichen) und Primer für den C-Terminus 5'-aaacgc**ggatcc**ttttcatcgttcatgca-3' (SEQ ID Nr.8, BamHI-Schnittstelle fettgedruckt). Das dadurch entstehende DNA-Fragment der SEQ ID Nr. 9 wird dann mit XbaI und BamHI hydrolysiert und in das analog hydrolysierte Plasmids pBHR71 ligiert. Das daraus resultierende Plasmid, genannt pBHR71-Cys (Abb. 12), wird mittels bekannter Techniken in *E. coli* transformiert, wo es dann die Bildung von Polymerpartikeln ermöglicht, die auf ihrer Oberfläche eine Polymer-Synthase tragen, die zwei Cystein-Reste für die Anbindung von diversen Substanzen, insbesondere pharmazeutischen Substanzen trägt.

Beispiel 6.1.1 Anbindung von Doxorubicin (Syn.: Hydroxyl daunorubicin)

Über diese Oberflächen-exponierten Cystein-Reste kann nun ein Hydrazon-vermitteltes *"cross-linking"* erfolgen. Dazu wird wie folgt vorgegangen: 100 mg des isolierten Polymerpartikels (Gesamtvolumen 1 ml) auf dem die Polymer-Synthase gebunden ist, werden mit Helium-begastem PBS (pH 7.5) und 5 mM Dithiothreitol (DTT) für 3 h bei 37°C inkubiert. Durch diese Behandlung werden die Disulfidbrücken in der Polymer-Synthase reduziert. Danach werden durch eine 30 min Zentrifugation bei 4°C und 40.000 x g die niedermolekularen Verbindungen entfernt. Dann werden die reduzierten Polymerpartikel mit 1 ml PBS-Puffer (pH 7.5), der 10 $\mu$mol des Hydrazon-gebundenen Doxorubicin enthält, suspendiert und für 30 min bei 4°C inkubiert. Danach findet eine erneute Zentrifugation unter den oben genannten Bedingungen statt, um die behandelten Polymerpartikel zu waschen. Durch eine anschließende HPLC wird ungebundenes Doxorubicin nachgewiesen und durch die verringerte Konzentration somit die erfolgreiche Anbindung an die Polymer-Synthase überprüft.

Beispiel 6.2 Anbindung von biologisch aktiven Substanzen, insbesondere pharmazeutischen Wirkstoffen an die Bindedomäne der Polymerpartikel

Wirkstoffe können ebenso an die Polymerpartikel gebunden werden, indem sie über die Bindedomäne an die auf der Oberfläche der Polymerpartikel gebundenen Proteine gebunden werden. Dazu muss erst einmal eine Bindedomäne geschaffen werden. Die Bindung von biologisch aktiven Substanzen wird durch genetische Modifikation der auf der Oberfläche gebundenen Proteine der Polymerpartikel (wie z.B. Polymer-Depolymerase, Phasin oder Phasin-ähnliche Proteine, Polymer-Synthase, Polymer-Regulator) erreicht, so dass diese Proteine eine nach außen gerichtete Bindedomäne ausbilden, über die ein Kopplungsreagenz oder eine biologisch aktive Substanz gebunden werden kann. Bei der Fusion der oben genannten Oberflächenproteine der Polymerpartikel mit einem Protein, das die Anbindung eines Kopplungsreagenzes oder einer biologisch aktiven Substanz direkt ermöglicht, ist bei diesem Verfahren darauf zu achten, dass nach der Fusion mit dem Oberflächenprotein des Polymerpartikels die Funktionalität sowohl des Oberflächenproteins als auch des fusionierten Proteins voll erhalten bleiben muss.

In einem Ausführungsbeispiel wird ein Polymerpartikel mit zwei FLAG-Epitope direkt an den N-Terminus der Polymer-Synthase PhaC1 aus *P. aeruginosa* fusioniert. Die FLAG-Epitope ermöglichen die Bindung von kommerziellen anti-FLAG mAbs (monoklonale Antikörper) (Anti-FLAG M2, Sigma-Aldrich) und anschließend von Enzymmarkern, welche die erfolgreiche Durchführung des Verfahrens belegen sollen. In diesem Beispiel werden sekundären Antikörper-alkalische Phosphatase Konjugate (Anti-Mouse-Alkalische Phosphatase, Sigma-Aldrich) als Enzymmarker verwendet. Die Aktivität der alkalischen Phosphatase an der Oberfläche der Polymerpartikel wird anschließend photometrisch bestimmt.

Beispiel 6.2.1 Herstellung eines Polymerpartikels mit einem FLAG-PhaC1-Fusionsprotein

Für die Herstellung des FLAG-Polymer-Synthase-Fusionsproteins werden die folgenden Oligonukleotide verwendet: 5'-tatg**actagt**gattataaagatgatgatgataaaca-3' und 5'-tatgtttatcatcatcatctttataatc**actagt**ca-3' (SEQ ID Nr. 10 bzw. SEQ ID Nr. 11, SpeI-Schnittstelle fettgedruckt, FLAG-Epitop unterstrichen). Um durch Hybridisierung doppelsträngige DNA zu erhalten, werden beide Oligonukleotide in äquimolarer Menge (je 10 μM) miteinander vermischt und für 30 min bei Raumtemperatur (RT) inkubiert. Die dadurch gebildete doppelsträngige DNA codiert für das FLAG-Epitop (DYKDDDDK) und besitzt überhängende Enden (TA), die mit den überhängenden Enden der NdeI Schnittstelle $\left(\frac{CA^{\triangledown}TATG}{GTAT_{\triangledown}AC}\right)$ komplementär sind. Dieses DNA-Fragment wird mit dem Restriktionsenzym NdeI hydrolysiert und in den analog dazu ebenfalls hydrolysierten Vektor pBHR71 (Abb. 10) kloniert. Das so entstehende Plasmid pBHR71-FLAG (Abb. 13) enthält das Gen mit der SEQ ID Nr. 12 und vermittelt die Expression einer Polymer-Synthase mit N-terminaler FLAG-Fusion (dieser Teil des Proteins bildet nun die Bindedomäne). Über diese Bindedomäne können nun biologisch aktive Substanzen und/oder Kopplungsreagenzien gebunden werden. Die bei der Klonierung ebenfalls eingefügte singuläre SpeI-Schnittstelle steht zusätzlich für die Insertion beliebiger weiterer DNA-Fragmente, die für Funktionsproteine codieren zur Verfügung.

Als Beispiel für die Funktionalität des zuvor geschilderten Konstruktes wird wie folgt vorgegangen. Die Polymerpartikel werden nach Transformation des pBHR71-FLAG Plasmids in *E. coli* Stämme, die bereits das Plasmid pBBad-F enthalten und einen veränderten Fettsäuremetabolismus aufweisen (s. Beispiel 3) exprimiert. Durch Aufschluss der Zellen werden die Polymerpartikel aus den Zellen isoliert und dreimal mit PBS-Puffer (pH 7.5) gewaschen. Dann werden die Polymerpartikel für 30 min bei RT mit monoklonalen anti-FLAG-Antikörpern inkubiert, die an die FLAG-Epitope binden. Danach werden die Polymerpartikel erneut, wie bereits oben beschrieben, gewaschen und anschließend mit sekundärem alkalische Phosphatase Konjugat für 30 min bei RT in PBS-Puffer inkubiert. Nach der 30 min Inkubation werden diese Partikeln in 0.1 M Tris-HCl (pH 8.5) gewaschen und dann werden der Partikelsuspension als Substrat für die alkalische Phosphatase 2 mg/ml p-Nitrophenylphosphat zugesetzt. Die Aktivität der alkalischen Phosphatase wird spektrometrisch bei 410 nm gemessen. Als Negativ-Kontrolle dienen Polymerpartikel, die eine Polymer-Synthase ohne Bindedomäne, d.h. ohne FLAG-Epitop enthalten. Aufgrund der fehlenden Umsetzung des zugegebenen p-Nitrophenylphosphates in der Kontrolle ergeben die spektrometrischen Messungen bei 410 nm ein negatives Ergebnis. Die Tatsache, dass die Polymerpartikel sich überhaupt gebildet haben, ist ein Beleg dafür, dass der Einbau des FLAG-Epitopes keine Auswirkung auf die Funktionalität der Polymer-Synthase gehabt hat.

Dieses Verfahren kann auch mit einem der anderen bereits genannten Oberflächenproteine der Polymerpartikel durchgeführt werden. Werden mehrere Oberflächenproteine gleichzeitig modifiziert, können eine Vielzahl unterschiedlichster Substanzen an die Polymerpartikel gebunden werden und somit eine Multi-Funktionalität ermöglichen, die sie vielfältig einsetzbar macht.

Während im vorigen Beispiel eine biologisch aktive Substanz nachträglich an das bereits exprimierte und gebildete Oberflächenprotein des Polymerpartikels gebunden worden ist, besteht natürlich auch die Möglichkeit, das Protein direkt mit dem Oberflächenprotein zu fusionieren und dann zu exprimieren. Dazu werden die codierenden Sequenzen der Proteine (z.B. Enzyme) mit dem C-terminalen Ende des phaC1 Gens des pBHR71-FLAG Plasmids fusioniert.

Durch PCR werden DNA-Fragmente gewonnen, die jeweils für das einzufügende Protein und für den C-terminalen Abschnitt der Polymer-Synthase codieren, um eine Fusion mit dem phaC1-Gen zu ermöglichen. Beide Fragmente werden über eine mit überhängenden Primern eingefügte Restriktionsschnittstelle miteinander ligiert. Am 5'-Ende dieses Hybrid-Genes wird im Abstand von 7 nt zum Startcodon eine ribosomale Bindestelle (GAGGAG) und eine Restriktionsschnittstelle mittels überhängender Primer eingefügt. Wenn der verwendet Vektor, wie z.B. der hier verwendete Vektor pBHR71-FLAG, bereits eine ribosomale Bindestelle besitzt, ist das zusätzliche Einfügen einer ribosomalen Bindestelle nicht mehr erforderlich. Zusammen mit einer eingefügten Restriktionsschnittstelle am 3'-Ende dieses Hybrid-Genes ist nun die gezielte Klonierung in den Expressionsvektor pBHR71-FLAG möglich. Die Restriktionsschnittstellen am 5' und 3' Ende des Hybrid-Genes müssen so gewählt werden, dass diese innerhalb des Hybrid-Genes nicht ein zweites mal vorkommen, damit eine Klonierung in einen Expressionvektor colinear zum vorliegenden Promotor erfolgen kann. In diesem Beispiel wird das lacZ-Gen aus *E. coli* mittels PCR mit Primem amplifiziert, die eine SpeI-Schnittstelle enthalten: 5'-gg**actagt**atgaccatgattacggattcactggc-3' (SEQ ID Nur.13, SpeI-Schnittstelle fettgedruckt) und 5'-cc**actagt**ttttgacaccagaccaactggtaatggtagcg-3' (SEQ ID Nr.14, SpeI-Schnittstelle fettgedruckt). Zusätzlich wird das Stop-Codon durch Verwendung dieser Primer aus der Sequenz des lacZ-Gens entfernt, um einen durchgehenden Leserahmen zu erhalten. Das daraus resultierende DNA-Fragment mit der SEQ ID Nr. 15 wird direkt in die SpeI-Schnittstelle des pBHR71-FLAG Plasmids kloniert. Das gewonnene Fusionsprotein führte zur Bildung von Polymerpartikel mit β-Galactosidase-Aktivität. Die entsprechenden Polymerpartikel werden isoliert und unter reduzierenden Bedingungen wird die β-Galactosidase-Aktivität mit dem Substrat o-Nitrophenyl-beta-D-galactopyranosid (Calbiochem) nachgewiesen.

Beispiel 7: Stabilität der Bindung zwischen den Oberflächenproteinen und dem Polymerkem der Polymerpartikel

Im Rahmen der Erfindung getätigte Untersuchungen haben ergeben, dass die Polymer-Synthase weder durch Behandlung mit denaturierenden Reagenzien, wie z.B. Sodiumdodecylsulfat (SDS), Harnstoff, Guanidiumhydrochlorid oder Dithiothreitol, noch durch Verwendung azider Bedingungen vom Kern des biologisch abbaubaren Polymer-Partikels gelöst werden kann. Dies deutet auf eine kovalente Verknüpfung hin, die zwischen dem Polymerpartikel und der Polymerpartikelbindedomäne der Polymer-Synthase besteht. Die hohe Stabilität der Bindung ermöglicht einen stabilen Transport von gebundenen oder in die Polymerpartikel eingebaute Substanzen an ihren Zielort. Der N-terminale Abschnitt der Oberflächen-gebundenen Polymer-Synthase (N-Terminus bis zum Beginn der konservierten a/β-Hydrolase-Domäne) ist äußerst variabel und kann durch gentechnische Verfahren durch Funktionsproteine ersetzt werden. Dabei bleibt die Polymer-Synthase-Aktivität und Synthese von Polymerpartikeln erhalten (Rehm, B.H.A. et al., Biochem. Biophys. Acta 2002, Vor.1594, S.178-190). Folglich wird eine Funktionalisierung der Oberfläche erreicht, die eine hohe Stabilität aufweist. Ein Gemisch unterschiedlicher Proteine mit unterschiedlichen Bindedomänen, an die biologisch aktive Substanzen und/oder Kopplungsreagenzien gebunden sind, werden bei Bedarf gleichzeitig appliziert werden, so dass eine Multifunktionalisierung der Oberfläche der Polymerpartikel erfolgt. Die Applikation dieser Proteine erfolgt *in vitro* durch Zugabe der gereinigten Proteine mit unterschiedlichen Bindedomänen zum Synthese-Ansatz bzw. *in vivo* durch Expression der Gene in dem entsprechenden Mikroorganismus, die für jeweils ein Protein mit einer Bindedomäne codieren.

Beispiel 7.1 Weitere Möglichkeiten für eine Modifikation der Oberflächenproteinen der Polymerpartikel

Der C-terminale Abschnitt des Oberflächenproteins Phasin (PhaP) aus *R. eutropha* (Aminosäurereste ab >Ala141) ist hydrophil und kann durch Funktionsproteine ersetzt werden, ohne eine Verankerung des Phasins über die Polymerpartikelbindedomäne an die Oberfläche der Polymerpartikel zu verhindern.

Diese Verankerung beruht auf hydrophoben Wechselwirkungen und ist reversibel (Hanley, S.Z. et al., FEBS Letters 1999, Vol.447, S.99-105). Dieser C-terminale Abschnitt der intrazellulären Polymer-Depolymerasen wird durch gentechnische Verfahren mit Funktionsproteinen fusioniert und ermöglicht somit eine Funktionalisierung der Oberfläche des Polymerpartikels über eine anschließende Anbindung von biologisch aktiven Substanzen und/oder Kopplungsreagenzien.

Der C-Terminus (Aminosäurerest ab >180) der intrazellulären Polymer-Depolymerase von *R. eutropha* vermittelt die Bindung des Enzyms an den Kern der Polymerpartikel (Saegusa, H. et al., J. Bacteriol. 2001, Vor.183(1), S. 94-100). Dieser C-terminale Abschnitt der intrazellulären Polymer-Depolymerasen wird durch gentechnische Verfahren mit Funktionsproteinen fusioniert und ermöglicht somit eine Funktionalisierung der Oberfläche des Polymerpartikels.

Der N-Terminus (Aminosäurerest ab <140) der an die Oberfläche des Polymerpartikels gebundenen Expressionsprodukte der Gene *phaI* und *phaF* aus *Pseudomonas oleovorans* vermittelt die Bindung der Proteine an den Polyester-Kern der Polymerpartikel (Prieto, M.A. et al., J. Bacteriol. 1999, Vol.181(3), S.858-868). Dieser N-terminale Abschnitt der Expressionsprodukte der Gene phaF und phaI wird durch gentechnische Verfahren mit einem Funktionsprotein fusioniert und die dadurch entstehende Bindedomäne ermöglicht anschließend eine Funktionalisierung der Oberfläche des Polymerpartikels über die Anbindung von biologisch aktiven Substanzen und/oder Kopplungsreagenzien.

Beispiel 8: Kovalente Modifikation der Oberflächenproteine der Polymerpartikel mit einem Kopplungsreagenz

Die in Abbildung 1 dargestellten Proteine auf der Oberfläche der biologisch abbaubaren Polymerpartikel können mit speziellen Markierungssubstanzen behandelt werden, die spezifisch an bestimmte Aminosäuren binden (z.B. N-Hydroxysuccinimid-Biotin an Lysin). Dies ermöglicht das Anbinden von biologisch aktiven Substanzen, wie z.B. von Biotin über die Iodacetamid vermittelte Verknüpfung an Cystein. Moleküle, wie Biotin, vermitteln dann eine weitere Verknüpfung von biologisch aktiven Substanzen and die Oberflächenproteine dieser Polymerpartikel. Dazu gehören z.B. Avidin oder Streptavidin, die selbst an Enzyme gebunden sein können und so eine schrittweise Funktionalisierung der Oberflächenproteine der biologisch abbaubaren Polymerpartikel erlauben (Rehm, B.H.A. et al., J. Bacteriol. 1994, Vol.176, S.5639-5647). Diese Funktionalisierung kann durch Anbinden von Antikörpern oder pharmazeutischen Wirkstoffen erfolgen. Es können auch Moleküle mit unterschiedlichen Oberflächenladungen angebracht werden, um dem Polymerpartikel eine bestimmte Ladung auf der Oberfläche zu verleihen, die für den Transport und die Fusion über/mit bestimmten Membranen vorteilhaft ist.

Eine Markierung von Lysinresten an den Oberflächenproteinen der Polymerpartikel mit Biotin, wird mittels N-Hydroxysuccinimid-Biotin erzielt. Bei diesem Experiment werden Polymerpartikel, welche die Polymer-Synthase PhaC1 aus *P. aeruginosa* tragen, aus rekombinanten *E. coli* isoliert, welches das Plasmid pBHR71 trägt. Die Polymerpartikel werden nach der Isolation dreimal in PBS (pH 8.0) gewaschen und N-Hydroxysuccinimid-Biotin (Sigma-Aldrich) wird dann der Lösung bis zu einer Endkonzentration von 5 mM hinzugegeben. Die Reaktion wird durch erneutes Waschen nach 5 min Inkubation bei 4°C beendet. Der Nachweis des an die Partikeloberfläche-gebundenen Biotins wird mit Hilfe des Streptavidin-Alkalische-Phosphatase-Konjugates (Sigma-Aldrich) und o-Nitrophenylphosphat (Calbiochem) als Substrat nachgewiesen. Im Kontrollansatz mit Partikeln, die nicht mit N-Hydroxysuccinimid-Biotin behandelt worden sind, zeigen diese keine alkalische Phosphatase-Aktivität.

Neben den hier aufgeführten Beispielen gibt es für die Anknüpfung von biologisch aktiven Substanzen noch viele Kopplungsreagenzien, mit deren Hilfe die Oberflächenproteine der hier hergestellten Polymerpartikel aktiviert werden können.

## Patentansprüche

**1.** Verfahren zur Herstellung von biologisch abbaubaren Polymerpartikeln, das umfaßt:

a) Bereitstellen einer Zelle, wobei zumindest ein induzierbares Gen in die Zelle eingebracht worden ist, wobei das Gen für ein Protein codiert, das die Größe der Polymerpartikel kontrolliert; und
wobei zumindest ein weiteres Gen, das für eine Polymer-Synthase codiert, in den Organismus eingebracht worden ist; und
wobei es sich bei der zumindest einen Polymer-Synthase um ein Fusionsprotein handelt, das ein biologisch aktives Protein umfaßt, oder wobei es sich bei der zumindest einen Polymer-Synthase um ein Fusionsprotein handelt, das dazu in der Lage ist, eine biologisch aktive Substanz direkt oder über ein Kopplungsreagenz zu binden, oder wobei es sich bei der zumindest einen Polymer-Synthase um ein modifiziertes Protein handelt, das dazu in der Lage ist, eine biologisch aktive Substanz direkt oder über ein Kopplungsreagenz zu binden; und
b) Kultivieren der Zelle unter Induktion des unter a) genannten zumindest einen induzierbaren Gens in einem Kulturmedium unter Bedingungen, die zur Herstellung der biologisch abbaubaren Polymerpartikel durch die Zelle geeignet sind.

**2.** Verfahren nach Anspruch 1, wobei es sich bei dem die Größe kontrollierenden Protein um ein Fusionsprotein handelt, das ein biologisch aktives Protein umfaßt, oder wobei das die Größe kontrollierende Protein dazu in der Lage ist, eine biologisch aktive Substanz direkt oder über ein Kopplungsreagenz zu binden.

**3.** Verfahren nach Anspruch 1, wobei die zumindest eine Polymer-Synthase eine Polymerpartikelbindedomäne und zumindest eine Bindedomäne umfaßt, oder Verfahren nach Anspruch 2, wobei die zumindest eine Polymer-Synthase und das die Größe kontrollierende Protein jeweils eine Polymerpartikelbindedomäne und zumindest eine Bindedomäne umfassen, wobei die zumindest eine Bindedomäne dazu in der Lage ist, die biologisch aktive Substanz direkt und/oder über ein Kopplungsreagenz zu binden.

**4.** Verfahren nach einem der vorhergehenden Ansprüche, wobei man die Partikelgröße durch das zumindest eine induzierbare Gen so steuert, daß die gebildeten Polymerpartikel einen Durchmesser von 10 nm bis 3 μm, bevorzugt einen Durchmesser von 10 nm bis 900 nm, und besonders bevorzugt einen Durchmesser von 10 nm bis 100 nm haben.

**5.** Verfahren zur Herstellung von biologisch abbaubaren Polymerpartikeln, das umfaßt:

a) Bereitstellen einer Zelle, wobei zumindest ein Gen in die Zelle eingebracht worden ist, wobei das Gen für eine Polymer-Synthase codiert;
wobei es sich bei der zumindest einen Polymer-Synthase um ein Fusionsprotein handelt, das ein biologisch aktives Protein umfaßt, oder
wobei es sich bei der zumindest einen Polymer-Synthase um ein Fusionsprotein handelt, das dazu in der Lage ist, eine biologisch aktive Substanz direkt oder über ein Kopplungsreagenz zu binden, oder
wobei es sich bei der zumindest einen Polymer-Synthase um ein modifiziertes Protein handelt, das dazu in der Lage ist, eine biologisch aktive Substanz direkt oder über ein Kopplungsreagenz zu binden; und
b) Kultivieren der Zelle in einem Kulturmedium unter Bedingungen, die zur Herstellung der biologisch abbaubaren Polymerpartikel durch die Zelle geeignet sind.

**6.** Verfahren nach einem der vorhergehenden Ansprüche, wobei man die Zelle in Gegenwart von zumindest einer pharmazeutisch aktiven Substanz und/oder eines Farbstoffs kultiviert, wobei der Farbstoff ausgewählt ist aus Nilrot und Rhodamin 123.

**7.** Verfahren nach einem der vorhergehenden Ansprüche, wobei man die kultivierten Zellen aufschließt und die Polymerpartikel anschließend von den Zellresten abtrennt.

**8.** Verfahren nach einem der vorhergehenden Ansprüche, wobei man eine sich auf der Oberfläche der Polymerpartikel befindliche Lipidschicht von den Polymerpartikeln abtrennt und durch eine Lipidschicht anderer Zusammensetzung ersetzt.

**9.** Verfahren zur *in vitro* Herstellung von biologisch abbaubaren Polymerpartikeln, das umfaßt:

a) Bereitstellen einer für die Polymerpartikelbildung geeigneten Lösung mit zumindest einem Substrat;
b) Einbringen eines Proteins in die Lösung, das dazu geeignet ist, die Größe der Polymerpartikel zu kontrollieren; und
c) Einbringen zumindest einer Polymer-Synthase,

wobei es sich bei der zumindest einen Polymer-Synthase um ein Fusionsprotein handelt, das ein biologisch aktives Protein umfaßt, oder
wobei es sich bei der zumindest einen Polymer-Synthase um ein Fusionsprotein handelt, das dazu in der Lage ist, eine biologisch aktive Substanz direkt oder über ein Kopplungsreagenz zu binden, oder
wobei es sich bei der zumindest einen Polymer-Synthase um ein modifiziertes Protein handelt, das dazu in der Lage ist, eine biologisch aktive Substanz direkt oder über ein Kopplungsreagenz zu binden.

**10.** Verfahren nach Anspruch 9, wobei es sich bei dem die Größe kontrollierenden Protein um ein Fusionsprotein handelt, das ein biologisch aktives Protein umfaßt, oder wobei das die Größe kontrollierende Protein dazu in der Lage ist, eine biologisch aktive Substanz direkt oder über ein Kopplungsreagenz zu binden.

**11.** Verfahren nach Anspruch 9, wobei die zumindest eine Polymer-Synthase eine Polymerpartikelbindedomäne und zumindest eine Bindedomäne umfaßt, oder Verfahren nach Anspruch 10, wobei die zumindest eine Polymer-Synthase und das die Größe kontrollierende Protein jeweils eine Polymerpartikelbindedomäne und zumindest eine Bindedomäne umfassen, wobei die zumindest eine Bindedomäne dazu in der Lage ist, die biologisch aktive Substanz und/oder über ein Kopplungsreagenz zu binden.

**12.** Verfahren nach einem der Ansprüche 1-11, wobei man die zumindest eine Polymer-Synthase aus der Gruppe auswählt, die die Polymer-Synthase von *R. eutropha, P. oleovorans, P. putida, P. aeruginosa, Aeromonas punctata* und *Thiocapsa pfennigii* umfaßt oder wobei es sich bei der zumindest einen Polymer-Synthase um phaC aus *Ralstonia eutropha* handelt.

**13.** Verfahren nach einem der Ansprüche 1 bis 12, wobei die Zelle ein Mikroorganismus ist, der aus den Gattungen ausgewählt wird, die Folgendes umfassen: *Ralstonia, Alcaligenes, Pseudomonas* und *Halobiforma* oder wobei die Zelle ein Mikroorganismus ist, der aus der Gruppe ausgewählt wird, die Folgendes umfasst: *Ralstonia eutropha, Alcaligenes latus, Escherichia coli, Pseudomonas fragi, Pseudomonas putida, Pseudomonas oleovorans, Pseudomonas aeruginosa, Pseudomonas fluorescens* oder *Halobiforma haloterrestris.*

**14.** Verfahren nach einem der Ansprüche 1 bis 4 oder 9 bis 11, wobei das Protein, das die Größe der Polymerpartikel kontrolliert, aus der Familie der phasinähnlichen Proteine einschließlich des Phasins aus *Ralstonia eutropha* und des Phasins aus *Pseudomonas oleovorans* gewonnen wird.

**15.** Verfahren nach einem der Ansprüche 3 oder 11, wobei man die zumindest eine Bindedomäne, die dazu in der Lage ist, die biologisch aktive Substanz und/oder das Kopplungsreagenz zu binden, aus der Gruppe auswählt, die Oligopeptide, Enzyme, Abzyme, nicht-katalytische Proteine, FLAG-Epitope oder zumindest einen Cysteinrest umfaßt.

**16.** Verfahren nach einem der Ansprüche 1 bis 15, wobei die biologisch aktive Substanz eine pharmazeutisch aktive Substanz umfaßt.

**17.** Verfahren nach einem der Ansprüche 1 bis 16, wobei es sich bei dem biologisch aktiven Protein bzw. der biologisch aktiven Substanz um ein Oligopeptid, Enzym, Abzym, nicht-katalytisches Protein oder einen Antikörper handelt.

**18.** Verfahren zur *in vitro* Herstellung von biologisch abbaubaren Polymerpartikeln, das umfaßt:

a) Bereitstellen einer für die Polymerpartikclbildung geeigneten Lösung mit zumindest einem Substrat;
b) Einbringen zumindest einer Polymer-Synthase in die Lösung,

wobei es sich bei der zumindest einen Polymer-Synthase um ein Fusionsprotein handelt, das ein biologisch aktives Protein umfaßt, oder
wobei es sich bei der zumindest einen Polymer-Synthase um ein Fusionsprotein handelt, das dazu in der Lage ist, eine biologisch aktive Substanz direkt oder über ein Kopplungsreagenz zu binden, oder
wobei es sich bei der zumindest einen Polymer-Synthase um ein modifiziertes Protein handelt, das dazu in der Lage ist, eine biologisch aktive Substanz direkt oder über ein Kopplungsreagenz zu binden.

**19.** Polymerpartikel mit zumindest einer Polymer-Synthase,
wobei es sich bei der zumindest einen Polymer-Synthase um ein Fusionsprotein handelt, das ein biologisch aktives Protein umfaßt, oder
wobei es sich bei der zumindest einen Polymer-Synthase um ein Fusionsprotein handelt, das dazu in der Lage ist, eine biologisch aktive Substanz direkt oder über ein Kopplungsreagenz zu binden, oder
wobei es sich bei der zumindest einen Polymer-Synthase um ein modifiziertes Protein handelt, das dazu in der Lage ist, eine biologisch aktive Substanz direkt oder über ein Kopplungsreagenz zu binden.

**20.** Polymerpartikel nach Anspruch 19, weiterhin mit zumindest einem Protein, wobei man das Protein aus der Gruppe auswählt, welche eine Polymer-Depolymerase, einen Polymer-Regulator, eine Polymer-Synthase und ein die Partikelgröße kontrollierendes Protein umfaßt,
wobei es sich bei dem zumindest einen Protein um ein Fusionsprotein handelt, das ein biologisch aktives Protein umfaßt, oder
wobei es sich bei dem zumindest einen Protein um ein Fusionsprotein handelt, das dazu in der Lage ist, eine biologisch aktive Substanz direkt oder über ein Kopplungsreagenz zu binden, oder
wobei es sich bei der zumindest einen Polymer-Synthase um ein modifiziertes Protein handelt, das dazu in der Lage ist, eine biologisch aktive Substanz direkt oder über ein Kopplungsreagenz zu binden.

**21.** Polymerpartikel nach Anspruch 19 oder 20, hergestellt nach einem der in den Ansprüchen 1-16 beschriebenen Verfahren.

**22.** Polymerpartikel nach einem der Ansprüche 19 bis 20, wobei um das Polymerpartikel eine Phospholipidschicht gebildet wird und die Zusammensetzung der Phospholipidschicht den Transport des Partikels über eine biologische Membran beeinflußt.

**23.** Polymerpartikel nach Anspruch 19, wobei die zumindest eine Polymer-Synthase eine Polymerpartikelbindedomäne und zumindest eine Bindedomäne umfaßt, oder Polymerpartikel nach Anspruch 20, wobei die zumindest eine Polymer-Synthase und das zumindest eine Protein jeweils eine Polymerpartikelbindedomäne und zumindest eine Bindedomäne umfassen, wobei die zumindest eine Bindedomäne dazu in der Lage ist, die biologisch aktive Substanz und/oder ein Kopplungsreagenz zu binden.

**24.** Polymerpartikel nach Anspruch 23, wobei die zumindest eine Polymer-Synthase, oder die zumindest eine Polymer-Synthase und das zumindest eine Protein, über die Polymerpartikelbindedomäne an das Polymerpartikel gebunden ist/sind.

**25.** Polymerpartikel nach Anspruch 23, wobei die biologisch aktive Substanz und/oder das Kopplungsreagenz an die Bindedomäne gebunden sind/ist.

**26.** Polymerpartikel nach einem der Ansprüche 23 bis 25, wobei man die zumindest eine Bindedomäne aus der Gruppe auswählt, die Oligopeptide, Enzyme, Abzyme, nicht-katalytische Proteine, FLAG-Epitope und zumindest einen Cysteinrest umfaßt.

**27.** Polymerpartikel nach einem der Ansprüche 23 bis 26, wobei die zumindest eine Bindedomäne erzeugt wird, indem man die zumindest eine Polymer-Synthase oder das zumindest eine Protein oder sowohl die zumindest eine Polymer-Synthase und das zumindest eine Protein mit einem Kopplungsreagenz chemisch modifiziert.

**28.** Polymerpartikel nach Anspruch 27, wobei die chemische Modifikation die Bindung zumindest eines Kopplungsreagenzes an die zumindest eine Polymer-Synthase oder das zumindest eine Protein oder sowohl die zumindest eine Polymer-Synthase als auch das zumindest eine Protein umfaßt, wobei man das aus der Gruppe auswählt, die bis (2-oxo-3-oxazolidinyl)phosphonsäurechlorid (BOP-Cl), Bromtrispyrrolidinophosphoniumhexafluorphosphat (PyBroP), Benzotriazol-1-yloxytrispyrrolidinophosphoniumhexafluorphosphat (PyBOP), n-Hydroxysuccinimid-Biotin, 2-(1H-Benzotriazol-1-yl)-1,1,3,-3-tetramethyluroniumh exaftuorphosphat (HBTU), Dicyclohexylcarbodiimid, Disuccinimidylcarbonat, 1-(3-Dimethylaminopropyl)-3-ethylcarbodiimid (EDC), bis(2-oxo-3-oxazolidinyl)phosphin, Diisopropylcarbodiimid (DIPC), 2-(1H-Benzotrioxazolyl)-1,1,3,3-tetramethyluroniumtetrafluorborat (TBTU), 2-(5-Norbornen-2,3-dicarboxyimido)-1,1,3,3-tetramethyluroniumtetrafluorborat (TNTU), Chlorameisensäure-para-nitrop henytester und O-(N-Succinimidyl)-1,1,3,3-tetramethyluroniumtetrafluorborat (TSTU) umfaßt.

**29.** Polymerpartikel nach einem der Ansprüche 19 bis 28, wobei zumindest eine pharmazeutisch aktive Substanz und/oder ein Farbstoff in das Polymerpartikel eingearbeitet wird, wobei der Farbstoffausgewählt ist aus Nilrot und Rhodamin 123.

**30.** Polymerpartikel nach Anspruch 29, wobei die pharmazeutisch aktive Substanz und/oder der Farbstoff durch Diffusion oder Abbau des Polymerpartikels freigesetzt wird.

**31.** Polymerpartikel nach einem der Ansprüche 19 bis 30, wobei die biologisch aktive Substanz eine pharmazeutisch aktive Substanz umfaßt.

**32.** Polymerpartikel nach einem der Ansprüche 19 bis 30, wobei man die biologisch aktive Substanz aus der Gruppe auswählt, die Didesoxyinosin, Floxuridin, 6-Mcrcaptöpurin, Doxorubicin, Daunorubicin, 1-Darubicin, Cisplatin, Methotrexat, Taxol, Antibiotika, Anticoagulantien, Germicide, Antiarrhythmika und Wirkstoffvorstufen oder Derivate davon umfaßt, oder aus der Gruppe, die Insulin, Calcitonin, ACTH, Glucagone, Somatostatin, Somatotropin, Somatomedin, Parathyroidhormon, Erythropoietin, Hypothalamus-Releasingfaktoren, Prolactin, Thyreotropin, Endorphine, Enkephaline, Vasopressine, synthetische Opiate, Superoxid-Dismutase, Antikörper, Interferone, Asparaginase, Arginase, Arginin-Deaminase, Adenosin-Deaminase, Ribonuclease, Trypsin, Chymotrypsin und Pepsin umfaßt.

**33.** Polymerpartikel nach einem der Ansprüche 19 bis 31, wobei man das biologisch aktive Protein aus der Gruppe auswählt, die Insulin, Calcitonin, ACTH, Glucagone, Somatostatin, Somatotropin, Somatomedin, Parathyroidhormon, Erythropoietin, Hypothalamus-Releasingfaktoren, Prolactin, Thyreotropin, Endorphine, Enkephaline, Vasopressine, synthetische Opiate, Superoxid-Dismutase, Antikörper, Interferone, Asparaginase, Arginase, Arginin-Deaminase, Adenosin-Deaminase, Ribonuclease, Trypsin, Chymotrypsin und Pepsin umfaßt.

**34.** Polymerpartikel nach einem der Ansprüche 19 bis 31, wobei es sich bei dem biologisch aktiven Protein bzw. der biologisch aktiven Substanz um ein Oligopeptid, ein Enzym, ein Abzym, ein nicht-katalytisches Protein oder einen Antikörper handelt.

**35.** Verwendung eines Polymerpartikels mit zumindest einer Polymer-Synthase, wobei es sich bei der zumindest einen Polymer-Synthase um ein Fusionsprotein handelt, das ein biologisch aktives Protein umfaßt, oder
wobei es sich bei der zumindest einen Polymer-Synthase um ein Fusionsprotein handelt, das direkt oder über ein Kopplungsreagenz an eine biologisch aktive Substanz gebunden ist, oder
wobei es sich bei der zumindest einen Polymer-Synthase um ein modifiziertes Protein handelt, das direkt oder über ein Kopplungsreagenz an eine biologisch aktive Substanz gebunden ist,
für die Herstellung eines Arzneimittels, eines Pestizids oder eines Herbizids.

**36.** Verwendung eines Polymerpartikels nach einem der Ansprüche 19 bis 34 für die Herstellung eines für die Behandlung von Erkrankungen des Zentralnervensystems geeigneten Arzneimittels.

**37.** Arzneimittel, Pestizid oder Herbizid, umfassend ein Polymerpartikel mit zumindest einer Polymer-Synthase,
wobei es sich bei der zumindest einen Polymer-Synthase um ein Fusionsprotein handelt, das ein biologisch aktives Protein umfaßt, oder
wobei es sich bei der zumindest einen Polymer-Synthase um ein Fusionsprotein handelt, das direkt oder über ein Kopplungsreagenz an eine biologisch aktive Substanz gebunden ist, oder

wobei es sich bei der zumindest einen Polymer-Synthase um ein modifiziertes Protein handelt, das direkt oder über ein Kopplungsreagenz an eine biologisch aktive Substanz gebunden ist.

**38.** Arzneimittel, umfassend ein Polymerpartikel nach einem der Ansprüche 19 bis 34, zur Verwendung für die Behandlung von Erkrankungen des Zentralnervensystems.

**39.** Verfahren zur Bindung einer biologisch aktiven Substanz, das umfaßt:

a) Bereitstellung eines oder mehrerer Polymerpartikel mit zumindest einer Polymer-Synthase,
wobei es sich bei der zumindest einen Polymer-Synthase um ein Fusionsprotein handelt, das ein biologisch aktives Protein umfaßt, oder
wobei es sich bei der zumindest einen Polymer-Synthase um ein Fusionsprotein handelt, das dazu in der Lage ist, eine biologisch aktive Substanz direkt oder über ein Kopptungsreagenz zu binden, oder
wobei es sich bei der zumindest einen Polymer-Synthase um ein modifiziertes Protein handelt, das dazu in der Lage ist, eine biologisch aktive Substanz direkt oder über ein Kopplungsreagenz zu binden, und
b) In-Kontakt-Bringen des Polymerpartikels mit einer Probe, die eine biologisch aktive Substanz umfaßt, sodaß das biologisch aktive Protein oder die zumindest eine Polymer-Synthase die biologisch aktive Substanz bindet.

**40.** Verfahren nach Anspruch 39, wobei die biologisch aktive Substanz eine pharmazeutisch aktive Substanz umfaßt.

**41.** Verfahren nach Anspruch 39, wobei man das biologisch aktive Protein aus der Gruppe auswählt, die Insulin, Calcitonin, ACTH, Glucagone, Somatostatin, Somatotropin, Somatomedin, Parathyroidhormon, Erythropoietin, Hypothalamus-Releasingfaktoren, Prolactin, Thyreotropin, Endorphine, Enkephaline, Vasopressine, synthetische Opiate, Superoxid-Dismutase, Antikörper, Interferone, Asparaginase, Arginase, Arginin-Deaminase, Adenosin-Deaminase, Ribonuclease, Trypsin, Chymotrypsin und Pepsin umfaßt.

**42.** Verfahren nach Anspruch 39, wobei es sich bei dem biologisch aktiven Protein bzw. der biologisch aktiven Substanz um ein Oligopeptid, ein Enzym, ein Abzym, ein nicht-katalytisches Protein oder einen Antikörper handelt.

**43.** Verfahren nach Anspruch 39, wobei die zumindest eine Polymer-Synthase eine Polymerpartikelbindedomäne und eine Bindedomäne, die dazu in der Lage ist, die biologisch aktive Substanz und/oder das Kopplungsreagenz zu binden, umfaßt.

**44.** Verfahren nach Anspruch 43, wobei man die zumindest eine Bindedomäne aus der Gruppe auswählt, die Oligopeptide, Enzyme, Abzyme, nicht-katalytische Proteine, FLAG-Epitope oder zumindest einen Cysteinrest umfaßt.

**45.** Verfahren nach Anspruch 43, wobei die zumindest eine Bindedomäne erzeugt wird, indem man die zumindest eine Polymer-Synthase auf der Oberfläche des Polymerpartikels mit einem Kopplungsreagenz chemisch modifiziert.

**46.** Verfahren nach Anspruch 45, wobei die chemische Modifikation die Bindung von zumindest einem Kopplungsreagenz an die zumindest eine Polymer-Synthase umfaßt, wobei man das Kopplungsmittel aus der Gruppe auswählt, die bis(2-xo-3-oxazolidinyl)-phosphonsäurechlorid (BOP-Cl), Bromtrispyrrolidinophosphoniumhexafluorphosphat (PyBroP), Benzotriazol-1-yloxytrispyrrolidinophosphoniumhexafluorphosphat (PyBOP), n-Hydroxysuccinimid-Biotin, 2-(1H-Benzotriazol--yl)-1,1,3,3-tetramethyluroniumhexafluorphosphat (HBTU), Dicyclohexylcarbodiimid, Disuccinimidylcarbonat, 1-(3-Dimethylaminopropyl)-3-ethylcarbodiimid (EDC), bis(2-oxo-3-oxazolidinyl)phosphin, Diisopropylcarbodiimid (DIPC), 2-(1H-Benzotrioxazolyl)-1,1,3,3-tetramethyluroniumtetrafluorborat (TBTU), 2-(5-Norbomen-2,3-dicarboxyimido)-1,1,3,3-tetramethyluroniumtetrafluorborat (TNTU), Chlorameisensäure-para-nitrophenylester und O-(N-Succinimidyl)-1,1,3,3-tetramethyluroniumtetrafluorborat (TSTU) umfaßt.

**47.** Partikel, Verwendung oder pharmazeutische Zusammensetzung, Pestizid oder Herbizid nach Anspruch 31, 35, 36, 37 oder 38, wobei das Partikel einen Durchmesser von 10 nm bis 3 um, 10 nm bis 100 nm, oder 50 nm bis 500 nm aufweist.

**48.** Verfahren nach Anspruch 5, wobei zumindest ein weiteres Gen, das für ein Protein codiert, in die Zelle eingebracht worden ist, wobei man das Protein aus der Gruppe auswählt, welche eine Polymer-Depolymerase, einen Polymer-Regulator, eine Polymer-Synthase und ein die Partikelgröße kontrollierendes Protein umfaßt,
wobei es sich bei dem zumindest einen weiteren Protein um ein Fusionsprotein handelt, das ein biologisch aktives Protein umfaßt, oder

wobei es sich bei dem zumindest einen weiteren Protein um ein Fusionsprotein handelt, das dazu in der Lage ist, eine biologisch aktive Substanz direkt oder über ein Kopplungsreagenz zu binden, oder
wobei es sich bei dem zumindest einen weiteren Protein um ein modifiziertes Protein handelt, das dazu in der Lage ist, eine biologisch aktive Substanz direkt oder über ein Kopplungsreagenz zu binden.

**49.** Verwendung nach Anspruch 35 oder 36, wobei weiterhin das Polymerpartikel zumindest ein weiteres Protein umfaßt, wobei man das Protein aus der Gruppe auswählt, welche eine Polymer-Depolymerase, einen Polymer-Regulator, eine Polymer-Synthase und ein die Partikelgröße kontrollierendes Protein umfaßt,
wobei es sich bei dem zumindest einen weiteren Protein um ein Fusionsprotein handelt, das ein biologisch aktives Protein umfaßt, oder
wobei es sich bei dem zumindest einen weiteren Protein um ein Fusionsprotein handelt, das dazu in der Lage ist, eine biologisch aktive Substanz direkt oder über ein Kopplungsreagenz zu binden, oder
wobei es sich bei dem zumindest einen weiteren Protein um ein modifiziertes Protein handelt, das dazu in der Lage ist, eine biologisch aktive Substanz direkt oder über ein Kopplungsreagenz zu binden.

**50.** Arzneimittel, Pestizid oder Herbizid nach Anspruch 37 oder 38, wobei weiterhin das Polymerpartikel zumindest ein weiteres Protein umfaßt, wobei man das weitere Protein aus der Gruppe auswählt, welche eine Polymer-Depolymerase, einen Polymer-Regulator, eine Polymer-Synthase und ein die Partikelgröße kontrollierendes Protein umfaßt,
wobei es sich bei dem zumindest einen weiteren Protein um ein Fusionsprotein handelt, das ein biologisch aktives Protein umfaßt, oder
wobei es sich bei dem zumindest einen weiteren Protein um ein Fusionsprotein handelt, das dazu in der Lage ist, eine biologisch aktive Substanz direkt oder über ein Kopplungsreagenz zu binden, oder
wobei es sich bei dem zumindest einen weiteren Protein um ein modifiziertes Protein handelt, das dazu in der Lage ist, eine biologisch aktive Substanz direkt oder über ein Kopplungsreagenz zu binden.

## Claims

**1.** A process for the production of biodegradable polymer particles which comprises:

a) provision of a cell, wherein at least one inducible gene has been introduced into the cell, wherein the gene codes for a protein which controls the size of the polymer particles; and
wherein at least one further gene which codes for a polymer synthase has been introduced into the organism; and wherein the at least one polymer synthase is a fusion protein comprising a biologically active protein, or wherein the at least one polymer synthase is a fusion protein capable of binding a biologically active substance, directly or via a coupling reagent, or wherein the at least one polymer synthase is a modified protein capable of binding a biologically active substance, directly or via a coupling reagent; and
b) cultivation of the cell with induction of the at least one inducible gene stated in a) in a culture medium under conditions which are suitable for the production of the biodegradable polymer particles by the cell.

**2.** A process according to claim 1, wherein the size-controlling protein is a fusion protein comprising a biologically active protein, or wherein the size-controlling protein is capable of binding a biologically active substance, directly or via a coupling reagent

**3.** A process according to claim 1, wherein the at least one polymer synthase comprises a polymer particle binding domain and at least one binding domain, or a process according to claim 2, wherein each of the at least one polymer synthase and size-controlling protein comprises a polymer particle binding domain and at least one binding domain, wherein the at least one binding domain is capable of binding the biologically active substance, directly and/or via a coupling reagent.

**4.** A process according to any one of the preceding claims, wherein particle size is controlled by the at least one inducible gene in such a manner that the polymer particles formed have a diameter of 10 nm to 3 μm, preferably a diameter of 10 nm to 900 nm, and particularly preferably a diameter of 10 nm to 100 nm.

**5.** A process for the production of biodegradable polymer particles which comprises:

a) provision of a cell, wherein at least one gene has been introduced into the

cell, wherein the gene codes for a polymer synthase;
wherein the at least one polymer synthase is a fusion protein comprising a biologically active protein, or wherein the at least one polymer synthase is a fusion protein capable of binding a biologically active substance, directly or via a coupling reagent, or wherein the at least one polymer synthase is a modified protein capable of binding a biologically active substance, directly or via a coupling reagent; and
b) cultivation of the cell in a culture medium under conditions which are suitable for the production of the biodegradable polymer particles by the cell.

**6.** A process according to any one of the preceding claims, wherein the cell is cultivated in the presence of at least one pharmaceutically active substance and/or a dye, wherein the dye is selected from Nile Red or Rhodamine 123.

**7.** A process according to any one of the preceding claims, wherein the cultivated cells are disrupted and the polymer particles then separated from the cell debris.

**8.** A process according to any one of the preceding claims, wherein a lipid layer located on the surface of the polymer particles is separated from the polymer particles and replaced by a lipid layer of another composition.

**9.** A process for the *in vitro* production of biodegradable polymer particles which comprises:

a) provision of a solution suited to polymer particle formation with at least one substrate;
b) introduction into the solution of a protein which is suited to controlling the size of the polymer particles; and
c) introduction of at least one polymer synthase,

wherein the at least one polymer synthase is a fusion protein comprising a biologically active protein, or wherein the at least one polymer synthase is a fusion protein capable of binding a biologically active substance, directly or via a coupling reagent, or wherein the at least one polymer synthase is a modified protein capable of binding a biologically active substance, directly or via a coupling reagent.

**10.** A process according to claim 9, wherein the size-controlling protein is a fusion protein comprising a biologically active protein, or wherein the size-controlling protein is capable of binding a biologically active substance, directly or via a coupling reagent.

**11.** A process according to claim 9, wherein the at least one polymer synthase comprises a polymer particle binding domain and at least one binding domain, or a process according to claim 10, wherein each of the at least one polymer synthase and size-controlling protein comprises a polymer particle binding domain and at least one binding domain, wherein the at least one binding domain is capable of binding the biologically active substance and/or via a coupling reagent.

**12.** A process according to any one of claims 1 to 11, wherein the at least one polymer synthase is selected from the group comprising the polymer synthase from *R. eutropha, P. oleovorans, P. putida, P. aeruginosa, Aeromonas punctata and Thiocapsa pfennigii* or the at least one polymer synthase is phaC from *Ralstonia eutropha.*

**13.** A process according to any one of claims 1 to 12, wherein the cell is a microorganism selected from the genera comprising *Ralstonia, Alcaligenes, Pseudomonas,* and *Halobiforma,* or a microorganism selected from the group comprising *Ralstonia eutropha, Alcaligenes latus, Escherichia coli, Pseudomonas fragi, Pseudomonas putida, Pseudomonas oleovorans, Pseudomonas aeruginosa, Pseudomonas fluorescen,* or *Halobiforma haloterrestris.*

**14.** A process according to any one of claims 1 to 4 or 9 to 11, wherein the protein which controls the size of the polymer particles is derived from the family of phasin-like proteins, including the phasin from *Ralstonia eutropha* and the phasin from *Pseudomonas oleovorans.*

**15.** A process according to any one of claims 3 or 11, wherein the at least one binding domain which is capable of binding the biologically active substance and/or the coupling reagent is selected from the group comprising oligopeptides, enzymes, abzymes, non-catalytic proteins, FLAG epitopes or at least one cysteine residue.

**16.** A process according to any one of claims 1 to 15, wherein the biologically active substance comprises a pharmaceutically active substance.

**17.** A process according to any one of claims 1 to 16, wherein the biologically active protein or substance is an oligopeptide, enzyme, abzyme, non-catalytic protein or antibody.

**18.** A process for the *in vitro* production of biodegradable polymer particles which comprises:

a) provision of a solution suited to polymer particle formation with at least one substrate;
b) introduction into the solution of at least one polymer synthase,

wherein the at least one polymer synthase is a fusion protein comprising a biologically active protein, or wherein the at least one polymer synthase is a fusion protein capable of binding a biologically active substance, directly or via a coupling reagent, or wherein the at least one polymer synthase is a modified protein capable of binding a biologically active substance, directly or via a coupling reagent.

**19.** A polymer particle comprising at least one polymer synthase, wherein the at least one polymer synthase is a fusion protein comprising a biologically active protein, or wherein the at least one polymer synthase is a fusion protein capable of binding a biologically active substance, directly or via a coupling reagent, or wherein the at least one polymer synthase is a modified protein capable of binding a biologically active substance, directly or via a coupling reagent.

**20.** A polymer particle according to claim 19, further comprising at least one protein selected from the group comprising a polymer depolymerise, a polymer regulator, a polymer synthase and a particle size-controlling protein, wherein the at least one protein is a fusion protein comprising a biologically active protein, or wherein the at least one protein is a fusion protein capable of binding a biologically active substance, directly or via a coupling reagent, or wherein the at least one protein is a modified protein capable of binding a biologically active substance, directly or via a coupling reagent.

**21.** A polymer particle according to claim 19 or 20, produced according to a process of any one of claims 1 to 16.

**22.** A polymer particle according to any one of claims 19 to 20, wherein a phospholipid layer is formed around the polymer particle and the composition of the phospholipid layer influences the transport of the particle through a biological membrane.

**23.** A polymer particle according to claim 19, wherein the at least one polymer synthase comprises a polymer particle binding domain and at least one binding domain, or a polymer particle according to claim 20, wherein each of the at least one polymer synthase and at least one protein comprises a polymer particle binding domain and at least one binding domain, wherein the at least one binding domain is capable of binding the biologically active substance and/or coupling reagent.

**24.** A polymer particle according to claim 23, wherein the at least one polymer synthase, or the at least one polymer synthase and the at least one protein is bound to the polymer particle through the polymer particle binding domain.

**25.** A polymer particle according to claim 23, wherein the biologically active substance and/or coupling reagent is bound to the binding domain.

**26.** A polymer particle according to claim 23 to 25, wherein the at least one binding domain is selected from the group comprising oligopeptides, enzymes, abzymes, non-catalytic proteins, FLAG epitopes or at least one cysteine residue.

**27.** A polymer particle according to claim 23 to 26, wherein the at least one binding domain is created by chemical modification with a coupling reagent of the at least one polymer synthase or the at least one protein or both the at least one polymer synthase and the at least one protein.

**28.** A polymer particle according to claim 27, wherein the chemical modification comprises the binding of at least one coupling reagent to the at least one polymer synthase or the at least one protein or both the at least one polymer synthase and the at least one protein, wherein the coupling reagent is selected from the group comprising bis(2-oxo-3-oxazolydinyl)phosphonic chloride (BOP-Cl), bromotrispyrrolidinophosphonium hexafluorophosphate (PyBroP), benzotriazol-1-yl-oxy-trispyrrolidinophosphonium hexafluorophosphate (PyBOP), n-hydroxysuccinimide biotin, 2-(1H-benzotriazol-1-yl)-1,1,3,3-tetramethyluronium hexafluorophosphate (HBTU), dicyclohexylcarbodiimide, disuccinimidyl carbonate, 1-(3-dimethylaminopropyl)-3-ethylcarbodiimide (EDC), bis(2-oxo-3-oxazolydinyl)phosphine, diisopropylcarbodiimide (DIPC), 2-(1H-benzotrioxazolyl)-1,1,3,3-tetramethyluronium tetrafluoroborate (TBTU), 2-(5-norbomene-2,3-dicarboxyimido)-1,1,3,3-tetramethyluronium tetrafluoroborate (TNTU), para-nitrophenylchloroformate, and O-(n-succinimidyl)-1,1,3,3-tetramethyluronium tetrafluoroborate (TSTU).

**29.** A polymer particle according to any one of claims 19 to 28, wherein at least one pharmaceutically active substance and/or a dye is incorporated into the polymer particle, wherein the dye is selected from Nile Red or Rhodamine 123.

**30.** A polymer particle according to claim 29, wherein the pharmaceutically active substance and/or dye is released by diffusion or by degradation of the polymer particle.

**31.** A polymer particle according to any one of claims 19 to 30, wherein the biologically active substance comprises a pharmaceutically active substance.

**32.** A polymer particle according to any one of claims 19 to 30, wherein the biologically active substance is selected from the group comprising dideoxyinosine, floxuridine, 6-mercaptopurine, doxorubicin, daunorubicin, 1-darubicin, cisplatin, methotrexate, taxol, antibiotics, anticoagulants, germicides, antiarrhythmic agents or active ingredient precursors or derivatives thereof, or from the group comprising insulin, calcitonin, ACTH, glucagons, somatostatin, somatotropin, somatomedin, parathyroid hormone, erythropoietin, hypothalamic release factors, prolactin, thyroid-stimulating hormone, endophins, enkephalins, vasopressins, non-naturally occurring opiates, superoxide dismutase, antibodies, interferons, asparaginase, arginase, arginine deaminase, adenosine deaminase, ribonuclease, trypsin, chymotrypsin or pepsin.

**33.** A polymer particle according to any one of claims 19 to 31, wherein the biologically active protein is selected from the group comprising insulin, calcitonin, ACTH, glucagons, somatostatin, somatotropin, somatomedin, parathyroid hormone, erythropoietin, hypothalamic release factors, prolactin, thyroid-stimulating hormone, endophins, enkephalins, vasopressins, non-naturally occurring opiates, superoxide dismutase, antibodies, interferons, asparaginase, arginase, arginine deaminase, adenosine deaminase, ribonuclease, trypsin, chymotrypsin or pepsin.

**34.** A polymer particle according to any one of claims 19 to 31, wherein the biologically active protein or substance is an oligopeptide, enzyme, abzyme, noncatalytic protein or antibody.

**35.** Use of a polymer particle comprising at least one polymer synthase, wherein the at least one polymer synthase is a fusion protein comprising a biologically active protein, or wherein the at least one polymer synthase is a fusion protein bound to a biologically active substance, directly or via a coupling reagent, or wherein the at least one polymer synthase is a modified protein bound to a biologically active substance, directly or via a coupling reagent, for the production of a pharmaceutical preparation, a pesticide or a herbicide.

**36.** Use of a polymer particle according to any one of claims 19 to 34 for the production of a pharmaceutical preparation suitable for the treatment of diseases of the central nervous system.

**37.** A pharmaceutical preparation, pesticide or herbicide comprising a polymer particle comprising at least one polymer synthase, wherein the at least one polymer synthase is a fusion protein comprising a biologically active protein, or wherein the at least one polymer synthase is a fusion protein bound to a biologically active substance, directly or via a coupling reagent, or wherein the at least one polymer synthase is a modified protein bound to a biologically active substance, directly or via a coupling reagent.

**38.** A pharmaceutical preparation comprising a polymer particle according to any one of claims 19 to 34 for use in the treatment of diseases of the central nervous system.

**39.** A method of binding a biologically active substance comprising (a) providing one or more polymer particles comprising at least one polymer synthase, wherein the at least one polymer synthase is a fusion protein comprising a biologically active protein, or wherein the at least one polymer synthase is a fusion protein capable of binding a biologically active substance, directly or via a coupling reagent, or wherein the at least one polymer synthase is a modified protein capable of binding a biologically active substance, directly or via a coupling reagent, and (b) contacting the polymer particle with a sample comprising a biologically active substance so that the biologically active protein or the at least one polymer synthase binds the biologically active substance.

**40.** A method according to claim 39, wherein the biologically active substance comprises a pharmaceutically active substance.

**41.** A method according to claim 39, wherein the biologically active protein is selected from the group comprising insulin, calcitonin, ACTH, glucagons, somatostatin, somatotropin, somatomedin, parathyroid hormone, erythropoietin, hypothalamic release factors, prolactin, thyroid-stimulating hormone, endophins, enkephalins, vasopressins, non-naturally occurring opiates, superoxide dismutase, antibodies, interferons, asparaginase, arginase, arginine deaminase, adenosine deaminase, ribonuclease, trypsin, chymotrypsin or pepsin.

**42.** A method according to claim 39, wherein the biologically active protein or substance is an oligopeptide, enzyme, abzyme noncatalytic protein or antibody.

**43.** A method according to claim 39, wherein the at least one polymer synthase comprises a polymer particle binding domain and a binding domain which is capable of binding the biologically active substance and/or coupling reagent.

**44.** A method according to claim 43, wherein the at least one binding domain is selected from the group comprising oligopeptides, enzymes, abzymes, noncatalytic proteins, FLAG epitopes or at least one cysteine residue.

**45.** A method according to claim 43, wherein the at least one binding domain is created by chemical modification of the at least one polymer synthase on the surface of the polymer particle with a coupling reagent.

**46.** A method according to claim 45, wherein the chemical modification comprises the binding of at least one coupling reagent to the at least one polymer synthase, wherein the coupling agent is selected from the group comprising bis (2-oxo-3-oxazolydinyl)phosphonic chloride (BOP-Cl), bromotrispyrrolidinophosphonium hexafluorophosphate (PyBroP), benzotriazol-1-yl-oxy-trispyrrolidinophosphonium hexafluorophosphate (PyBOP), n-hydroxysuccinimide biotin, 2-(1 H-benzotriazol-1-yl)-1,1,3,3-tetramethyluronium hexafluorophosphate (HBTU), dicyclohexylcarbodiimide, disuccinimidyl carbonate, 1 -(3-dimethylaminopropyl)-3-ethylcarbodümide (EDC), bis(2-oxo-3-oxazolydinyl)phosphine, diisopropylcarbodiimide (DIPC), 2-(1H-benzotrioxazolyl)-1,1,3,3-tetramethyluronium tetrafluoroborate (TBTU), 2-(5-norbornene-2,3-dicarboxyimido)-1,1,3,3-tetramethyluronium tetrafluoroborate (TNTU), para-nitrophenylchloroformate, and O-(n-succinimidyl)-1,1,3,3-tetramethyluronium tetrafluoroborate (TSTU).

**47.** A particle, use or pharmaceutical preparation, pesticide or herbicide of claim 31, 35, 36, 37 or 38 wherein the particle has a diameter of 10nm to 3μm, 10nm to 100nm, or 50nm to 500nm.

**48.** A process of claim 5, wherein at least one additional gene encoding a protein selected from the group comprising a polymer depolymerase, a polymer regulator, a polymer synthase and a particle size-controlling protein has been introduced into the cell, wherein the at least one additional protein is a fusion protein comprising a biologically active protein, or wherein the at least one additional protein is a fusion protein capable of binding a biologically active substance, directly or via a coupling reagent, or wherein the at least one additional protein is a modified protein capable of binding a biologically active substance, directly or via a coupling reagent.

**49.** A use of claim 35 or 36, wherein the polymer particle further comprises at least one additional protein selected from the group comprising a polymer depolymerase, a polymer regulator, a polymer synthase and a particle size-controlling protein, wherein the at least one additional protein is a fusion protein comprising a biologically active protein, or wherein the at least one additional protein is a fusion protein capable of binding a biologically active substance, directly or via a coupling reagent, or wherein the at least one additional protein is a modified protein capable of binding a biologically active substance, directly or via a coupling reagent.

**50.** A pharmaceutical preparation, pesticide or herbicide of claim 37 or 38, wherein the polymer particle further comprises at least one additional protein selected from the group comprising a polymer depolymerase, a polymer regulator, a polymer synthase and a particle size-controlling protein, wherein the at least one additional protein is a fusion protein comprising a biologically active protein, or wherein the at least one additional protein is a fusion protein capable of binding a biologically active substance, directly or via a coupling reagent, or wherein the at least one additional protein is a modified protein capable of binding a biologically active substance, directly or via a coupling reagent.

## Revendications

**1.** Procédé de fabrication de particules polymères biodégradables, qui comprend :

a) la mise à disposition d'une cellule, au moins un gène inductible ayant été introduit dans la cellule, le gène codant pour une protéine qui contrôle la granulométrie des particules polymères ; et
au moins un autre gène, qui code pour une polymère-synthase, ayant été introduit dans l'organisme ; et dans lequel, pour ce qui concerne l'au moins une polymère-synthase, il s'agit d'une protéine de fusion, qui comprend une protéine bioactive, ou encore, pour ce qui concerne l'au moins une polymère-synthase, il s'agit d'une protéine de fusion, qui est à même de lier une substance bioactive, directement ou par l'intermédiaire d'un réactif de couplage, ou encore, pour ce qui concerne l'au moins une polymère-synthase, il s'agit d'une protéine modifiée, qui est à même de lier une substance bioactive, directement ou par l'intermédiaire d'un réactif de couplage ; et
b) la culture de la cellule, par induction de l'au moins un gène inductible mentionné en a), dans un milieu de culture, dans des conditions convenant à la fabrication, par la cellule, des particules polymères biodégradables.

**2.** Procédé selon la revendication 1, dans lequel, pour ce qui concerne la protéine contrôlant la granulométrie, il s'agit d'une protéine de fusion, qui comprend une protéine bioactive, ou encore dans lequel la protéine contrôlant la granulométrie est à même de lier une substance bioactive, directement ou par l'intermédiaire d'un réactif de couplage.

**3.** Procédé selon la revendication 1, dans lequel l'au moins une polymère-synthase comprend un domaine de liaison des particules polymères et au moins un domaine de liaison, ou procédé selon la revendication 2, dans lequel l'au moins une polymère-synthase et la protéine contrôlant la granulométrie comprennent chacune une domaine de liaison des particules polymères et au moins un domaine de liaison, l'au moins un domaine de liaison étant à même de lier la substance bioactive, directement et/ou par l'intermédiaire d'un réactif de couplage.

**4.** Procédé selon l'une des revendications précédentes, dans lequel on pilote la granulométrie des particules par l'au moins un gène inductible de telle sorte que les particules polymères formées aient un diamètre de 10 nm à 3 μm, de préférence un diamètre de 10 nm à 900 nm et d'une manière particulièrement préférée un diamètre de 10 nm à 100 nm.

**5.** Procédé de fabrication de particules polymères biodégradables, qui comprend :

a) la mise à disposition d'une cellule, au moins un gène ayant été introduit dans la cellule, le gène codant pour une polymère-synthase ;
où, pour ce qui concerne l'au moins une polymère-synthase, il s'agit d'une protéine de fusion qui comprend une protéine bioactive, ou
où, pour ce qui concerne l'au moins une polymère-synthase, il s'agit d'une protéine de fusion qui est à même de lier une substance bioactive, directement ou par l'intermédiaire d'un réactif de couplage, ou
où, pour ce qui concerne l'au moins une polymère-synthase, il s'agit d'une protéine modifiée, qui est à même de lier une substance biologiquement active, directement ou par l'intermédiaire d'un réactif de couplage ; et
b) la culture de la cellule dans un milieu de culture, dans des conditions convenant à la fabrication, par la cellule, des particules polymères biodégradables.

**6.** Procédé selon l'une des revendications précédentes, dans lequel on cultive la cellule en présence d'au moins une substance pharmaceutiquement active et/ou d'un colorant, le colorant étant choisi parmi le rouge du Nil et la rhodamine 123.

**7.** Procédé selon l'une des revendications précédentes, dans lequel on désagrège les cellules cultivées, puis on sépare les particules polymères des résidus cellulaires.

**8.** Procédé selon l'une des revendications précédentes, dans lequel on sépare des particules polymères une couche lipidique se trouvant sur la surface des particules polymères, et on la remplace par une couche lipidique ayant une autre composition.

**9.** Procédé de fabrication *in vitro* de particules polymères biodégradables, qui comprend :

a) la mise à disposition d'une solution convenant à la formation de particules polymères, avec au moins un substrat ;
b) l'introduction d'une protéine dans la solution, qui est à même de contrôler la granulométrie des particules polymères ; et
c) l'introduction d'au moins une polymère-synthase,

où, pour ce qui concerne l'au moins une polymère-synthase, il s'agit d'une protéine de fusion qui comprend une protéine bioactive, ou
où, pour ce qui concerne l'au moins une polymère-synthase, il s'agit d'une protéine de fusion qui est à même de lier une substance biologiquement active, directement ou par l'intermédiaire d'un réactif de couplage, ou
où, pour ce qui concerne l'au moins une polymère-synthase, il s'agit d'une protéine modifiée, qui est à même de lier une substance biologiquement active, directement ou par l'intermédiaire d'un réactif de couplage.

**10.** Procédé selon la revendication 9, dans lequel, pour ce qui concerne la protéine contrôlant la granulométrie, il s'agit d'une protéine de fusion, qui comprend une protéine bioactive, ou dans lequel la protéine contrôlant la granulométrie est à même de lier une substance biologiquement active, directement ou par l'intermédiaire d'un réactif de couplage.

**11.** Procédé selon la revendication 9, dans lequel l'au moins une polymère-synthase comprennent un domaine de liaison des particules polymères et au moins un domaine de liaison, ou procédé selon la revendication 10, dans lequel l'au moins une polymère-synthase, et la protéine contrôlant la granulométrie, comportent chacune un domaine de liaison des particules polymères et au moins un domaine de liaison, l'au moins un domaine de liaison étant à même de lier la substance biologiquement active, directement et/ou par l'intermédiaire d'un réactif de couplage.

**12.** Procédé selon l'une des revendications 1 à 11, dans lequel on choisit l'au moins une polymère-synthase dans le groupe qui comprend les polymère-synthases de *R. eutropha, P. oleovorans, P. putida, P. aeruginosa, Aeromonas punctata* et *Thiocapsa pfennigii,* ou dans lequel, pour ce qui concerne l'au moins une polymère-synthase, il s'agit de la phaC de *Ralstonia eutropha.*

**13.** Procédé selon l'une des revendications 1 à 12, dans lequel la cellule est un micro-organisme qui est choisi parmi les genres comprenant ce qui suit : *Ralstonia, Alcaligenes, Pseudomonas* ou *Halobiforma,* ou dans lequel la cellule est un micro-organisme choisi dans le groupe comprenant ce qui suit : *Ralstonia eutropha, Alcaligenes latus, Escherichia coli, Pseudomonas fragi, Pseudomonas putida, Pseudomonas oleovorans, Pseudomonas aeruginosa, Pseudomonas fluorescens* ou *Halobiforma haloterrestris.*

**14.** Procédé selon l'une des revendications 1 à 4 ou 9 à 11, dans lequel la protéine qui contrôle la granulométrie des particules polymères est obtenue à partir de la famille des protéines analogues à la phasine, y compris de la phasine de *Ralstonia eutropha* et de la phasine de *Pseudomonas oleovorans.*

**15.** Procédé selon l'une des revendications 3 ou 11, dans lequel on choisit l'au moins un domaine de liaison qui est à même de lier la substance bioactive et/ou le réactif de couplage, dans le groupe qui comprend les oligopeptides, les enzymes, les abzymes, les protéines non catalytiques, les épitopes FLAG, ou au moins un résidu de cystéine.

**16.** Procédé selon l'une des revendications 1 à 15, dans lequel la substance bioactive est une substance pharmaceutiquement active.

**17.** Procédé selon l'une des revendications 1 à 16, dans lequel, pour ce qui concerne la protéine bioactive ou la substance bioactive, il s'agit d'un oligopeptide, d'une enzyme, d'une abzyme, d'une protéine non catalytique ou d'un anticorps.

**18.** Procédé de fabrication *in vitro* de particules polymères biodégradables, qui comprend :

a) la mise à disposition d'une solution convenant à la formation de particules polymères, avec au moins un substrat ;
b) l'introduction d'au moins une polymère-synthase dans la solution,

où, pour ce qui concerne l'au moins une polymère-synthase, il s'agit d'une protéine de fusion qui comprend une protéine bioactive, ou
où, pour ce qui concerne l'au moins une polymère-synthase, il s'agit d'une protéine de fusion qui est à même de lier une substance bioactive, directement ou par l'intermédiaire d'un réactif de couplage, ou
où, pour ce qui concerne l'au moins une polymère-synthase, il s'agit d'une protéine modifiée, qui est à même de lier une substance bioactive, directement ou par l'intermédiaire d'un réactif de couplage.

**19.** Particule polymère comportant au moins une polymère-synthase,
où, pour ce qui concerne l'au moins une polymère-synthase, il s'agit d'une protéine de fusion qui comprend une protéine bioactive, ou

où, pour ce qui concerne l'au moins une polymère-synthase, il s'agit d'une protéine de fusion qui est à même de lier une substance bioactive, directement ou par l'intermédiaire d'un réactif de couplage, ou
où, pour ce qui concerne l'au moins une polymère-synthase, il s'agit d'une protéine modifiée, qui est à même de lier une substance bioactive, directement ou par l'intermédiaire d'un réactif de couplage.

**20.** Particule polymère selon la revendication 19, comportant en outre au moins une protéine, la protéine étant choisie dans le groupe qui comprend une polymère dépolymérase, un régulateur de polymères, une polymère-synthase et une protéine contrôlant la granulométrie, où, pour ce qui concerne l'au moins une protéine, il s'agit d'une protéine de fusion, qui comprend une protéine bioactive, ou
où, pour ce qui concerne l'au moins une protéine, il s'agit d'une protéine de fusion, qui est à même de lier une substance bioactive, directement ou par l'intermédiaire de réactif de couplage, ou
où, pour ce qui concerne l'au moins une protéine, il s'agit d'une protéine modifiée, qui est à même de lier une substance bioactive, directement ou par l'intermédiaire d'un réactif de couplage.

**21.** Particule polymère selon la revendication 19 ou 20, fabriquée par l'un des procédés décrits dans les revendications 1 à 16.

**22.** Particule polymère selon l'une des revendications 19 à 20, où on forme une couche phospholipidique autour de la particule polymère, et la composition de la couche phospholipidique influe sur le transport de la particule à travers une membrane biologique.

**23.** Particule polymère selon la revendication 19, dans laquelle l'au moins une polymère-synthase comprend un domaine de liaison des particules polymères et au moins un domaine de liaison, ou une particule polymère selon la revendication 20, dans laquelle l'au moins une polymère-synthase et l'au moins une protéine comprennent chacune un domaine de liaison des particules polymères et au moins un domaine de liaison, l'au moins un domaine de liaison étant à même de lier la substance bioactive et/ou un réactif de couplage.

**24.** Particule polymère selon la revendication 23, dans laquelle l'au moins une polymère-synthase, ou l'au moins une polymère-synthase et l'au moins une protéine, sont liées à la particule polymère par l'intermédiaire d'un domaine de liaison des particules polymères.

**25.** Particule polymère selon la revendication 23, dans laquelle la substance bioactive et/ou le réactif de couplage sont liés au domaine de liaison.

**26.** Particule polymère selon l'une des revendications 23 à 25, dans laquelle on choisit l'au moins un domaine de liaison dans le groupe comprenant les oligopeptides, les enzymes, les abzymes, les protéines non catalytiques, les épitopes FLAG et au moins un résidu de cystéine.

**27.** Particule polymère selon l'une des revendications 23 à 26, dans laquelle on produit l'au moins un domaine de liaison en faisant subir une modification chimique à l'au moins une polymère-synthase, ou à l'au moins une protéine, ou tant à l'au moins une polymère-synthase qu'à l'au moins une protéine, à l'aide d'un réactif de couplage.

**28.** Particule polymère selon la revendication 27, dans laquelle la modification chimique comprend la liaison d'au moins un réactif de couplage à l'au moins une polymère-synthase ou à l'au moins une polymère-synthase, ou à l'au moins une protéine, ou tant à l'au moins une polymère-synthase qu'à l'au moins une protéine, où on choisit le réactif de couplage dans le groupe comprenant le chlorure de bis(2-oxo-3-oxazolidinyl)phosphonyle (BOP-Cl), l'hexafluorophosphate de bromotrispyrrolidinophosphonium (PyBroP), l'hexafluorophosphate de benzotriazol-1-yloxytrispyrrolidinophosphonium (PyBOP), la n-hydroxysuccinimide-biotine, l'hexafluorophosphate de 2-(1H-benzotriazol-1-yl)-1,1,3,3-tétraméthyluronium (HBTU), le dicyclohexylcarbodiimide, le carbonate de disuccinimidyle, le 1-(3-diméthylaminopropyl)-3-éthylcarbodiimide (EDC), la bis(2-oxo-3-oxazolidinyl)phosphine, le diisopropylcarbodiimide (DIPC), le tétrafluoroborate de 2-(1H-benzotrioxazolyl)-1,1,3,3-tétraméthyluronium (TBTU), le tétrafluoroborate d e 2-(5-norbornène-2,3-dicarboxyimido)-1,1,3,3-tétraméthyluronium (TNTU), l'ester paranitrophénylique de l'acide chloroformique, et le tétrafluoroborate d'O-(N-succinimidyl)-1,1,3,3-tétraméthyluronium (TSTU).

**29.** Particule polymère selon l'une des revendications 19 à 28, dans laquelle l'au moins une substance pharmaceutiquement active, et/ou un colorant, sont incorporés dans la particule polymère, le colorant étant choisi parmi le rouge du Nil et la rhodamine 123.

**30.** Particule polymère selon la revendication 29, dans laquelle la substance pharmaceutiquement active et/ou le colorant sont libérés par diffusion ou dégradation de la particule polymère.

**31.** Particule polymère selon l'une des revendications 19 à 30, la substance bioactive comprenant une substance pharmaceutiquement active.

**32.** Particule polymère selon l'une des revendications 19 à 30, dans laquelle la substance bioactive est choisie dans le groupe comprenant la didésoxyinosine, la floxuridine, la 6-mercaptopurine, la doxorubicine, la daunorubicine, la 1-darubicine, le cisplatine, le méthotrexate, le taxol, les antibiotiques, les anticoagulants, les germicides, les antiarythmiques et les précurseurs de principes actifs ou dérivés de ces derniers, ou dans le groupe qui comprend l'insuline, la calcitonine, l'ACTH, le glucagon, la somatostatine, la somatotropine, la somadomédine, l'hormone parathyroïdienne, l'érythropoïétine, les facteurs de libération de l'hypothalamus, la prolactine, la thyréotropine, les endorphines, les encéphalines, les vasopressines, les opiacés synthétiques, la superoxyde-dismutase, les anticorps, les interférons, l'asparaginase, l'arginase, l'arginine-désaminase, l'adénosine-désaminase, la ribonucléase, la trypsine, la chymotrypsine et la pepsine.

**33.** Particule polymère selon l'une des revendications 19 à 31, dans laquelle la protéine bioactive est choisie dans le groupe qui comprend l'insuline, la calcitonine, l'ACTH, le glucagon, la somatostatine, la somatotropine, la somatomédine, l'hormone parathyroïdienne, l'érythropoïétine, les facteurs de libération de l'hypothalamus, la prolactine, la thyréotropine, les endorphines, les encéphalines, les vasopressines, les opiacés synthétiques, la superoxyde-dismutase, les anticorps, les interférons, l'asparaginase, l'arginase, l'arginine-désaminase, l'adénosine-désaminase, la ribonucléase, la trypsine, la chymotrypsine et la pepsine.

**34.** Particule polymère selon l'une des revendications 19 à 31, dans laquelle, pour ce qui concerne la protéine bioactive ou la substance bioactive, il s'agit d'un oligopeptide, d'une enzyme, d'une abzyme, d'une protéine non catalytique ou d'un anticorps.

**35.** Utilisation d'une particule polymère comportant au moins une polymère-synthase,
pour laquelle, pour ce qui concerne l'au moins une polymère-synthase, il s'agit d'une protéine de fusion qui comprend une protéine bioactive, ou
pour laquelle, pour ce qui concerne l'au moins une polymère-synthase, il s'agit d'une protéine de fusion qui est liée, directement ou par l'intermédiaire d'un réactif de couplage, à une substance bioactive, ou
pour laquelle, pour ce qui concerne l'au moins une polymère-synthase, il s'agit d'une protéine modifiée, qui est liée directement ou par l'intermédiaire d'un réactif de couplage à une substance bioactive,
pour la préparation d'un médicament, d'un pesticide ou d'un herbicide.

**36.** Utilisation d'une particule polymère selon l'une des revendications 19 à 34 pour la préparation d'un médicament convenant au traitement de maladies du système nerveux central.

**37.** Médicament, pesticide ou herbicide, comprenant une particule polymère ayant au moins une polymère-synthase, dans lequel, pour ce qui concerne l'au moins une polymère-synthase, il s'agit d'une protéine de fusion qui comprend une protéine bioactive, ou
pour laquelle, pour ce qui concerne l'au moins une polymère-synthase, il s'agit d'une protéine de fusion qui est liée directement ou par l'intermédiaire d'un réactif de couplage à une substance bioactive, ou
pour laquelle, pour ce qui concerne l'au moins une polymère-synthase, il s'agit d'une protéine modifiée, qui est liée directement ou par l'intermédiaire d'un réactif de couplage à une substance bioactive.

**38.** Médicament comprenant une particule polymère selon l'une des revendications 19 à 34, pour utilisation pour le traitement de maladies du système nerveux central.

**39.** Procédé de liaison d'une substance bioactive, qui comprend :

a) la mise à disposition d'une ou plusieurs particules polymères comportant au moins une polymère-synthase, où, pour ce qui concerne l'au moins une polymère-synthase, il s'agit d'une protéine de fusion qui comprend une protéine bioactive, ou
où, pour ce qui concerne l'au moins une polymère-synthase, il s'agit d'une protéine de fusion qui est à même de lier une substance bioactive, directement ou par l'intermédiaire d'un réactif de couplage, ou
où, pour ce qui concerne l'au moins une polymère-synthase, il s'agit d'une protéine modifiée, qui est à même de lier une substance biologiquement active, directement ou par l'intermédiaire d'un réactif de couplage, et
b) la mise en contact de la particule polymère avec une sonde qui comprend une substance bioactive, de telle sorte que la protéine bioactive ou l'au moins une polymère-synthase lient la substance bioactive.

**40.** Procédé selon la revendication 39, dans lequel la substance bioactive est une substance pharmaceutiquement active.

**41.** Procédé selon la revendication 39, dans lequel la protéine bioactive est choisie dans le groupe qui comprend l'insuline, la calcitonine, l'ACTH, le glucagon, la somatostatine, la somatotropine, la somatomédine, l'hormone parathyroïdienne, l'érythropoïétine, les facteurs de libération de l'hypothalamus, la prolactine, la thyréotropine, les endorphines, les encéphalines, les vasopressines, les opiacés synthétiques, la superoxyde-dismutase, les anticorps, les interférons, l'asparaginase, l'arginase, l'arginine-désaminase, l'adénosine-désaminase, la ribonucléase, la trypsine, la chymotrypsine et la pepsine.

**42.** Procédé selon la revendication 39, dans lequel, pour ce qui concerne la protéine bioactive ou la substance bioactive, il s'agit d'un oligopeptide, d'une enzyme, d'une abzyme, d'une protéine non catalytique ou d'un anticorps.

**43.** Procédé selon la revendication 39, dans lequel l'au moins une polymère-synthase comprend un domaine de liaison des particules polymères et un domaine de liaison, qui est à même de lier la substance bioactive et/ou le réactif de couplage.

**44.** Procédé selon la revendication 43, dans lequel l'au moins un domaine de liaison est choisi dans le groupe comprenant les oligopeptides, les enzymes, les abzymes, les protéines non catalytiques, les épitopes FLAG ou au moins un résidu de cystéine.

**45.** Procédé selon la revendication 43, dans lequel l'au moins un domaine de liaison est produit par modification chimique, avec un réactif de couplage, de l'au moins une polymère-synthase sur la surface de la particule polymère.

**46.** Procédé selon la revendication 45, dans lequel la modification chimique comprend la liaison d'au moins un réactif de couplage à l'au moins une polymère-synthase, ou à l'au moins une protéine, ou tant à l'au moins une polymère-synthase qu'à l'au moins une protéine, où on le choisit dans le groupe comprenant le chlorure de bis(2-oxo-3-oxazolidinyl)phosphonyle (BOP-Cl), l'hexafluorophosphate de bromotrispyrrolidinophosphonium (PyBroP), l'hexafluorophosphate de benzotriazol-1-yloxytrispyrrolidinophosphonium (PyBOP), la n-hydroxysuccinimide-biotine, l'hexafluorophosphate de 2-(1H-benzotriazol-1-yl)-1,1,3,3-tétraméthyluronium (HBTU), le dicyclohexylcarbodiimide, le carbonate de disuccinimidyle, le 1-(3-diméthylaminopropyl)-3-éthylcarbodiimide (EDC), la bis(2-oxo-3-oxazolidinyl)phosphine, le diisopropylcarbodiimide (DIPC), le tétrafluoroborate de 2-(1H-benzotrioxazolyl)-1,1,3,3-tétraméthyluronium (TBTU), le tétrafluoroborate d e 2-(5-norbornène-2,3-dicarboxyimido)-1,1,3,3-tétraméthyluronium (TNTU), l'ester paranitrophénylique de l'acide chloroformique, et le tétrafluoroborate d'O-(N-succinimidyl)-1,1,3,3-tétraméthyluronium (TSTU).

**47.** Particule, utilisation ou composition pharmaceutique, pesticide ou herbicide selon la revendication 31, 35, 36, 37 ou 38, la particule ayant un diamètre de 10 nm à 3 µm, de 10 nm à 100 nm ou de 50 nm à 500 nm.

**48.** Procédé selon la revendication 5, dans lequel on a introduit dans la cellule au moins un autre gène qui code pour une protéine, où on choisit la protéine dans le groupe qui comprend une polymère-dépolymérase, un régulateur de polymère, une polymère-synthase, et une protéine contrôlant la granulométrie de la particule, où, pour ce qui concerne l'aumoins une autre protéine, il s'agit d'une protéine de fusion qui comprend une protéine bioactive, ou
où, pour ce qui concerne l'aumoins une autre protéine, il s'agit d'une protéine de fusion qui est à même de lier une substance bioactive, directement ou par l'intermédiaire d'un réactif de couplage, ou
où, pour ce qui concerne l'aumoins une autre protéine, il s'agit d'une protéine modifiée, qui est à même de lier une substance bioactive, directement ou par l'intermédiaire d'un réactif de couplage.

**49.** Utilisation selon la revendication 35 ou 36, pour laquelle la particule polymère comprend en outre au moins une autre protéine, la protéine étant choisie dans le groupe qui comprend une polymère-dépolymérase, un régulateur de polymère, une polymère-synthase et une protéine contrôlant la granulométrie de la particule, pour laquelle, pour ce qui concerne l'aumoins une autre protéine, il s'agit d'une protéine de fusion qui comprend une protéine bioactive, ou
où, pour ce qui concerne l'aumoins une autre protéine, il s'agit d'une protéine de fusion qui est à même de lier une substance bioactive, directement ou par l'intermédiaire d'un réactif de couplage, ou
pour laquelle, pour ce qui concerne l'aumoins une autre protéine, il s'agit d'une protéine modifiée qui est à même de lier une substance biologiquement active, directement ou par l'intermédiaire d'un réactif de couplage.

**50.** Médicament, pesticide ou herbicide selon la revendication 37 ou 38, dans lequel la particule polymère comprend en outre au moins une autre protéine, l'autre protéine étant choisie dans le groupe qui comprend une polymère-dépolymérase, un régulateur de polymère, une polymère-synthase et une protéine contrôlant la granulométrie de la particule,
où, pour ce qui concerne l'aumoins une autre protéine, il s'agit d'une protéine de fusion qui comprend une protéine bioactive, ou
pour laquelle, pour ce qui concerne l'aumoins une autre protéine, il s'agit d'une protéine de fusion qui est à même de lier une substance bioactive, directement ou par l'intermédiaire d'un réactif de couplage, ou
pour laquelle, pour ce qui concerne l'aumoins une autre protéine, il s'agit d'une protéine modifiée qui est à même de lier la substance bioactive, directement ou par l'intermédiaire d'un réactif de couplage.

Abbildung 1

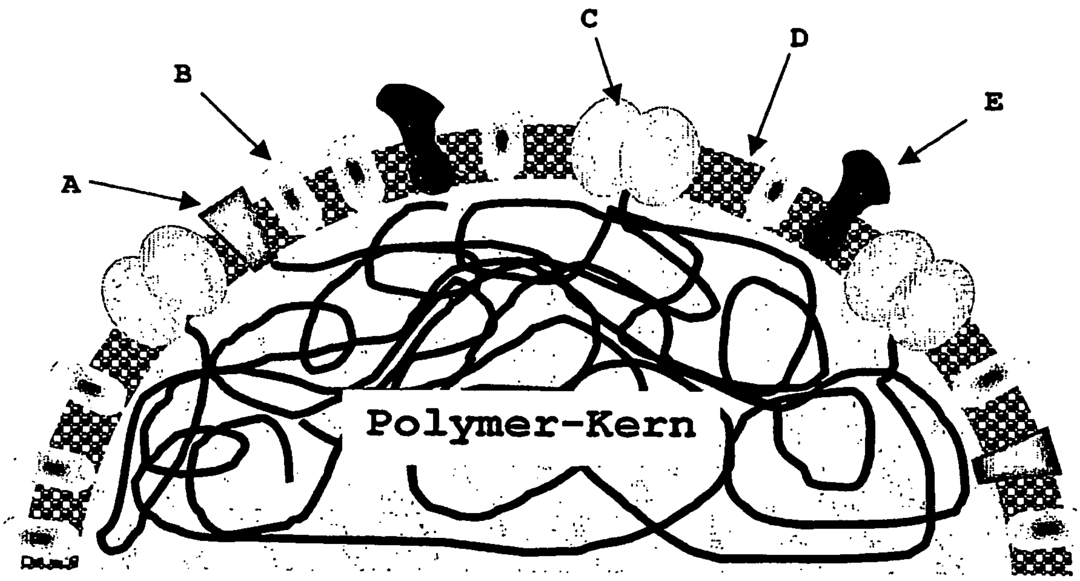


Abbildung 2

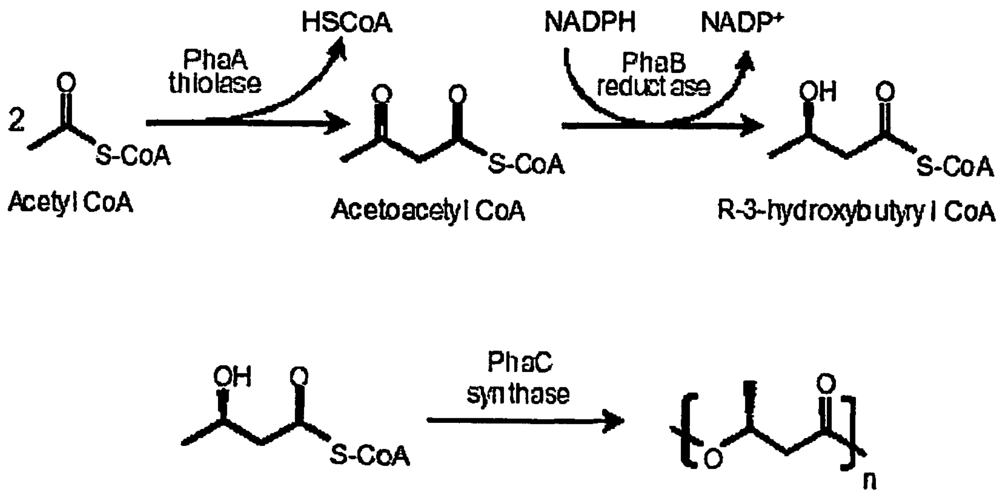

Abbildung 3

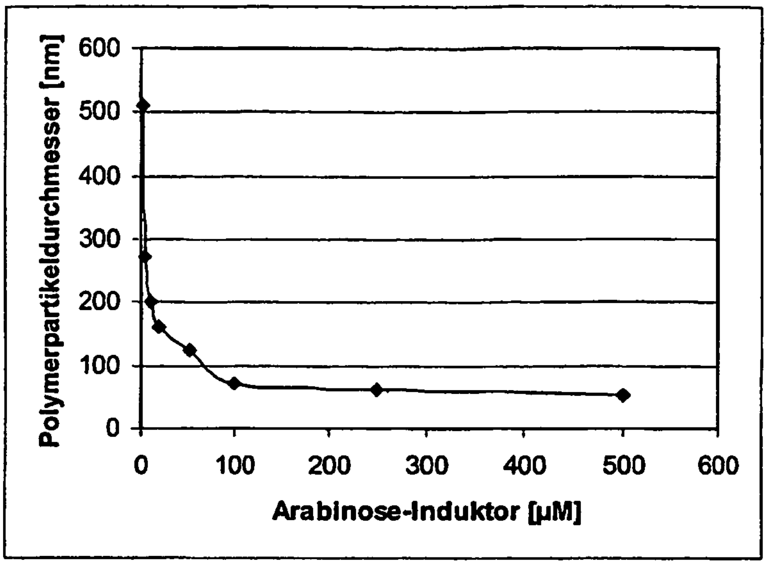


Abbildung 4

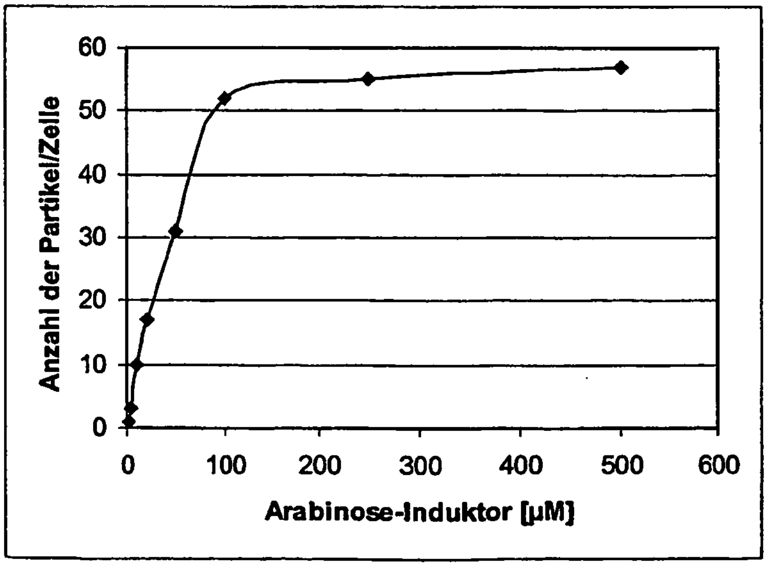

Abbildung 5

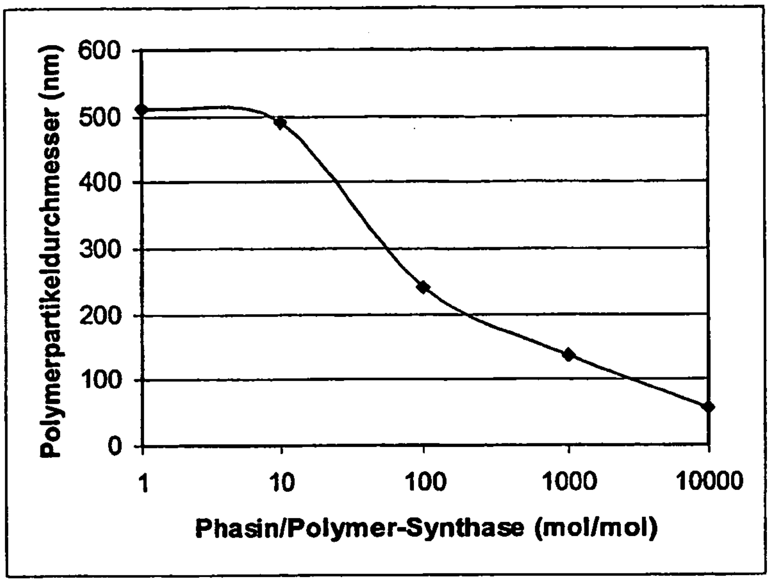


Abbildung 6

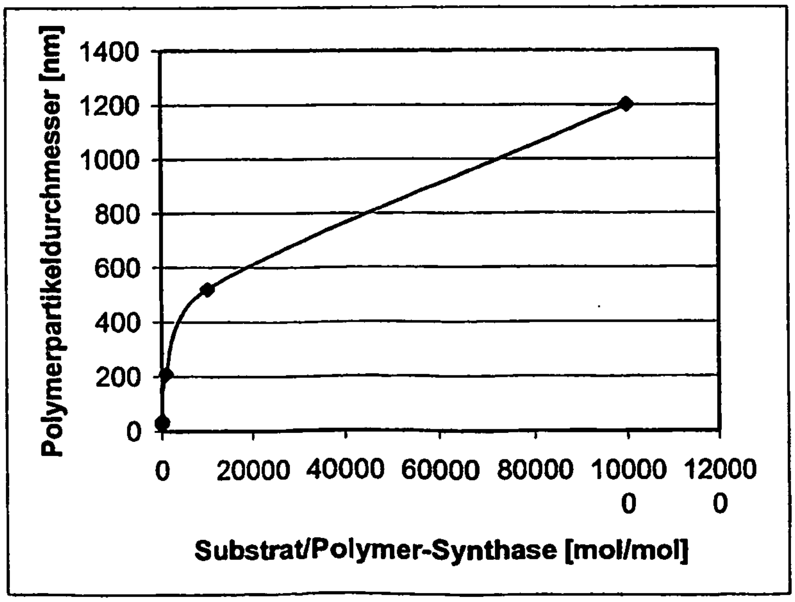

Abbildung 7

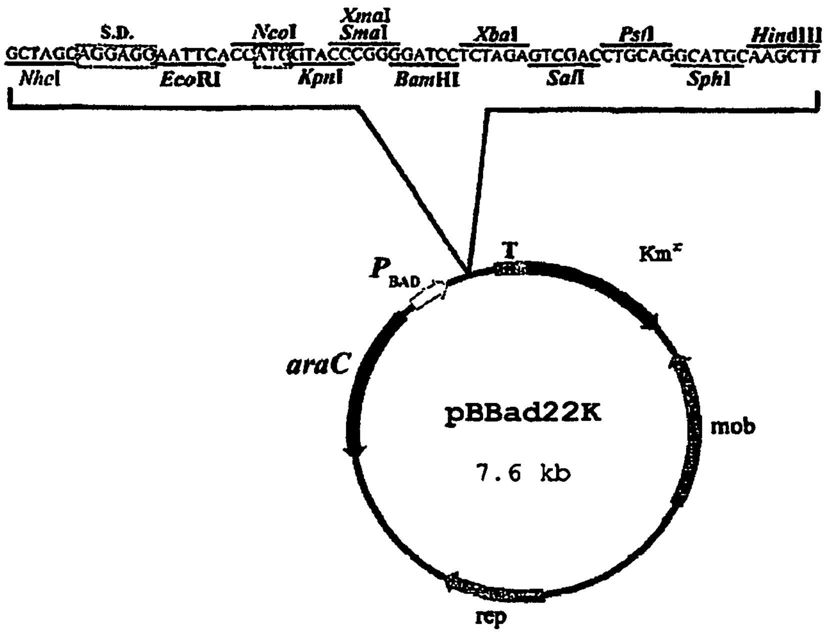


Abbildung 8

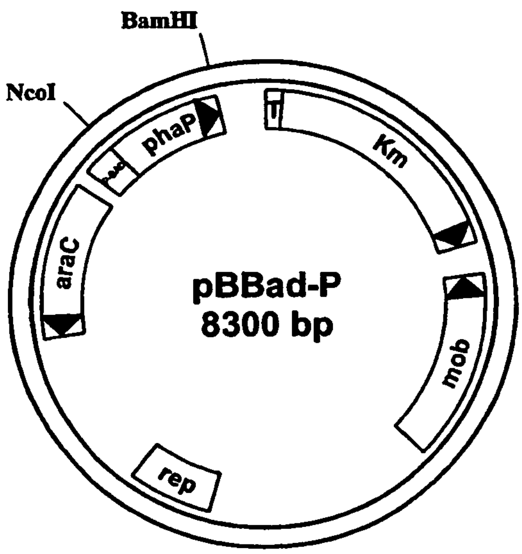

Abbildung 9

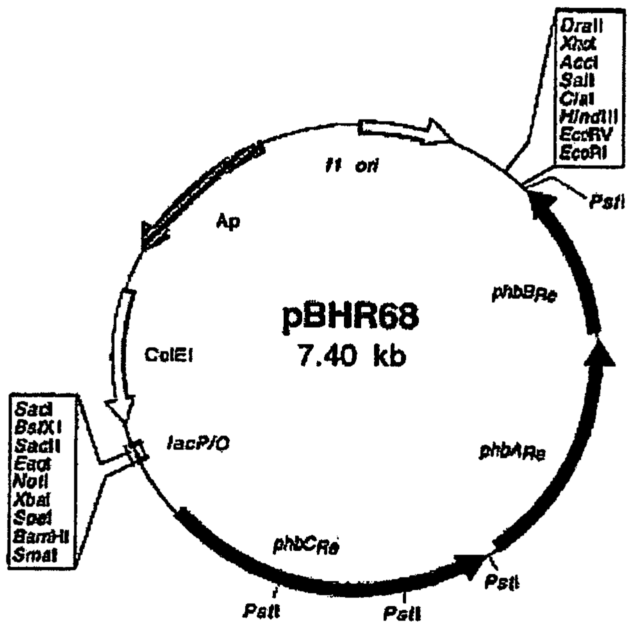


Abbildung 10

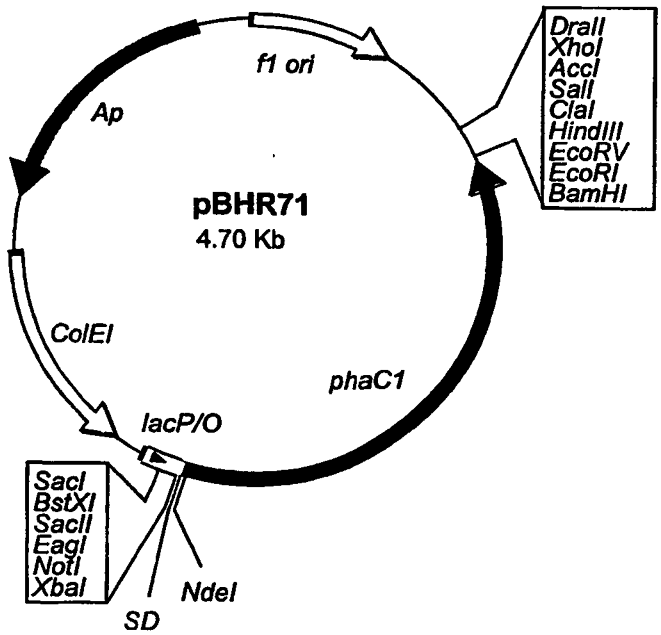

Abbildung 11

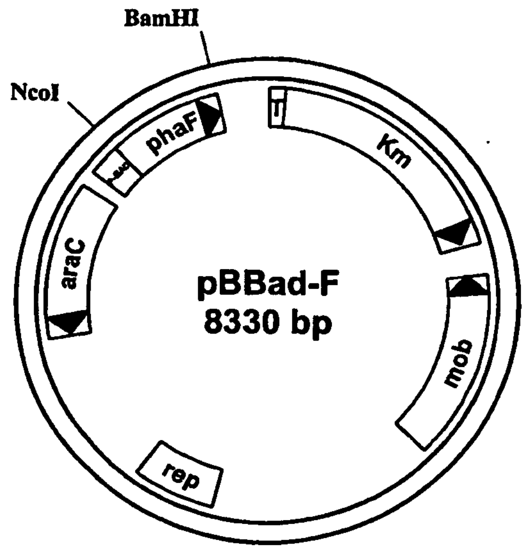


Abbildung 12

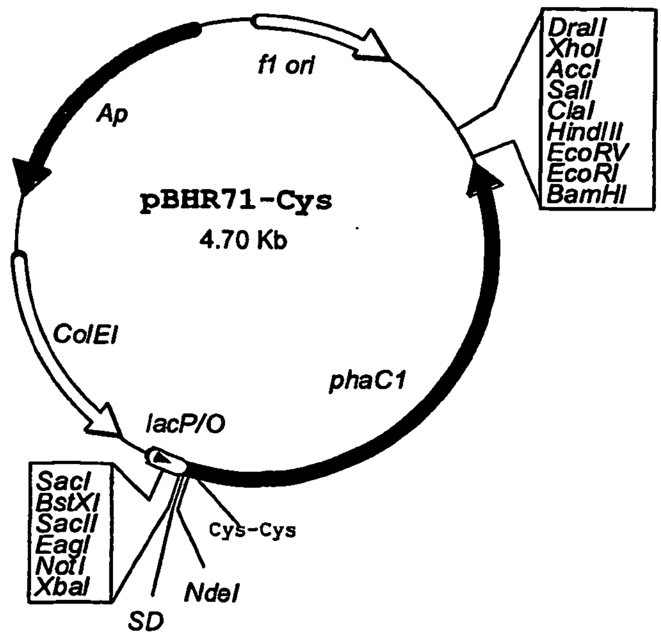

Abbildung 13

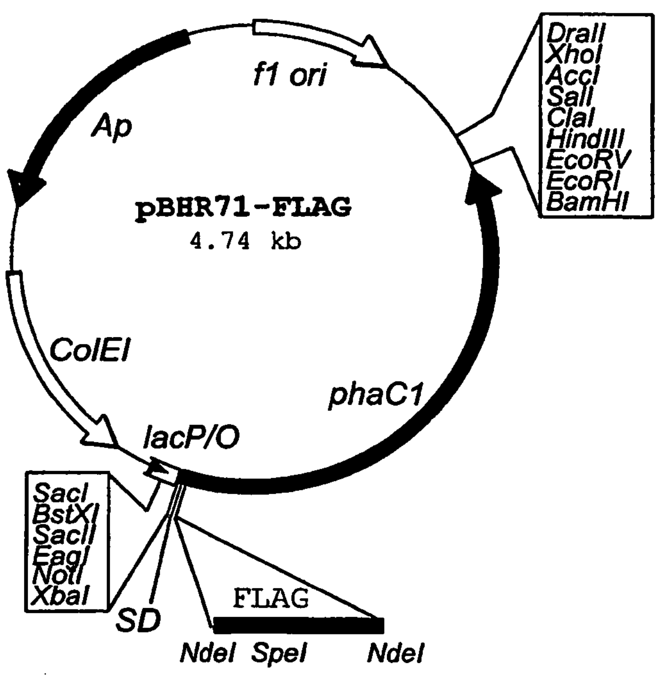

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 19745950 A1
- DE 10240034


### In der Beschreibung aufgeführte Nicht-Patentliteratur


- **Sanovich, E. et al.** *Brain Res.,* 1995, vol. 705 (1-2), 125-135
- **Greig, N.H. et al.** *Cancer Chemother. Pharmacol.,* 1990, vol. 25 (5), 320-325
- **Kreuter, J.** *J. Anat.,* 1996, vol. 189 (3), 503-505
- **Lockman, P.R et al.** *Drug Develop. Indust. Pharmacy,* 2002, vol. 28 (1), 1-12
- **Lockman, P.R. et al.** *Drug Develop. Indust. Pharmacy,* 2002, vol. 28 (1), 1-12
- **Wieczorek, R. et al.** *J. Bacteriol.,* 1997, vol. 177 (9), 5.2425-2435
- **Fernart, L. et al.** *J. Pharmacol. Exp. Ther.,* 1999, vol. 291 (3), 1017-1022
- **Sukchawalit, R. et al.** *FEMS Microbiol Lett.,* 1999, vol. 181 (2), 217-223
- **Spiekermann, P. et al.** *Arch. Microbiol.,* 1999, vol. 171, 73-80
- **Langenbach, S. et al.** *FEMS Microbiol. Lett.,* 1997, vol. 150, 303-309
- **York, G.M. et al.** *J. Bacteriol.,* 2001, vol. 183, 4217-4226
- **Hanley, S.Z. et al.** *FEBS Letters,* 1999, vol. 447, 99-105
- **Qi, Q. et al.** *FEMS Microbiol. Lett.,* 1998, vol. 167, 89-94
- **Prieto, M.A. et al.** *J. Bacteriol.,* 1999, vol. 181 (3), 858-868
- **Gerngross, T.U. ; Martin, D.P.** *Proc. Natl. Acad. Sci. USA,* 1995, vol. 92, 6279-6283
- **Qi, Q. et al.** *Appl. Microbiol. Biotechnol.,* 2000, vol. 54, 37-43
- **King, H.D. et al.** *Biconjugate Chem.,* 1999, vol. 10, 279-288
- **Rehm, B.H.A. et al.** *Biochem. Biophys. Acta,* 2002, vol. 1594, 178-190
- **Saegusa, H. et al.** *J. Bacteriol.,* 2001, vol. 183 (1), 94-100
- **Rehm, B.H.A. et al.** *J. Bacteriol.,* 1994, vol. 176, 5639-5647

Sequenzliste

<110> Rehm, PD Dr. Bern H.A.
<120> Verfahren zur Herstellung von biologisch abbaubaren, funktionalisierten Polymerpartikeln und deren Verwendung als Arzneimittelträger
<130> P200428
<150> DE 102 40 034.0
<151> 2002-08-30
<160> 15
<170> PatentIn version 3.2
<210> 1
<211> 29
<212> DNA
<213> Artificial
<220>
<223> Primer
<400> 1
aaaggcccca tggtcctcac cccggaaca 29
<210> 2
<211> 33
<212> DNA
<213> Artificial
<220>
<223> Primer
<400> 2
aaaggccgga tcctcagggc actaccttca tcg 33
<210> 3
<211> 708
<212> DNA
<213> Artificial
<220>
<223> Sequenz enthält phaP codierende DNA aus R. eutropha
<400> 3

aaaggcccca tgatcctcac cccggaacaa gttgcagcag cgcaaaaggc caacctcgaa 60
acgctgttcg gcctgaccac caaggcgttt gaaggcgtcg aaaagctcgt cgagctgaac 120
ctgcaggtcg tcaagacttc gttcgcagaa ggcgttgaca acgccaagaa ggcgctgtcg 180
gccaaggacg cacaggaact gctggccatc caggccgcag ccgtgcagcc ggttgccgaa 240
aagaccctgg cctacacccg ccacctgtat gaaatcgctt cggaaaccca gagcgagttc 300
accaaggtag ccgaggctca actggccgaa ggctcgaaga acgtgcaagc gctggtcgag 360
aacctcgcca agaacgcccc ggccggttcg gaatcgaccg tggccatcgt gaagtcggcg 420

atctccgctg ccaacaacgc ctacgagtcg gtgcagaagg cgaccaagca agcggtcgaa 480
atcgctgaaa ccaacttcca ggctgcggct acggctgcca ccaaggctgc ccagcaagcc 540
agcgccacgg cccgtacggc cacggcaaag aagacgacgg ctgcctgata actgcctgcg 600
ttgaagatgg accggctgcg gccggtccgt tggcaaagca tatcgacgcc tggcgtttgc 660
ggtgtgtttt gccaacgatg aaggtagtgc cctgaggatc cggccttt 708

<210> 4
<211> 34
<212> DNA
<213> Artificial
<220>
<223> Primer
<400> 4
aaagggccat ggctggcaag aagaattccg agaa 34
<210> 5
<211> 39
<212> DNA
<213> Artificial
<220>
<223> Primer
<400> 5
aaagggggat cctcagatca gggtaccggt gcctgtctg 39
<210> 6
<211> 793
<212> DNA
<213> Artificial
<220>
<223> Sequenz enthält phaF codierende DNA aus P. oleovorans
<400> 6

aaagggccat ggctggcaag aagaattccg agaaagaagg cagctcctgg gtcggcggga 60
tcgagaagta ctcccgcaag atctggctgg cggggcttgg tatctattcg aagatcgacc 120
aggacggccc gaagctgttc gactcgctgg taaaggatgg cgagaaggcc gagaaacagg 180
cgaagaagac cgcagaagat gttgctgaaa ctgccaagtc gtcgaccact tcgcgtgtgt 240
cgggcgtgaa ggaccgtgcg ctaggcaagt ggagcgaact cgaagaggcc ttcgacaagc 300
gcctgaacag tgccatctcg cgccttggcg tgccgagccg caacgagatc aaggccctgc 360
accagcaggt ggacagcctg accaagcaga tcgagaaact caccggcgct tcggttaccc 420
cgatttcgtc gcgcactgca gccaaaccgg ctgcgagcaa ggcggcggcc aagccactgg 480
ccaagacggc agcggccaag cctgcggcaa aaaccgcggc agccaagccg gcagccaagg 540
ccgcagcggc taaacctgct gccaagactg cggcggccaa gcctgcggcg aaaccggcag 600

cggccaaacc ggctgtggcg aagaagcctg cagtgaagaa agcaccggcc aagccggcag 660
ccgccaagcc ggcagctcca gcggccagcg ccgctccggc cgctagcgca gttcggcgcc 720
cactgcggct ccggccagca acccgccttc ggcacagaca ggcaccggta ccctgatctg 780
aggatccccc ttt 793

<210> 7
<211> 54
<212> DNA
<213> Artificial
<220>
<223> Primer
<400> 7
gggctctaga aataaggaga tatacatatg tgttgtaaga acaataacga gctt 54
<210> 8
<211> 29
<212> DNA
<213> Artificial
<220>
<223> Primer
<400> 8
aaacgcggat ccttttcatc gttcatgca 29
<210> 9
<211> 1722
<212> DNA
<213> Artificial
<220>
<223> Sequenz enthält die für PhaC1 codierende DNA aus P. aeruginosa
<400> 9

gggctctaga aataaggaga tatacatatg tgttgtaaga acaataacga gcttcccaag 60
caagccgcgg aaaacacgct gaacctgaat ccggtgatcg gcatccgggg caaggacctg 120
ctcacctccg cgcgcatggt cctgctccag gcggtgcgcc agccgctgca cagcgccagg 180
cacgtggcgc atttcagcct ggagctgaag aacgtcctgc tcggccagtc ggagctacgc 240
ccaggcgatg acgaccgacg cttttccgat ccggcctgga gccagaatcc actgtacaag 300
cgctacatgc agacctacct ggcctggcgc aaggagctgc acagctggat cagccacagc 360
gacctgtcgc cgcaggacat cagtcgtggc cagttcgtca tcaacctgct gaccgaggcg 420
atgtcgccga ccaacagcct gagcaacccg gcggcggtca agcgcttctt cgagaccggc 480
ggcaagagcc tgctggacgg cctcggccac ctggccaagg acctggtgaa caacggcggg 540
atgccgagcc aggtggacat ggacgccttc gaggtgggca agaacctggc caccaccgag 600
ggcgccgtgg tgttccgcaa cgacgtgctg gaactgatcc agtaccggcc gatcaccgag 660

tcggtgcacg aacgcccgct gctggtggtg ccgccgcaga tcaacaagtt ctacgtcttc 720
gacctgtcgc cggacaagag cctggcgcgc ttctgcctgc gcaacggcgt gcagaccttc 780
atcgtcagtt ggcgcaaccc gaccaagtcg cagcgcgaat ggggcctgac cacctatatc 840
gaggcgctca aggaggccat cgaggtagtc ctgtcgatca ccggcagcaa ggacctcaac 900
ctcctcggcg cctgctccgg cgggatcacc accgcgaccc tggtcggcca ctacgtggcc 960
agcggcgaga agaaggtcaa cgccttcacc caactggtca gcgtgctcga cttcgaactg 1020
aatacccagg tcgcgctgtt cgccgacgag aagactctgg aggccgccaa gcgtcgttcc 1080
taccagtccg gcgtgctgga gggcaaggac atggccaagg tgttcgcctg gatgcgcccc 1140
aacgacctga tctggaacta ctgggtcaac aactacctgc tcggcaacca gccgccggcg 1200
ttcgacatcc tctactggaa caacgacacc acgcgcctgc ccgccgcgct gcacggcgag 1260
ttcgtcgaac tgttcaagag caacccgctg aaccgccccg gcgccctgga ggtctccggc 1320
acgcccatcg acctgaagca ggtgacttgc gacttctact gtgtcgccgg tctgaacgac 1380
cacatcaccc cctgggagtc gtgctacaag tcggccaggc tgctgggtgg caagtgcgag 1440
ttcatcctct ccaacagcgg tcacatccag agcatcctca acccaccggg caaccccaag 1500
gcacgcttca tgaccaatcc ggaactgccc gccgagccca aggcctggct ggaacaggcc 1560
ggcaagcacg ccgactcgtg gtggttgcac tggcagcaat ggctggccga acgctccggc 1620
aagacccgca aggcgcccgc cagcctgggc aacaagacct atccggccgg cgaagccgcg 1680
cccggaacct acgtgcatga acgatgaaaa ggatccgcgt tt 1722

<210> 10
<211> 36

<212> DNA
<213> Artificial
<220>
<223> Primer
<400> 10
tatgactagt gattataaag atgatgatga taaaca 36
<210> 11
<211> 36
<212> DNA
<213> Artificial
<220>
<223> Primer
<400> 11
tatgtttatc atcatcatct ttataatcac tagtca 36
<210> 12
<211> 1716
<212> UNA
<2.13> Artificial
<220>
<223> Sequenz enthält die für PhaC1 codierende DNA aus P. aeruginosa und die für ein FLAG-Epitop codierende DNA
<400> 12

atgactagtg attataaaga tgatgatgat aaacatatga gtcagaagaa caataacgag 60
cttcccaagc aagccgcgga aaacacgctg aacctgaatc cggtgatcgg catccggggc 120
aaggacctgc tcacctccgc gcgcatggtc ctgctccagg cggtgcgcca gccgctgcac 180
agcgccaggc acgtggcgca tttcagcctg gagctgaaga acgtcctgct cggccagtcg 240
gagctacgcc caggcgatga cgaccgacgc ttttccgatc cggcctggag ccagaatcca 300
ctgtacaagc gctacatgca gacctacctg gcctggcgca aggagctgca cagctggatc 360
agccacagcg acctgtcgcc gcaggacatc agtcgtggcc agttcgtcat caacctgctg 420
accgaggcga tgtcgccgac caacagcctg agcaacccgg cggcggtcaa gcgcttcttc 480
gagaccggcg gcaagagcct gctggacggc ctcggccacc tggccaagga cctggtgaac 540
aacggcggga tgccgagcca ggtggacatg gacgccttcg aggtgggcaa gaacctggcc 600
accaccgagg gcgccgtggt gttccgcaac gacgtgctgg aactgatcca gtaccggccg 660
atcaccgagt cggtgcacga acgcccgctg ctggtggtgc cgccgcagat caacaagttc 720
tacgtcttcg acctgtcgcc ggacaagagc ctggcgcgct tctgcctgcg caacggcgtg 780
cagaccttca tcgtcagttg gcgcaacccg accaagtcgc agcgcgaatg ggcctgacc 840
acctatatcg aggcgctcaa ggaggccatc gaggtagtcc tgtcgatcac cggcagcaag 900
gacctcaacc tcctcggcgc ctgctccggc gggatcacca ccgcgaccct ggtcggccac 960
tacgtggcca gcggcgagaa gaaggtcaac gccttcaccc aactggtcag cgtgctcgac 1020
ttcgaactga atacccaggt cgcgctgttc gccgacgaga agactctgga ggccgccaag 1080
cgtcgttcct accagtccgg cgtgctggag ggcaaggaca tggccaaggt gttcgcctgg 1140
atgcgcccca acgacctgat ctggaactac tgggtcaaca actacctgct cggcaaccag 1200
ccgccggcgt tcgacatcct ctactggaac aacgacacca cgcgcctgcc cgccgcgctg 1260
cacggcgagt tcgtcgaact gttcaagagc aacccgctga accgccccgg cgccctggag 1320
gtctccggca cgcccatcga cctgaagcag gtgacttgcg acttctactg tgtcgccggt 1380
ctgaacgacc acatcacccc ctgggagtcg tgctacaagt cggccaggct gctgggtggc 1440
aagtgcgagt tcatcctctc caacagcggt cacatccaga gcatcctcaa cccaccgggc 1500
aaccccaagg cacgcttcat gaccaatccg gaactgcccg ccgagcccaa ggcctggctg 1560
gaacaggccg gcaagcacgc cgactcgtgg tggttgcact ggcagcaatg gctggccgaa 1620
cgctccggca agacccgcaa ggcgcccgcc agcctgggca acaagaccta tccggccggc 1680

gaagccgcgc ccggaaccta cgtgcatgaa cgatga 1716

<210> 13
<211> 34
<212> DNA
<213> Artificial

<220>
<223> Primer
<400> 13
ggactagtat gaccatgatt acggattcac tggc 34
<210> 14
<211> 41
<212> DNA
<213> Artificial
<220>
<223> Primer
<400> 14
ccactagttt tttgacacca gaccaactgg taatggtagc g 41
<210> 15
<211> 3088
<212> DNA
<213> Artificial
<220>
<223> Sequenz enthält das lacZ Gen aus E. coli
<400> 15

```
ggactagtat gaccatgatt acggattcac tggccgtcgt tttacaacgt cgtgactggg      60
aaaaccctgg cgttacccaa cttaatcgcc ttgcagcaca tccccctttc gccagctggc     120
gtaatagcga agaggcccgc accgatcgcc cttcccaaca gttgcgcagc ctgaatggcg     180
aatggcgctt tgcctggttt ccggcaccag aagcggtgcc ggaaagctgg ctggagtgcg     240
atcttcctga ggccgatact gtcgtcgtcc cctcaaactg gcagatgcac ggttacgatg     300
cgcccatcta caccaacgtg acctatccca ttacggtcaa tccgccgttt gttcccacgg     360
agaatccgac gggttgttac tcgctcacat ttaatgttga tgaaagctgg ctacaggaag     420
gccagacgcg aattattttt gatggcgtta actcggcgtt tcatctgtgg tgcaacgggc     480
gctgggtcgg ttacggccag gacagtcgtt tgccgtctga atttgacctg agcgcatttt     540
tacgcgccgg agaaaaccgc ctcgcggtga tggtgctgcg ctggagtgac ggcagttatc     600
tggaagatca ggatatgtgg cggatgagcg gcattttccg tgacgtctcg ttgctgcata     660
aaccgactac acaaatcagc gatttccatg ttgccactcg ctttaatgat gatttcagcc     720
gcgctgtact ggaggctgaa gttcagatgt gcggcgagtt gcgtgactac ctacgggtaa     780
cagtttcttt atggcagggt gaaacgcagg tcgccagcgg caccgcgcct ttcggcggtg     840
```

aaattatcga tgagcgtggt ggttatgccg atcgcgtcac actacgtctg aacgtcgaaa 900
acccgaaact gtggagcgcc gaaatcccga atctctatcg tgcggtggtt gaactgcaca 960
ccgccgacgg cacgctgatt gaagcagaag cctgcgatgt cggtttccgc gaggtgcgga 1020
ttgaaaatgg tctgctgctg ctgaacggca agccgttgct gattcgaggc gttaaccgtc 1080
acgagcatca tcctctgcat ggtcaggtca tggatgagca gacgatggtg caggatatcc 1140
tgctgatgaa gcagaacaac tttaacgccg tgcgctgttc gcattatccg aaccatccgc 1200
tgtggtacac gctgtgcgac cgctacggcc tgtatgtggt ggatgaagcc aatattgaaa 1260
cccacggcat ggtgccaatg aatcgtctga ccgatgatcc gcgctggcta ccggcgatga 1320
gcgaacgcgt aacgcgaatg gtgcagcgcg atcgtaatca cccgagtgtg atcatctggt 1380
cgctggggaa tgaatcaggc cacggcgcta atcacgacgc gctgtatcgc tggatcaaat 1440
ctgtcgatcc ttcccgcccg gtgcagtatg aaggcggcgg agccgacacc acggccaccg 1500
atattatttg cccgatgtac gcgcgcgtgg atgaagacca gcccttcccg gctgtgccga 1560
aatggtccat caaaaaatgg ctttcgctac ctggagagac gcgcccgctg atcctttgcg 1620
aatacgccca cgcgatgggt aacagtcttg gcggtttcgc taaatactgg caggcgtttc 1680
gtcagtatcc ccgtttacag ggcggcttcg tctgggactg ggtggatcag tcgctgatta 1740
aatatgatga aaacggcaac ccgtggtcgg cttacggcgg tgattttggc gatacgccga 1800
acgatcgcca gttctgtatg aacggtctgg tctttgccga ccgcacgccg catccagcgc 1860
tgacggaagc aaaacaccag cagcagtttt tccagttccg tttatccggg caaaccatcg 1920
aagtgaccag cgaatacctg ttccgtcata gcgataacga gctcctgcac tggatggtgg 1980
cgctggatgg taagccgctg gcaagcggtg aagtgcctct ggatgtcgct ccacaaggta 2040
aacagttgat tgaactgcct gaactaccgc agccggagag cgccgggcaa ctctggctca 2100
cagtacgcgt agtgcaaccg aacgcgaccg catggtcaga agccgggcac atcagcgcct 2160
ggcagcagtg gcgtctggcg gaaaacctca gtgtgacgct ccccgccgcg tcccacgcca 2220
tcccgcatct gaccaccagc gaaatggatt tttgcatcga gctgggtaat aagcgttggc 2280
aatttaaccg ccagtcaggc tttctttcac agatgtggat tggcgataaa aaacaactgc 2340
tgacgccgct gcgcgatcag ttcacccgtg caccgctgga taacgacatt ggcgtaagtg 2400
aagcgacccg cattgaccct aacgcctggg tcgaacgctg gaaggcggcg ggccattacc 2460
aggccgaagc agcgttgttg cagtgcacgg cagatacact tgctgatgcg gtgctgatta 2520
cgaccgctca cgcgtggcag catcagggga aaaccttatt tatcagccgg aaaacctacc 2580
ggattgatgg tagtggtcaa atggcgatta ccgttgatgt tgaagtggcg agcgatacac 2640
cgcatccggc gcggattggc ctgaactgcc agctggcgca ggtagcagag cgggtaaact 2700
ggctcggatt agggccgcaa gaaaactatc ccgaccgcct tactgccgcc tgttttgacc 2760

```
gctgggatct gccattgtca gacatgtata ccccgtacgt cttcccgagc gaaaacggtc   2820
tgcgctgcgg gacgcgcgaa ttgaattatg gcccacacca gtggcgcggc gacttccagt   2880
tcaacatcag ccgctacagt caacagcaac tgatggaaac cagccatcgc catctgctgc   2940
acgcggaaga aggcacatgg ctgaatatcg acggtttcca tatggggatt ggtggcgacg   3000
actcctggag cccgtcagta tcggcggaat tccagctgag cgccggtcgc taccattacc   3060
agttggtctg gtgtcaaaaa actagtgg                                      3088
```